# EUROPEAN PATENT SPECIFICATION

(11) **EP 2 412 387 B1**
(45) Date of publication and mention of the grant of the patent: **19.11.2014**
(21) Application number: 10714045.1
(22) Date of filing: 26.03.2010
(51) Int. Cl.: A61K 48/00, C07K 14/65, C12N 15/86, C12N 15/864

(54) **METHODS AND COMPOSITIONS FOR THE TREATMENT OF CIRRHOSIS AND LIVER FIBROSIS**
VERFAHREN UND ZUSAMMENSETZUNGEN ZUR BEHANDLUNG VON ZIRRHOSE UND LEBERFIBROSE
MÉTHODES ET COMPOSITIONS POUR LE TRAITEMENT DE LA CIRRHOSE ET DE LA FIBROSE HÉPATIQUE

(30) Priority: 27.03.2009 ES 200900846
(43) Date of publication of application: 01.02.2012
(73) Proprietor: Proyecto de Biomedicina Cima, S.L., 31008 Pamplona Navarra (ES)
(72) Inventor: FORTES ALONSO, María Purificacíon, 31008 Pamplona Navarra (ES); PRIETO VALTUEÑA, Jesús Maria, 31008 Pamplona Navarra (ES); SOBREVALS, Luciano Matías, 31008 Pamplona Navarra (ES); PETRY, Harald, 1105 BA Amsterdam (NL); TIMMERMANS, Eric Jacobus Hubertus, 1105 BA Amsterdam (NL)
(74) Representative: ABG Patentes, S.L.
(86) International application number: PCT/ES2010/070184
(87) International publication number: WO 2010/109053

(56) References cited:
- WO-A1-98/09524
- WO-A2-2008/137490
- VERA M ET AL: "Liver transduction with a simian virus 40 vector encoding insulin-like growth factor I reduces hepatic damage and the development of liver cirrhosis" GENE THERAPY, vol. 14, no. 3, February 2007 (2007-02), pages 203-210, XP002589972 ISSN: 0969-7128
- SOBREVALS L ET AL: "89 TREATMENT OF ESTABLISHED CIRRHOSIS USING SV40 VECTOR ENCODING INSULIN-LIKE GROWTH FACTOR I" JOURNAL OF HEPATOLOGY, MUNKSGAARD INTERNATIONAL PUBLISHERS, COPENHAGEN, DK LNKD- DOI:10.1016/S0168-8278(08)60091-4, vol. 48, 1 January 2008 (2008-01-01), page S39, XP026661815 ISSN: 0168-8278 [retrieved on 2008-01-01]
- ZARATIEGUI M ET AL: "IGF1 gene transfer into skeletal muscle using recombinant adeno-associated virus in a rat model of liver cirrhosis." JOURNAL OF PHYSIOLOGY AND BIOCHEMISTRY, vol. 58, no. 3, September 2002 (2002-09), pages 169-176, XP8123888 ISSN: 1138-7548
- SOBREVALS LUCIANO ET AL: "Insulin-Like Growth Factor I Gene Transfer to Cirrhotic Liver Induces Fibrolysis and Reduces Fibrogenesis Leading to Cirrhosis Reversion in Rats" HEPATOLOGY, vol. 51, no. 3, March 2010 (2010-03), pages 912-921, XP002589974 ISSN: 0270-9139 DOI: DOI:10.1002/HEP.23412
- GAO GUANG-PING ET AL: "Novel adeno-associated viruses from rhesus monkeys as vectors for human gene therapy" PROCEEDINGS OF THE NATIONAL ACADEMY OF SCIENCES OF THE UNITED STATES OF AMERICA, vol. 99, no. 18, 3 September 2002 (2002-09-03), pages 11854-11859, XP002589975 ISSN: 0027-8424
- MCCARTY D M ET AL: "Adeno-associated virus terminal repeat (TR) mutant generates self-complementary vectors to overcome the rate-limiting step to transduction in vivo." GENE THERAPY, vol. 10, no. 26, December 2003 (2003-12), pages 2112-2118, XP002367806 ISSN: 0969-7128

## Description

### TECHNICAL FIELD

The invention relates to the field of gene therapy and, more particularly, to methods for the treatment of cirrhosis and liver fibrosis by the use of viral vectors.

### BACKGROUND OF THE INVENTION

Liver transplantation is the only curative option for patients with advanced liver cirrhosis. This procedure can only be applied to a minority of patients due to the presence of surgical contraindications and organ scarcity. In fact, the waiting list in USA includes ∼12500 patients with a median time to transplantation of ∼300 days, more than 45% of the patients exceed two years in the waiting list where the mortality reaches 130 per 1000 patients/year (Freeman R.B. et al., Am J. Transplant. 2008; 8:958-976).

Insulin-like growth factor I (IGF-I) is a potent cytoprotective and anabolic hormone. Serum IGF-I is mostly of hepatic origin and circulates bound to a set of binding proteins (IGFBPs) which regulate IGF-I biological activity (Adamo M, et al., 1989, Endocrinology, 124:2737-2744 and Mohan S et al., 2002, J. Endocrinol., 175:19-31). Degradation of IGFBPs releases free IGF-I which can interact and activate IGF-I receptor (IGF-IR). IGF-I can also bind insulin receptor and insulin can activate IGF-IR, but the affinity for their cognate receptor is 100-1000 fold higher (Nitert MD et al., 2005, Mol. Cell. Endocrinol. 229:31-37). Interaction with IGF-IR leads to activation of the MAP kinase and PI3 kinase cascades which regulate genes involved in cell survival, growth and differentiation (Riedemann J. et al., 2006, Endocr. Relat., Cancer. 13 Suppl 1:S33-43).

In liver cirrhosis, as result of hepatocellular insufficiency, there is a marked reduction in the levels of IGF-I. This hormonal deficiency may play a role in the systemic metabolic derangement present in liver cirrhosis (Conchillo M. et al., 2005; 43:630-636 and Lorenzo-Zuniga V. et al., 2006, Gut 55:1306-1312). In fact, treatment of cirrhotic rats with recombinant IGF-I (rIGF-I) promotes weight gain, nitrogen retention, and intestinal absorption of nutrients (Castilla-Cortazar I. et al., 2000, Hepatology, 31:592-600). In addition, rIGF-I has been shown to exert hepatoprotective activities in cirrhotic rats (Castilla-Cortazar I. et al., 1997, Gastroenterology, 113:1682-1691 and Muguerza B, et al., 2001, Biochim Biophys Acta. 1536:185-195). Also, a recent pilot clinical trial with a daily dose of rIGF-I of 100 µg/kg bw in cirrhotic patients, resulted in a significant increase of serum albumin and improvement of Child-Pugh score (Conchillo M. et al., 2005, J. Hepatol. 43:630-636). However, because of the high amount of rIGF-I needed, the potential benefit of IGF-I therapy in liver cirrhosis is counterbalanced by the high cost of the treatment.

As an alternative to the direct administration of IGF-I, the use of viral vectors comprising a polynucleotide encoding IGF-I has been proposed. Vera M. et al. (Gene Ther., 2007, 14:203-210) have described that the injection of a recombinant Simian Virus 40 vector encoding IGF-I into healthy rats can protect the liver against CCl₄-induced liver damage. Sobrevals et al (Molecular Therapy Volume 16, Supplement 1, May 2008, S145) and Sobrevals et al.(J. Hepatology. volume 48, 2008, 539) have described that the administration of a recombinant Simian Virus 40 vector encoding IGF-I may revert part of the effects of CCl₄-induced liver cirrhosis. However, not all results concerning viral vectors have provided positive results. For instance, Zaraitegui et al (J.Physiol.Biochem., 2002, 58:169-176) have described that the intra-muscular administration of adenoassociated virus encoding IGF-I to rats with CCl₄-induced cirrhosis did not result in any significant amelioration of liver damage.

The document WO2008137490 describes an adeno-associated virus (AAV) vector encoding IGF-I. The document WO9809524 describes an AAV vector carrying the liver-specific alpha1-antitrypsin promoter and a liver-specific enhancer, for liver-specific delivery of a therapeutic molecule in a mammalian patient.

Accordingly, there is a need in the art for improved delivery of IGF-I which are useful for the treatment of liver cirrhosis.

### SUMMARY OF THE INVENTION

The invention in its broadest sense is as characterized in the independent claims.

The invention thus relates to:
1. A parvoviral vector comprising a nucleotide sequence encoding insulin-like growth factor I IGF-I or a functionally equivalent variant thereof having the biological activity of IGF-I which is operably linked to a liver-specific promoter comprising the albumin gene enhancer region and the alphal-antitrypsin promoter.
2. A parvoviral vector as defined in Item 1 wherein the parvoviral vector is an AAV.
3. A parvoviral vector as defined in Item 2 wherein the AAV vector is an AAV1, AAV2, AAV5 or AAV8.
4. A parvoviral vector as defined in Items 2 or 3 wherein the AAV vector is a single-stranded AAV or a double-stranded AAV.
5. A parvoviral vector as defined in Item 4 wherein the AAV vector is a double-stranded AAV.
6. A parvoviral vector as defined in any of Items 1 to 5 wherein IGF-I is a human IGF-1.
7. A virion comprising a parvoviral vector as defined in any of Items 1 to 6.
8. A virion according to Item 7 for use as a medicament.
9. A virion as defined in Item 7 for use in a method for the treatment and/or prevention of hepatic cirrhosis or hepatic fibrosis.
10. An *in vitro* method for preparing a recombinant AAV virion comprising the steps of
   (i) contacting a cell with
      (a) a first nucleic acid sequence comprising
         i. an expression cassette comprising a sequence encoding IGF-I or a functionally equivalent variant thereof having the biological activity of IGF-I which is operably linked to a liver-specific promoter comprising the albumin gene enhancer region and the alphal-antitrypsin promoter and
         ii. an AAV 5'-ITR and a 3'-ITR flanking the expression cassette defined in (i),
      (b) a second nucleic acid sequence encoding an AAV rep protein,
      (c) a third nucleic acid sequence encoding an AAV cap protein and, optionally,
      (d) a fourth nucleic acid sequence encoding viral and/or cellular functions upon which AAV is dependent for replication,
      under conditions adequate for entry of the three components in the cell and
   (ii) recovering the recombinant AAV virion from the cells.
11. A method as defined in Item 10 wherein the cell used in the step (i) is an insect cell and wherein the first, second and third nucleic acid sequences are comprised in a baculoviral vector.
12. A method as defined in Items 10 or 11 wherein the recombinant AAV virion is a single-stranded AAV virion or a double-stranded AAV virion.
13. A method as defined in Item 12 wherein the recombinant AAV virion is a double-stranded AAV virion.
14. A method as defined in Items 10 to 13 wherein the expression cassette (a) comprises a polyadenylation signal downstream of the sequence encoding IGF-I or the functional equivalent thereof having the biological activity of IGF-I.
15. A method as defined in Items 10 to 14 wherein component (b) comprises the gene encoding the AAV2 rep gene and/or component (c) comprises the AAV1, AAV2, AAV5 or the AAV8 cap gene.

### BRIEF DESCRIPTION OF THE FIGURES

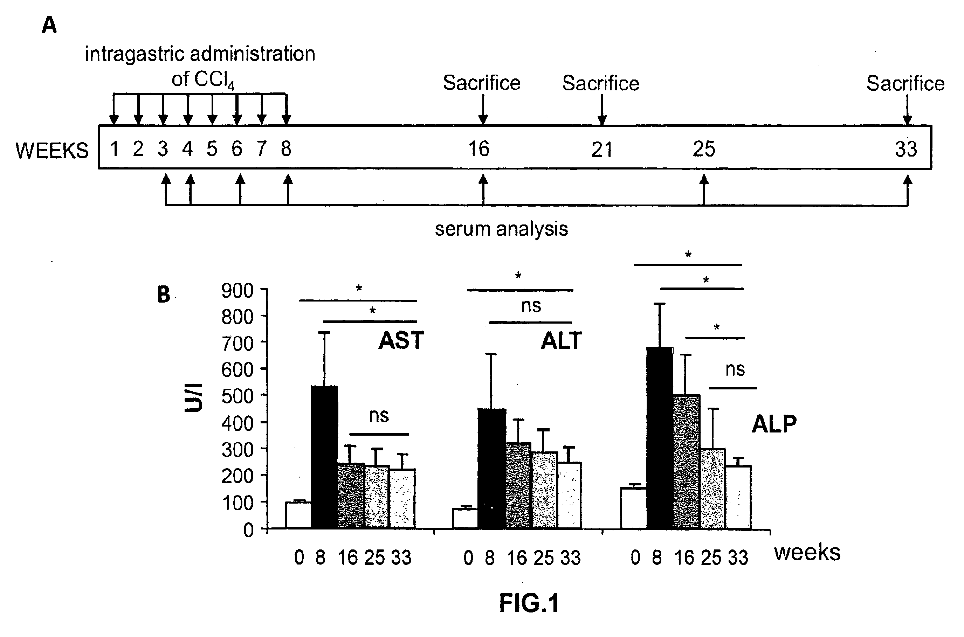

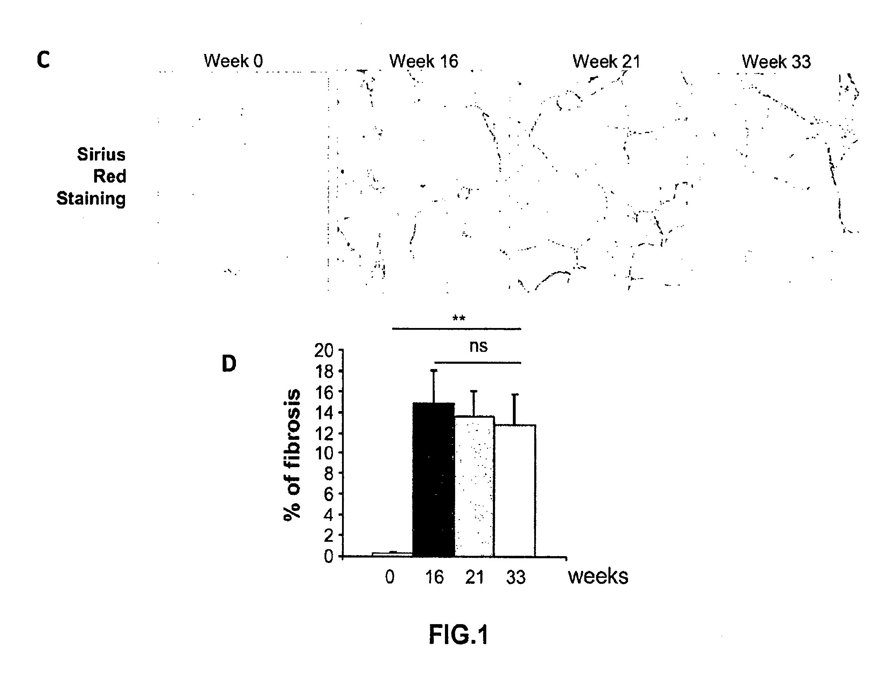

**Figure 1****. Analysis of the model of established liver cirrhosis.** A. Schematic representation of the experiment performed for the analysis of the model. B. Analysis of transaminases. Transaminases (AST, ALT and ALP) were evaluated in serum from healthy rats or from cirrhotic control animals at the indicated times. C and D. Assessment of liver fibrosis. Extracellular deposition was stained with Sirius Red (C) and quantified by image analysis (D) at the indicated times.

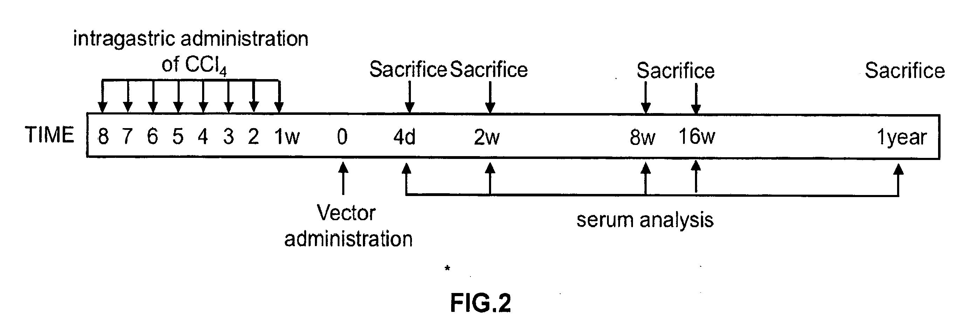

**Figure 2****. Schematic representation of the experiment performed for the evaluation of the effect of dsAAVIGF-I in liver cirrhosis.** Cirrhosis was induced for 8 weeks and animals were sacrificed and blood samples were evaluated 4 days, 2 weeks, 8 weeks, 16 weeks and 1 year after vector administration.

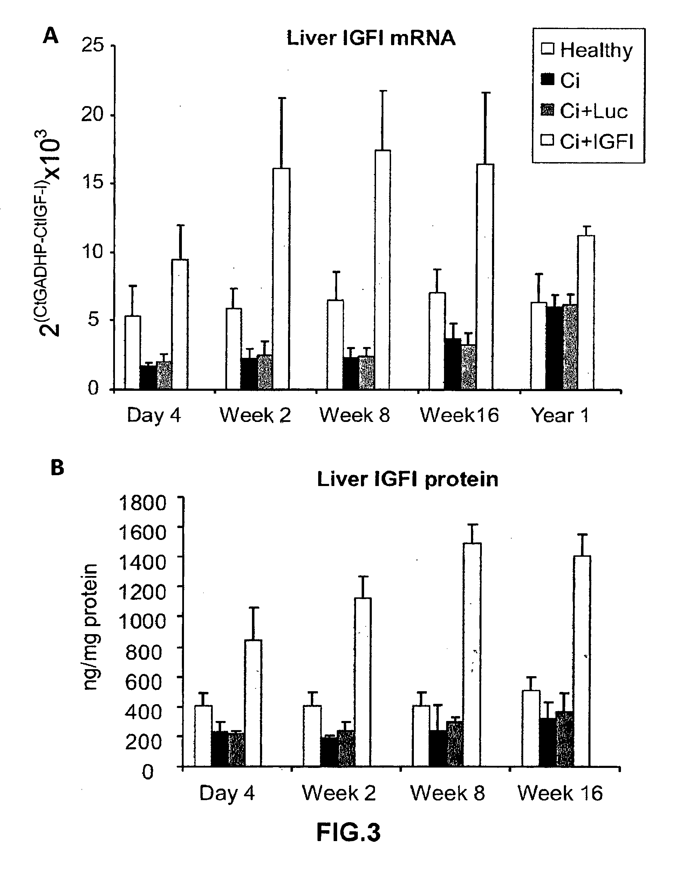

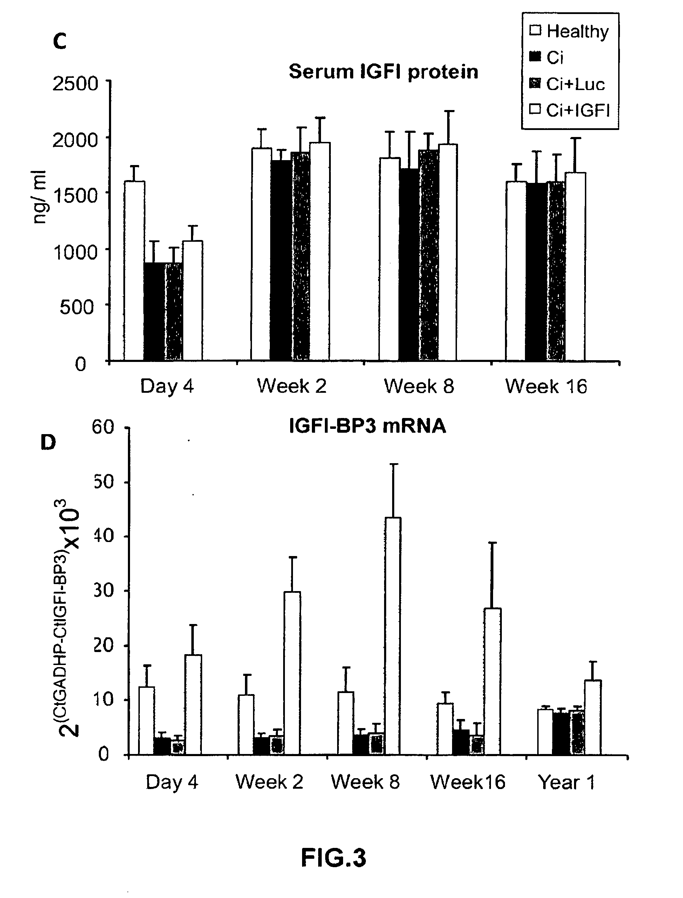

**Figure 3****. Analysis of IGF-I expression in IGF-I treated livers and controls.** IGF-I mRNA (A) and protein (B and C) were quantified by qRT-PCR (A) or ELISA (B and C) from livers (A and B) or serum (C) obtained from healthy or cirrhotic control animals (Ci) or from animals treated with dsAAVLuc (Ci+Luc) or dsAAVIGF-I (Ci+IGF-I) for the indicated times. IGFI-BP3 mRNA (D) was also quantified by RT-PCR from livers obtained from animal groups as for IGF-I above.

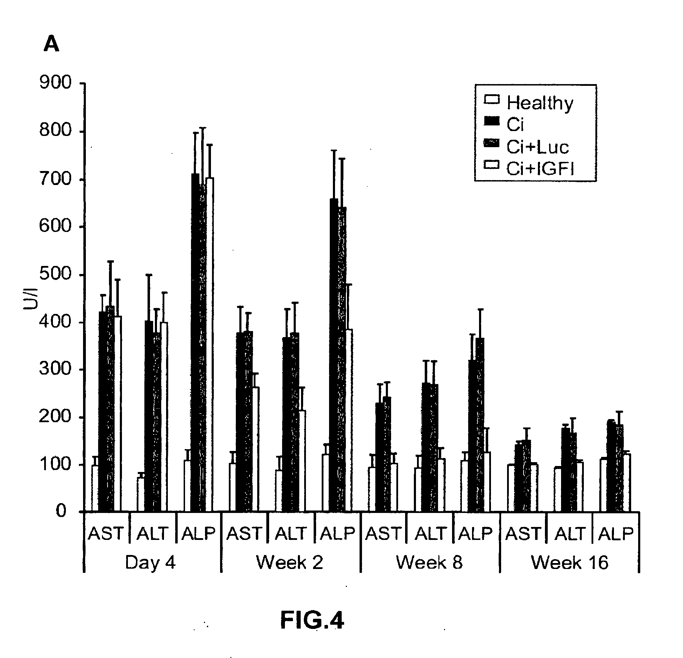

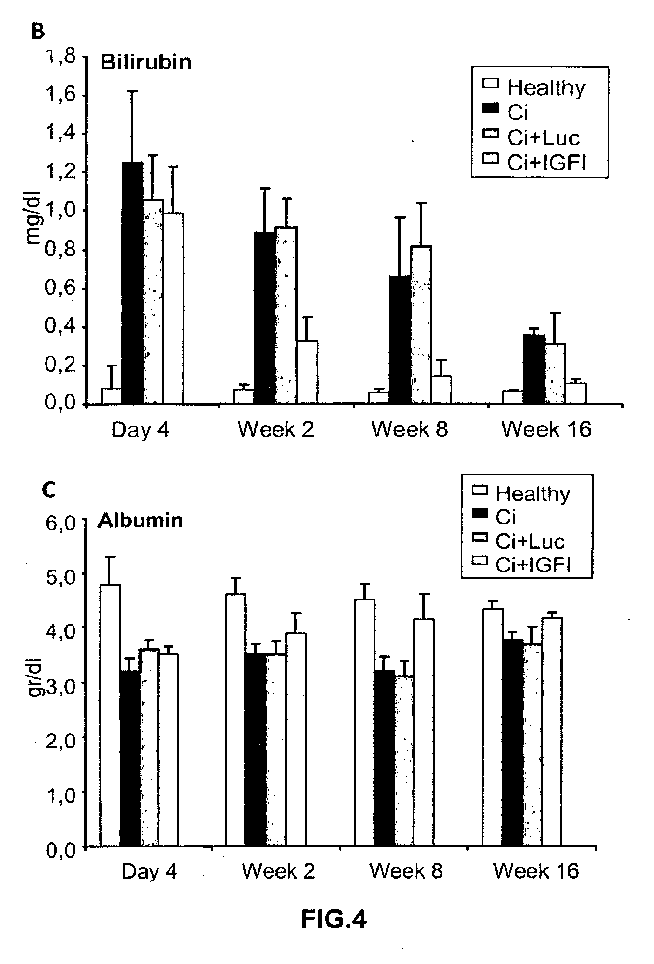

**Figure 4****. Analysis of serum parameters from IGF-I treated rats and controls.** Transaminases (AST, ALT and ALP) (A), bilirubin (B) and albumin (C) were evaluated in serum from healthy rats, from cirrhotic control animals (Ci) or from cirrhotic animals treated with dsAAVLuc (Ci+Luc) or dsAAVIGF-I (Ci+IGF-I) for the indicated times.

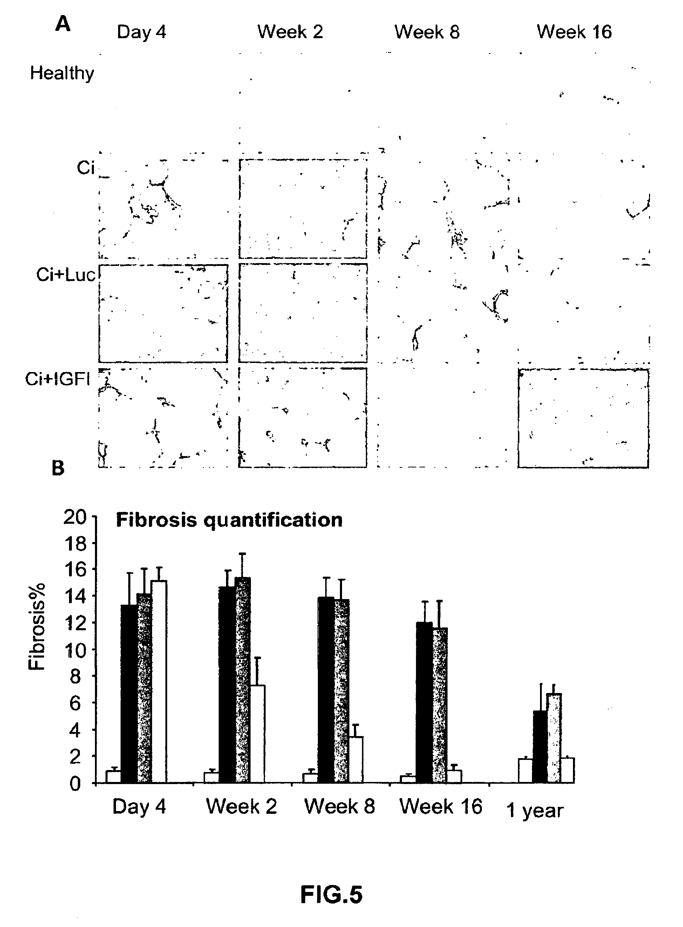

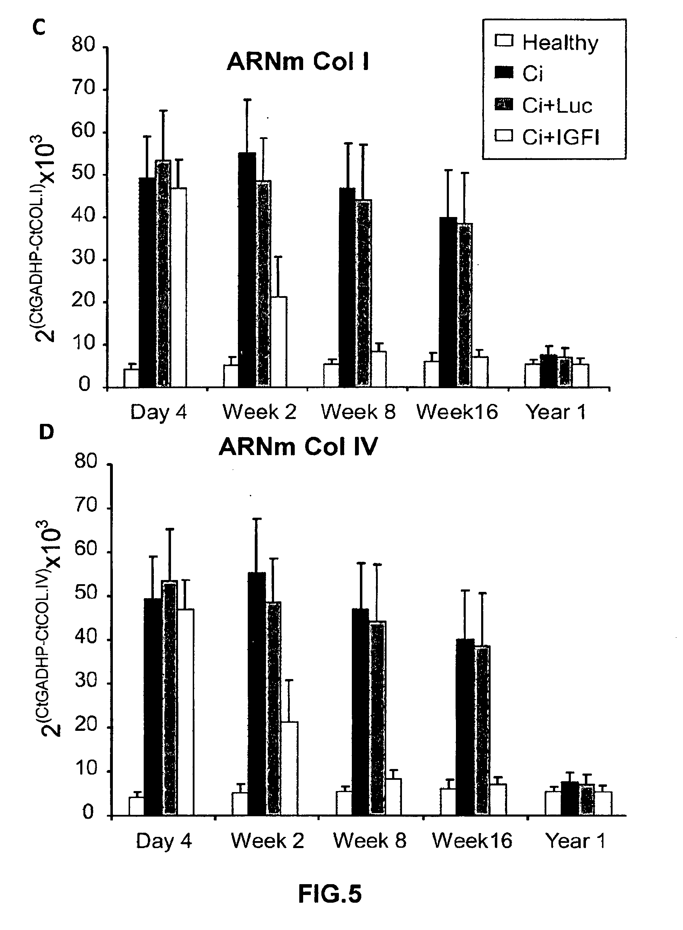

**Figure 5****. Assessment of liver fibrosis in IGF-I treated rats and controls.** Extracellular deposition was stained with Sirius Red (A) and quantified by image analysis (B). Collagen I (C) and IV (D) mRNAs were quantified by qRT-PCR. All samples were obtained from healthy rats, control cirrhotic animals (Ci) or from cirrhotic animals treated with dsAAVLuc (Ci+Luc) or dsAAVIGF-I (Ci+IGF-I) for the indicated times.

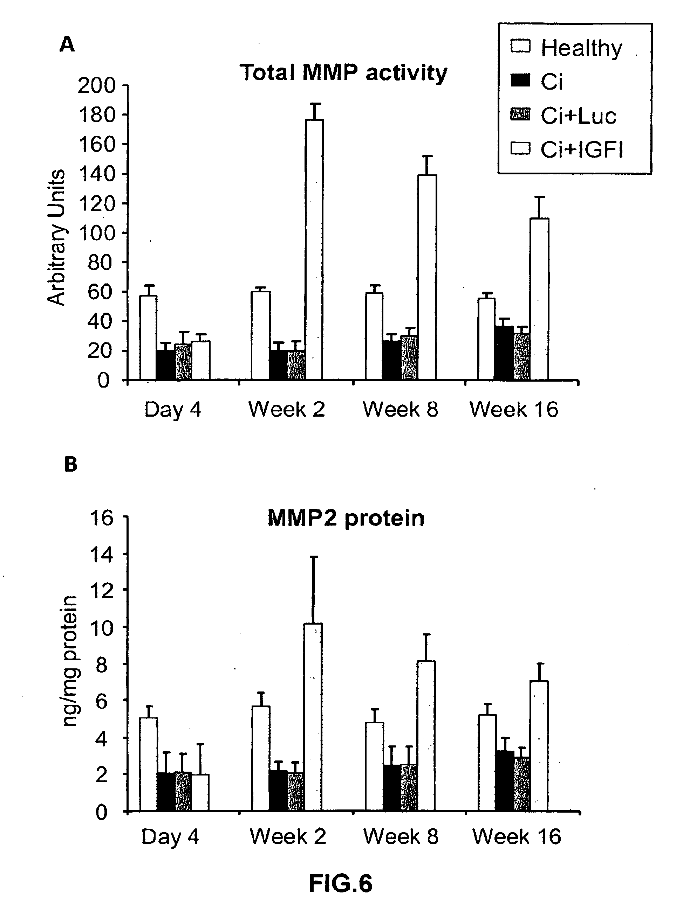

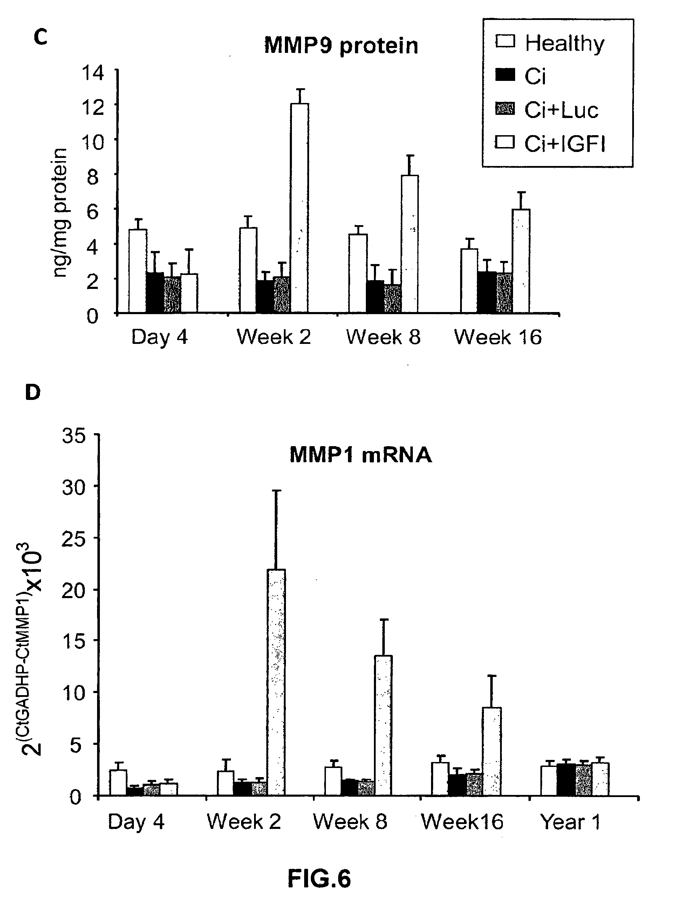

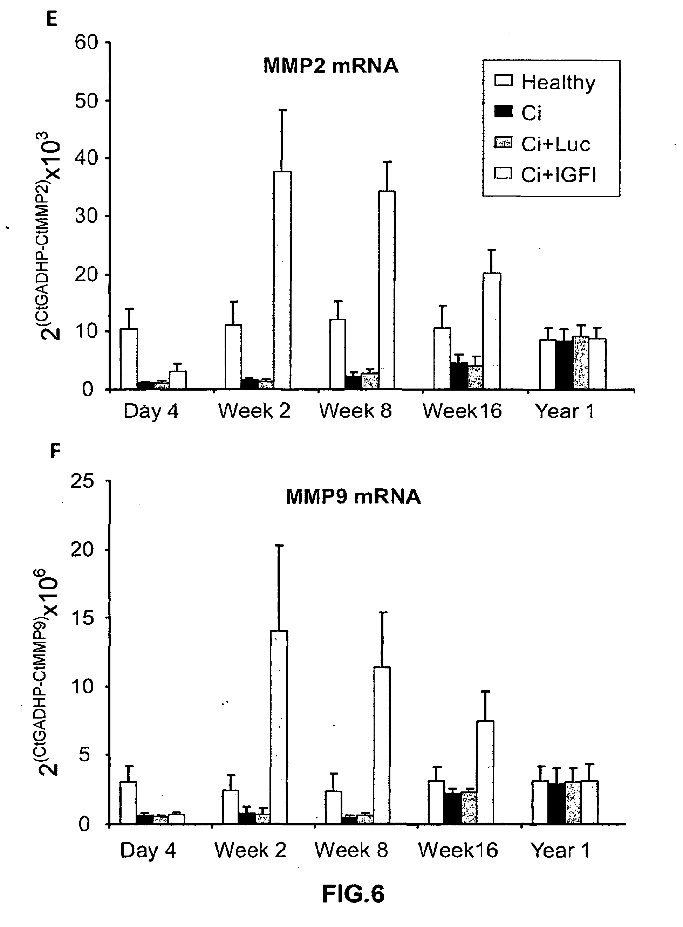

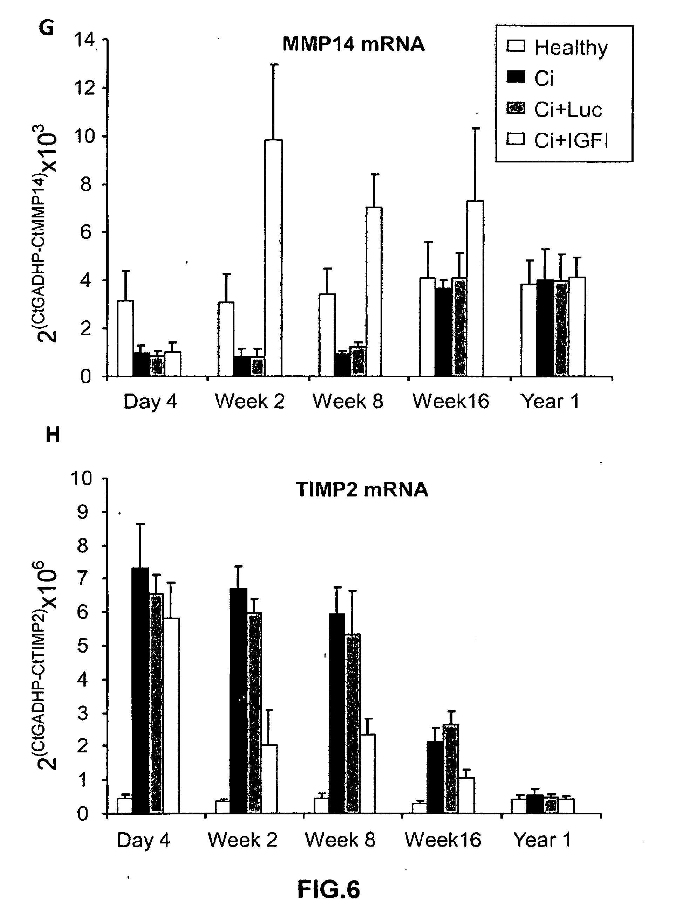

**Figure 6****. Analysis of the expression of MMPs and MMP activity in IGF-I treated livers and controls.** MMP activity was measured with a fluorogenic substrate (A), MMP2 (B) and MMP9 (C) proteins were quantified by ELISA and MMP 1 (D), 2 (E), 9 (F), 14 (G) mRNAs and TIMP2 (H) were quantified by qRT-PCR from healthy livers or from cirrhotic livers obtained from control animals (Ci) or from animals treated with dsAAVLuc (Ci+Luc) or dsAAVIGF-I (Ci+IGF-I) for the indicated times.

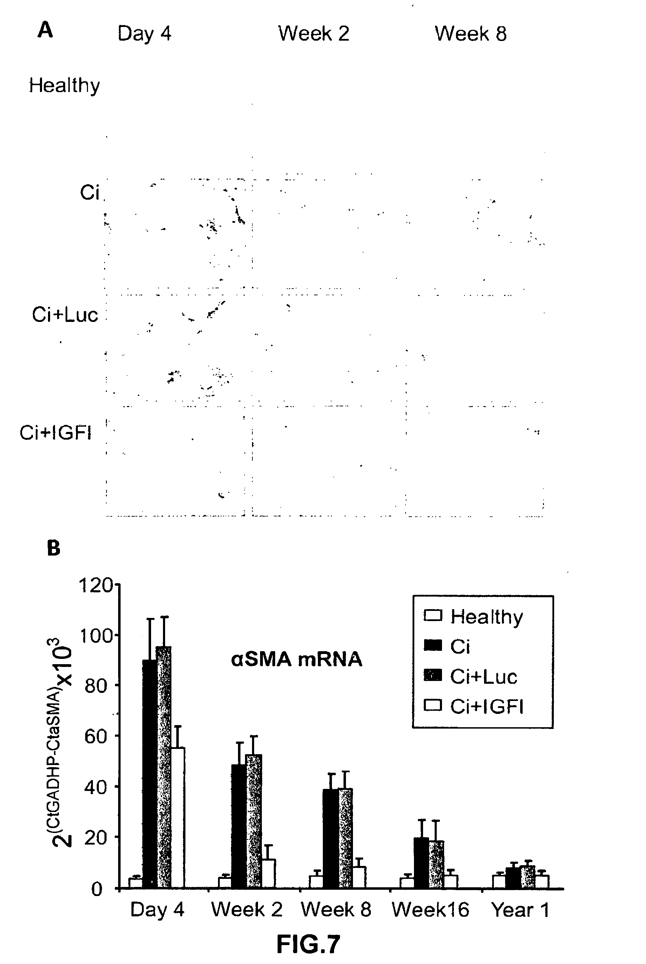

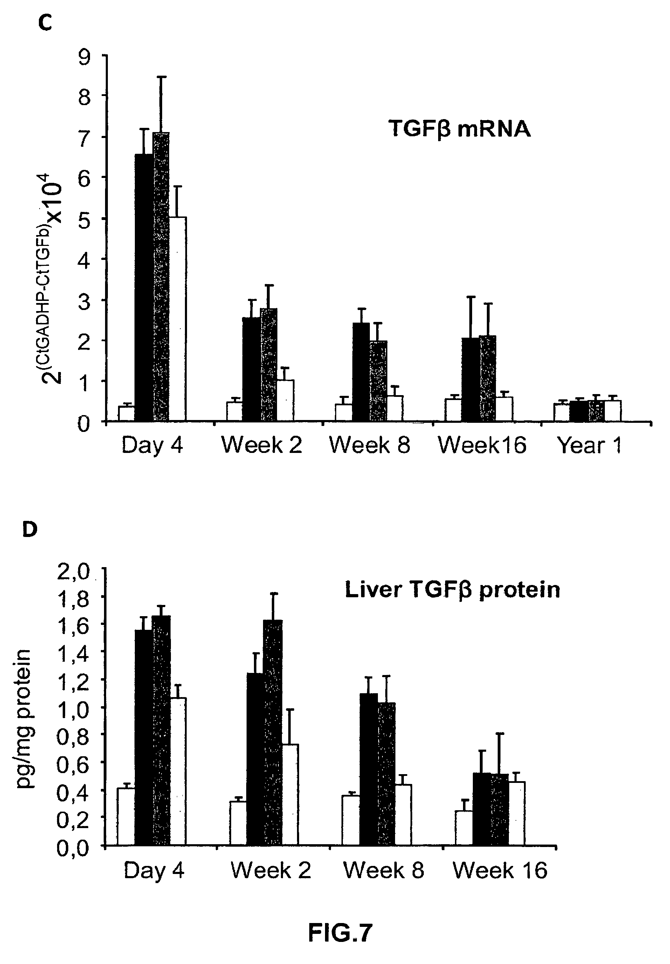

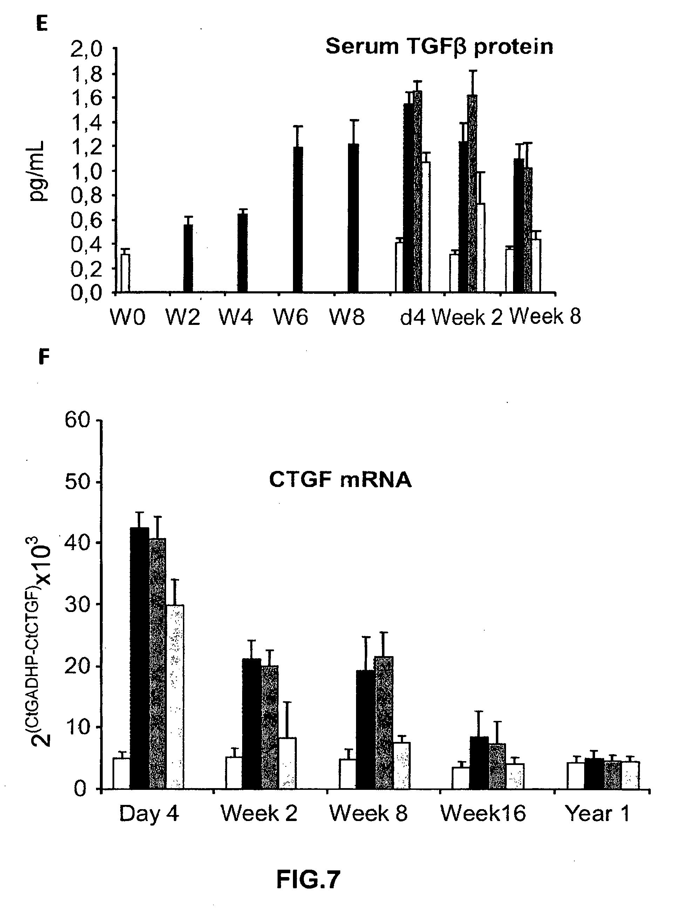

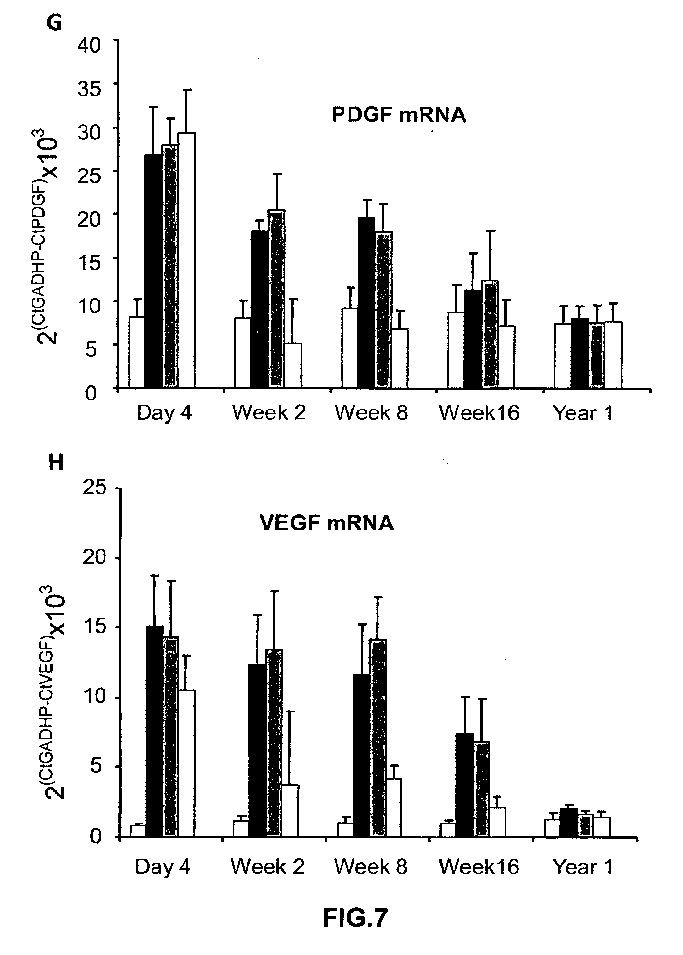

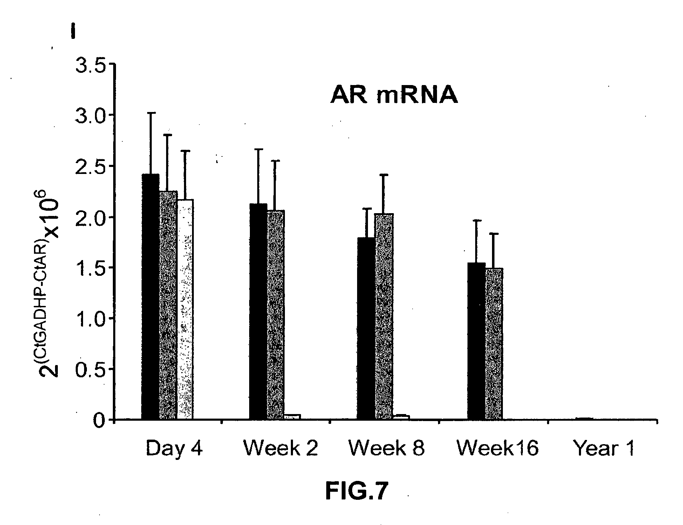

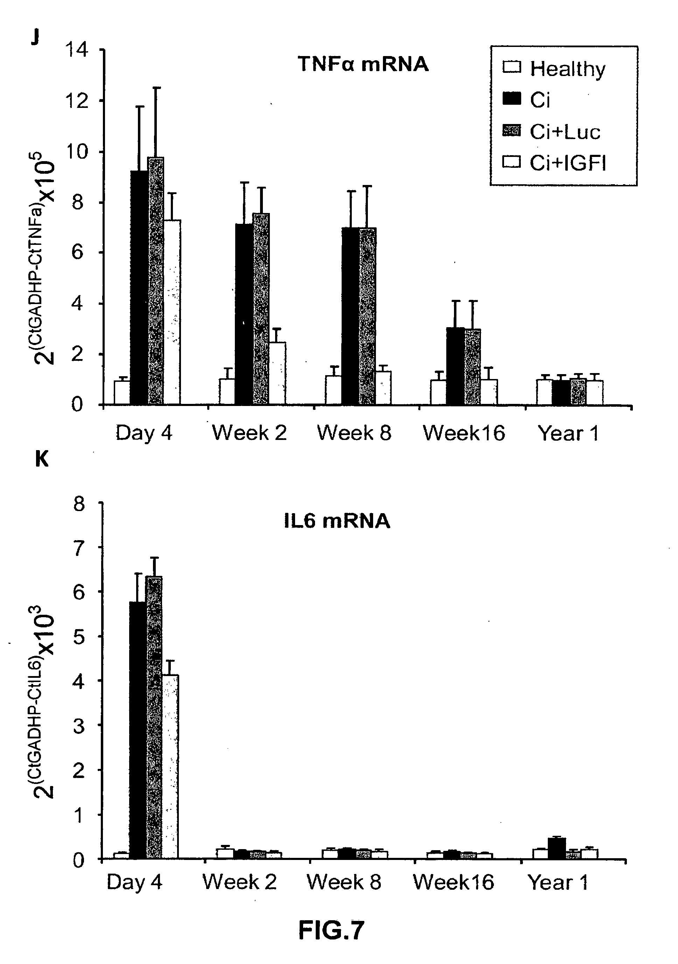

**Figure 7****. Analysis of profibrogenic factors, TNF**α **and IL6 in IGF-I treated animals and controls.** αSMA protein (A) and mRNA (B) were detected by immunohistochemistry (A) or quantified by qRT-PCR (B) from healthy livers or cirrhotic livers from control animals (Ci) or from animals treated with dsAAVLuc (Ci+Luc) or dsAAVIGF-I (Ci+IGF-I) for the indicated times. In these animals, TGFβ mRNA (C) and protein (D and E) were also analyzed in liver (C and D) or blood (E) and liver CTGF (F), PDGF (G), VEGF (H), amphiregulin (AR, I), TNFα (J) and IL6 (K) mRNAs were quantified by qRT-PCR.

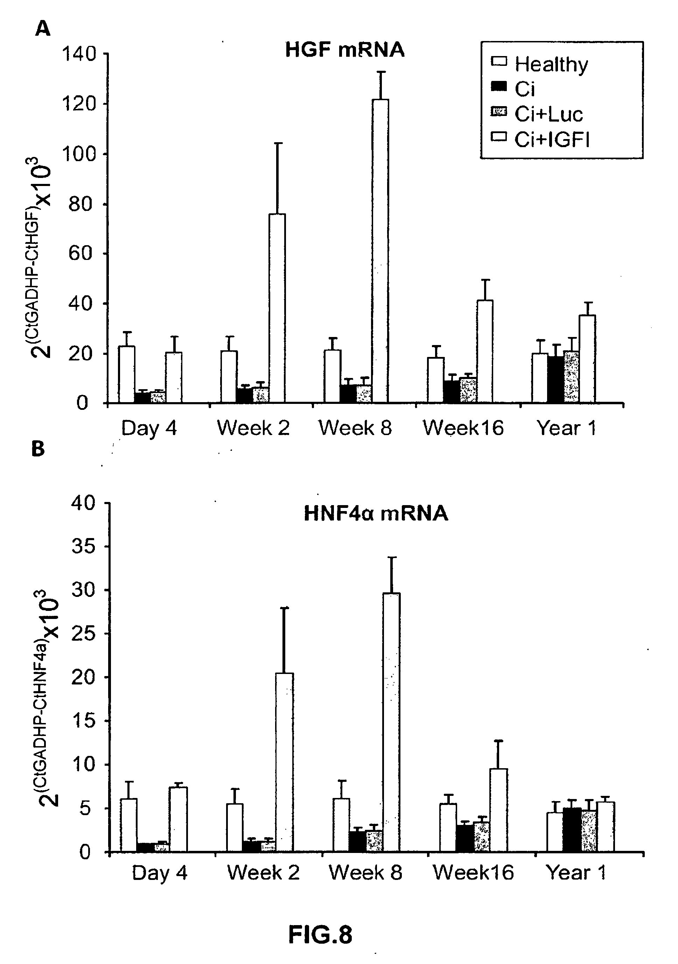

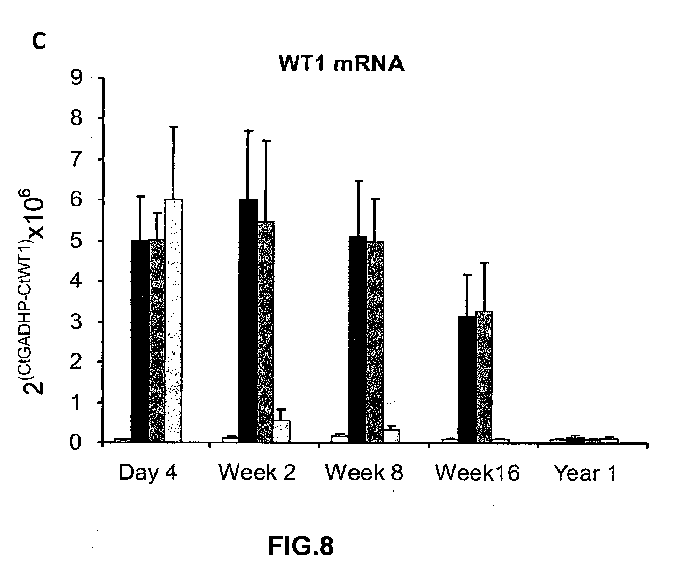

**Figure 8****. Analysis of HGF, HNF4**α **and WT in IGF-I treated animals and controls.** HGF (A), HNF4α (B) and WT-1 mRNA (C) were quantified by qRT-PCR from healthy livers or cirrhotic livers from control animals (Ci) or from animals treated with dsAAVLuc (Ci+Luc) or dsAAVIGF-I (Ci+IGF-I) at the indicated times.

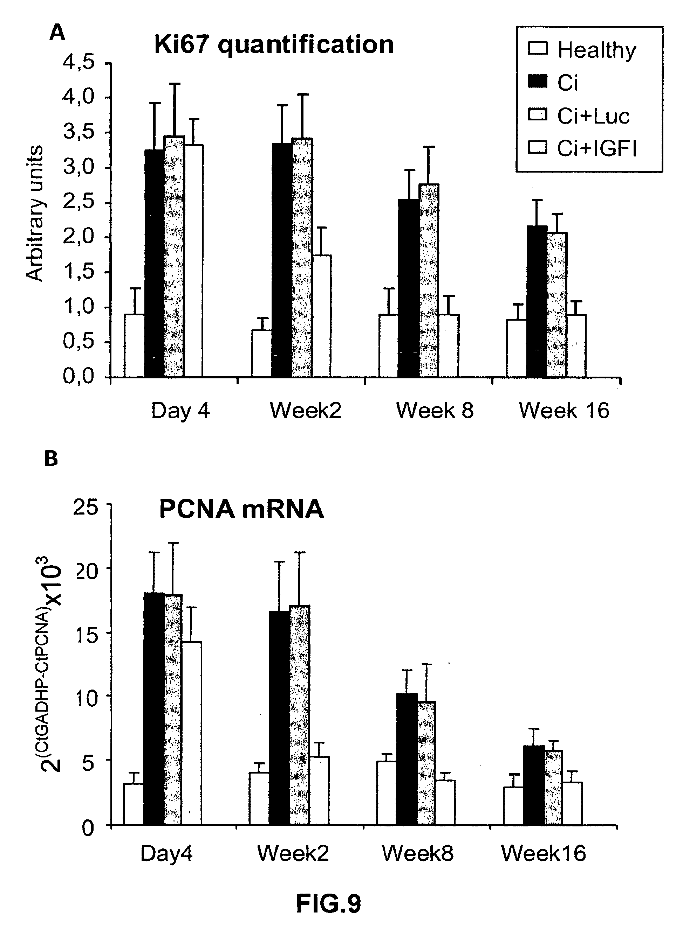

**Figure 9****. Analysis of proliferation in IGF-I treated animals and controls.** Ki67 staining was quantified from histological samples (A) and PCNA mRNA was quantified by qRT-PCR from healthy livers or cirrhotic livers from control animals (Ci) or from animals treated with dsAAVLuc (Ci+Luc) or dsAAVIGF-I (Ci+IGF-I) for the indicated times.

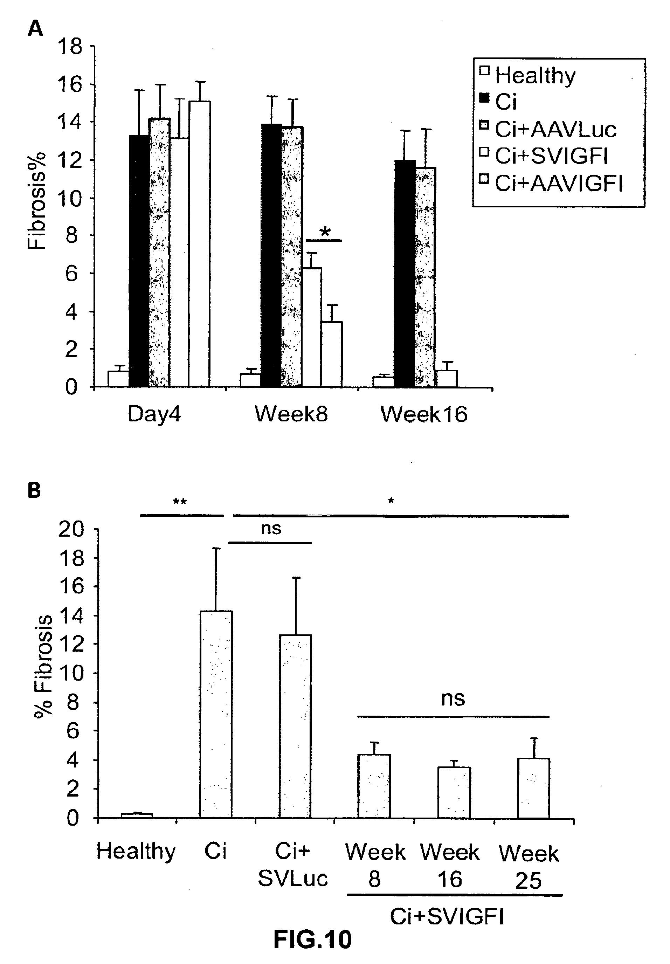

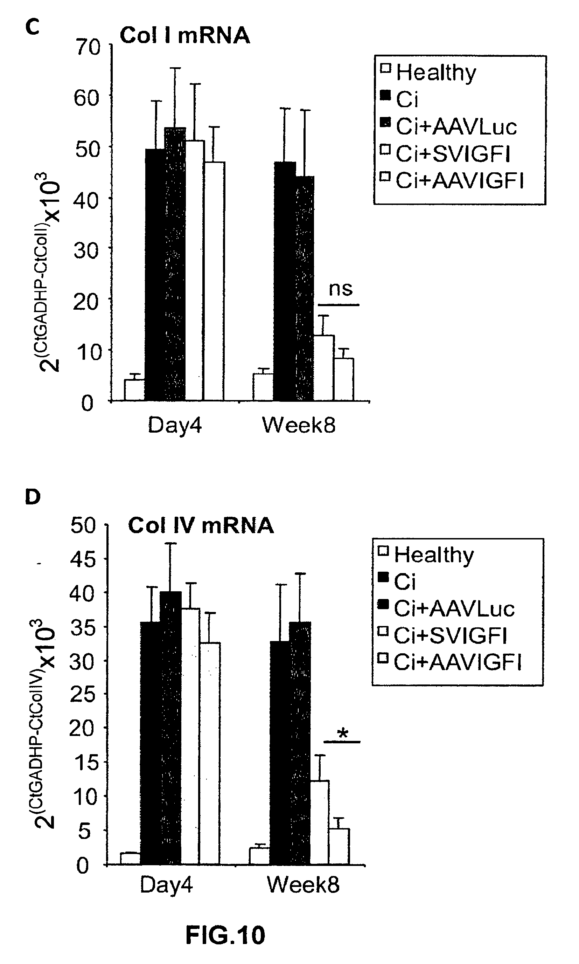

**Figure 10****. Assessment of liver fibrosis in rats treated with SVIGF-I, dsAAV-IGF-I and controls.** Extracellular deposition was stained with Sirius Red and quantified by image analysis (A and B). Collagen I (C) and IV (D) mRNAs were quantified by qRT-PCR. All samples were obtained from healthy rats, control cirrhotic animals (Ci) or from cirrhotic animals treated with dsAAVLuc (Ci+AAVLuc), dsAAVIGF-I (Ci+AAVIGF-I), SVLuc (Ci+SVLuc), or SVIGF-I (Ci+SVIGF-I) for the indicated times.

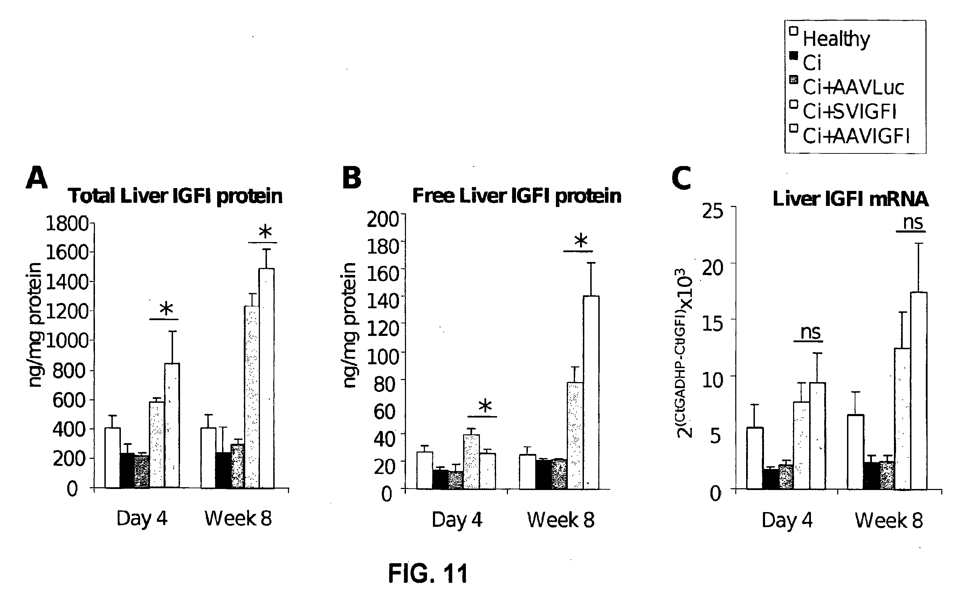

**Figure 11****. Analysis of IGF-I expression in rats treated with SVIGF-I, dsAAV-IGF-I and controls.** Total (A) and free (B) IGF-I protein and mRNA (C) were quantified by Elisa (A and B) or qRT-PCR (C) from livers from healthy rats, control cirrhotic animals (Ci) or from cirrhotic animals treated with dsAAVLuc (Ci+AAVLuc), SVIGF-I (Ci+SVIGF-I), or dsAAVIGF-I (Ci+AAVIGF-I) for the indicated times.

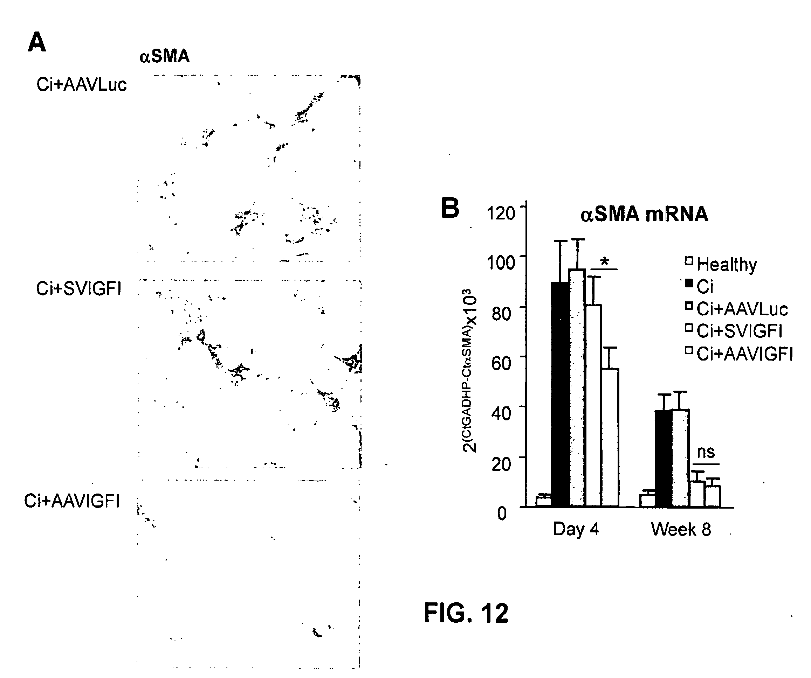

**Figure 12****. Analysis of activated HSCs in rats treated with SVIGF-I, AAV-IGF-I and controls.** αSMA protein (A) and mRNA (B) were detected by immunohistochemistry (A) or quantified by qRT-PCR (B) from livers from healthy rats, control cirrhotic animals (Ci) or from cirrhotic animals treated with dsAAVLuc (Ci+AAVLuc), SVIGF-I (Ci+SVIGF-I), or dsAAVIGF-I (Ci+AAVIGF-I) for 4 days (A and B) or 8 weeks (B).

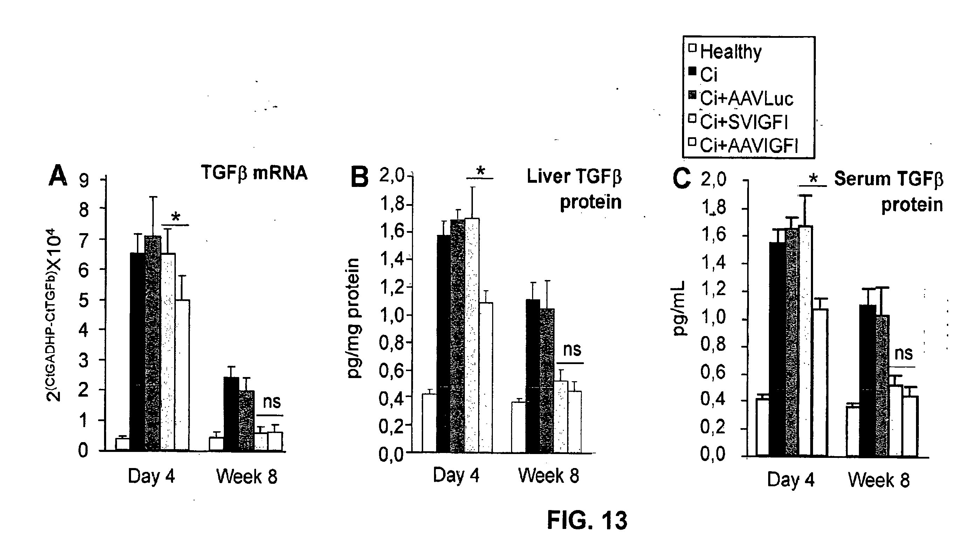

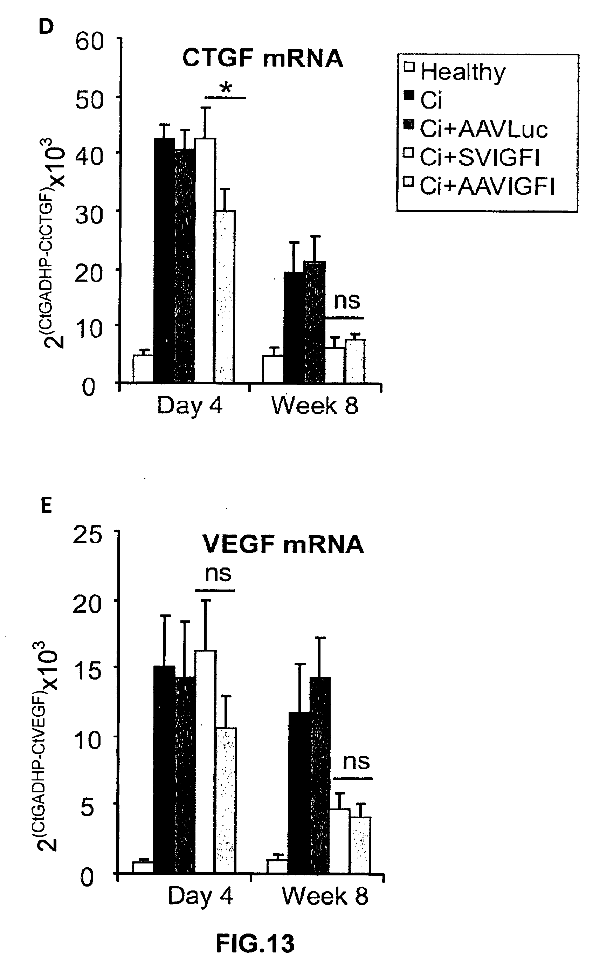

**Figure 13****. Analysis of TGF**β**, CTGF and VEGF in rats treated with SVIGF-I, dsAAV-IGF-I and controls.** CTGFβ mRNA (A) and protein (B and C) were quantified by qRT-PCR (A) or ELISA (B and C) from livers (A and B) or serum (C) obtained from healthy rats, control cirrhotic animals (Ci) or from cirrhotic animals treated with dsAAVLuc (Ci+AAVLuc), SVIGF-I (Ci+SVIGF-I), or dsAAVIGF-I (Ci+AAVIGF-I) for 4 days or 8 weeks. In these animals CTGF (D) and VEGF (E) mRNAs were also quantified by qRT-PCR.

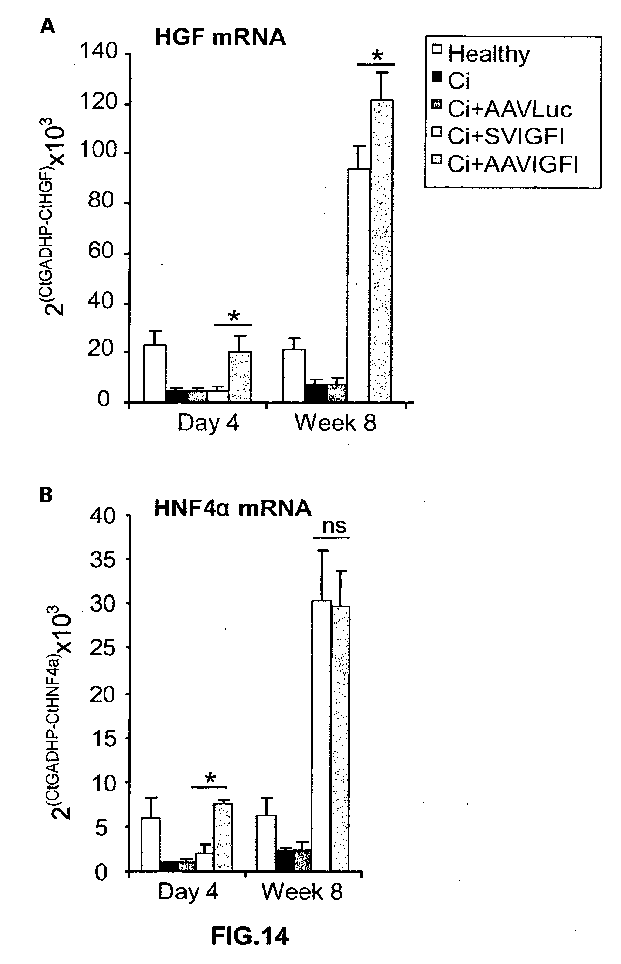

**Figure 14****. Analysis of HGF and HNF4**α **in rats treated with SVIGF-I, dsAAV-TGF-I and controls.** HGF (A) and HNF4α (B) mRNAs were quantified by qRT-PCR from livers isolated from healthy rats, control cirrhotic animals (Ci) or from cirrhotic animals treated with dsAA VLuc (Ci+AAVLuc), SVIGF-I (Ci+SVIGF-I), or dsAAVIGF-I (Ci+AAVIGF-I) for 4 days or 8 weeks.

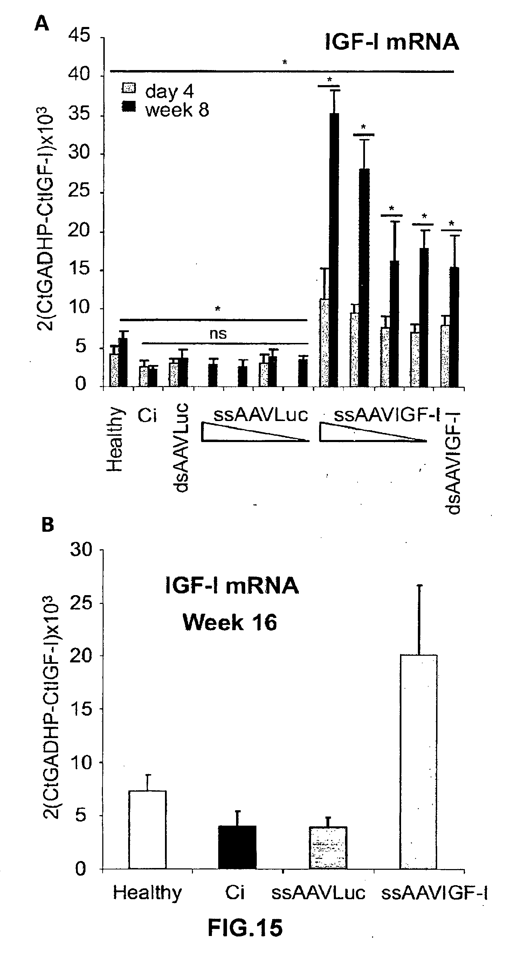

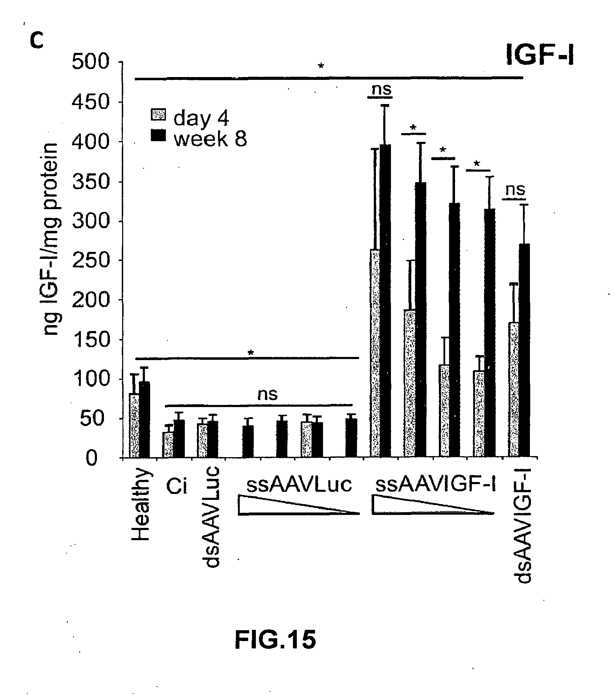

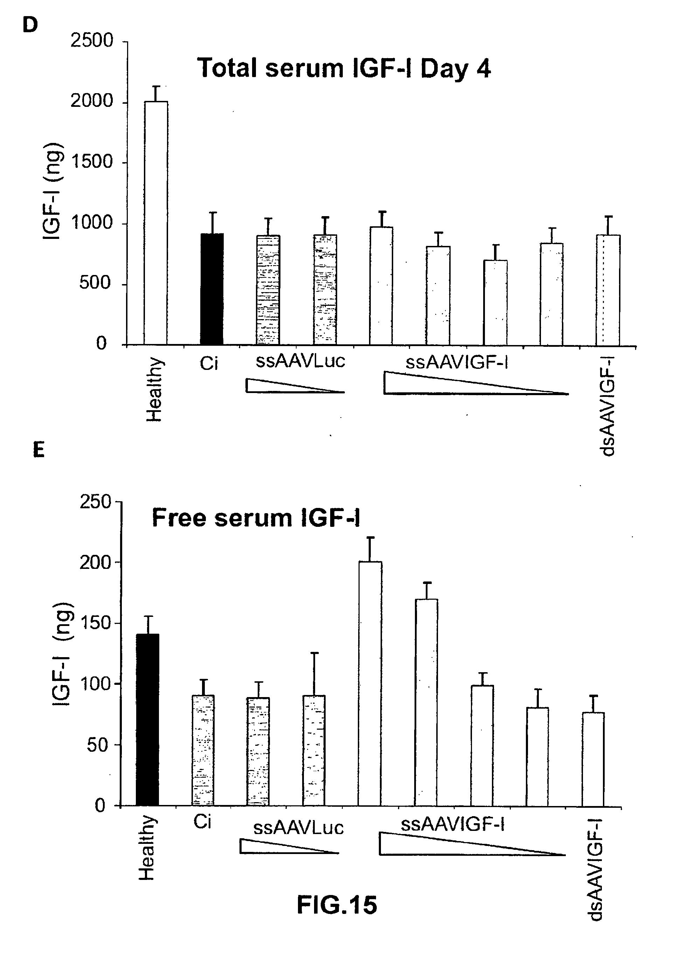

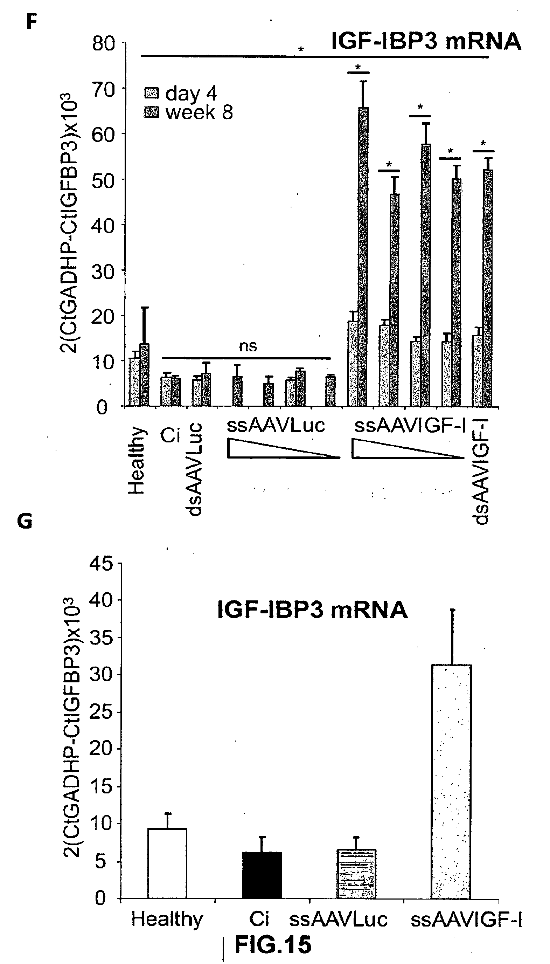

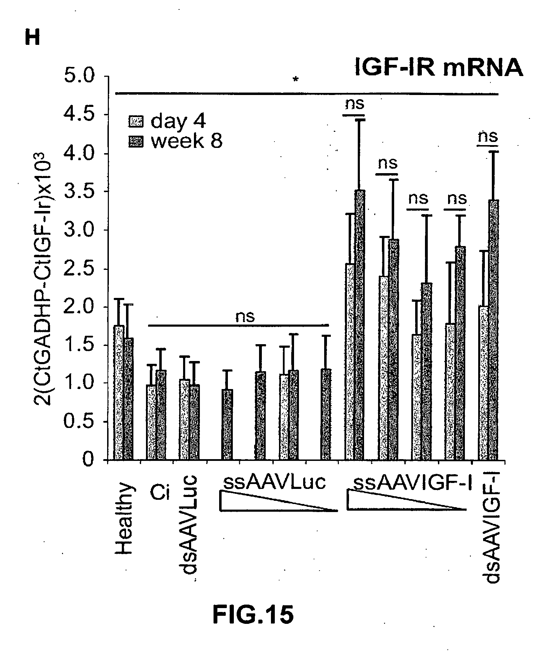

**Figure 15****. Analysis of liver IGF-I expression and activity. A:** IGF-I mRNA expression levels in liver extracts, 4 days or 8 weeks after treatment. **B:** IGF-I mRNA expression levels in liver extracts 16 weeks after vector administration, (low dose of 4.8 x 10¹⁰ vp/rat). **C:** IGF-I protein levels in liver extracts. **D and E:** Total serum IGF-I (D) amd Free serum IGF-I (E) levels quantified 4 days after treatment; in the case of ssAAVLuc, represented IGF-I levels correspond to the high dose 1.2 x 10¹² vp/rat and the very low dose 9.7 x 10⁹ vp/rat. **F:** IGF-IBP3 mRNA expression levels in liver extracts, 4 days or 8 weeks after treatment. **G:** IGF-IBP3 mRNA expression levels in liver extracts 16 weeks after vector administration, (low dose 4.8 x 10¹⁰ vp/rat). **H:** IGF-I receptor (IGF-IR) mRNA expression levels in liver extracts. All mRNA expression levels were quantified by qRT-PCR and IGF-I protein levels were quantified by ELISA. Triangles indicate doses in decreasing order.

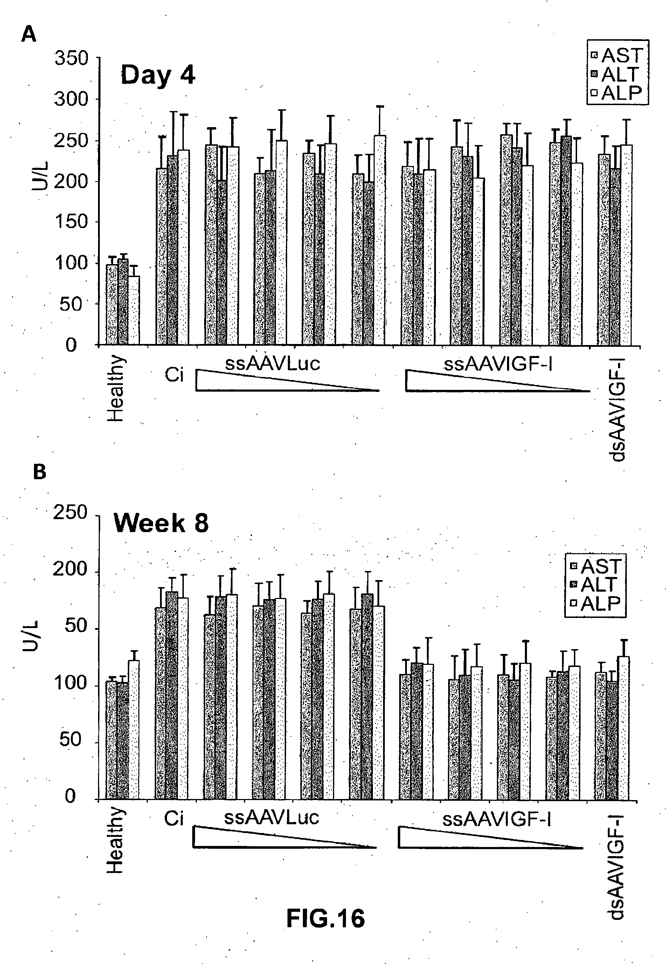

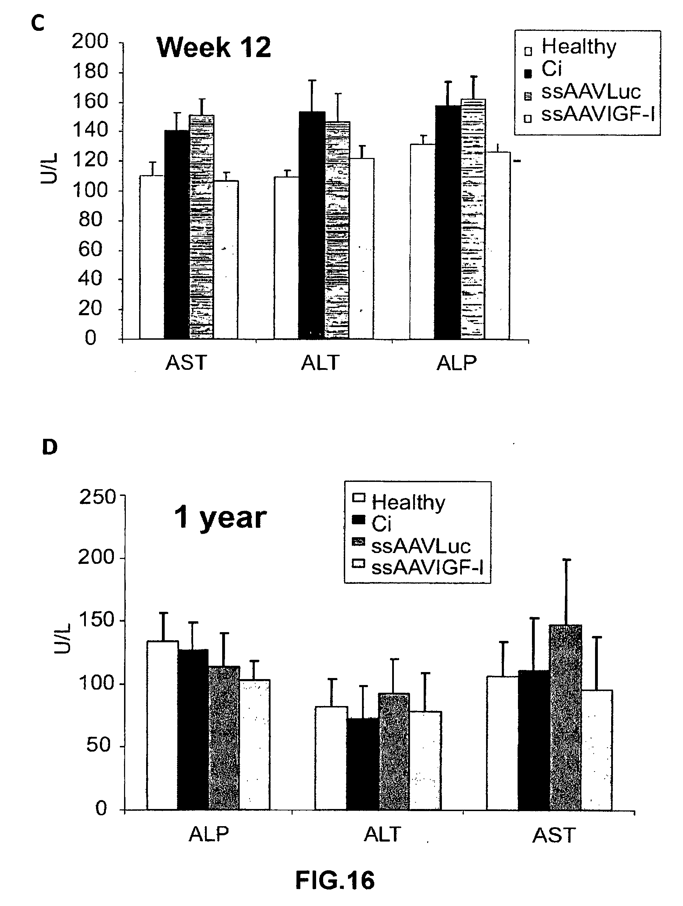

**Figure 16****. Analysis of serum transaminases.** Transaminases levels (AST, ALT and ALP) quantified in the serum 4 days (A), 8 weeks (B), 12 weeks (C), and 1 year (D) after vector administration. For week 12, represented levels correspond to the low dose of vector (4.8 x 10¹⁰ vp/rat). For 1. year, represented level correspond to the high dose of vector (1.2 x 10¹² vp/rat). Triangles indicate doses in decreasing order.

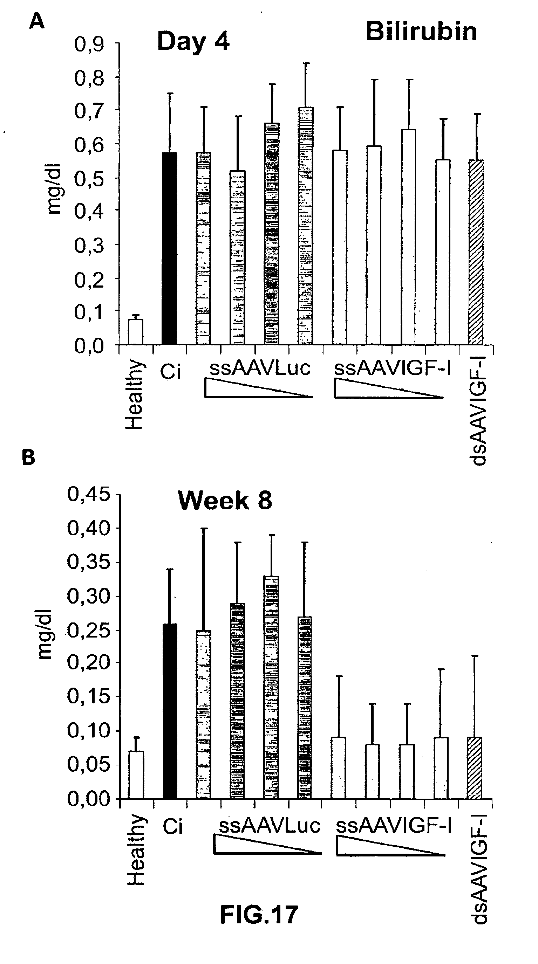

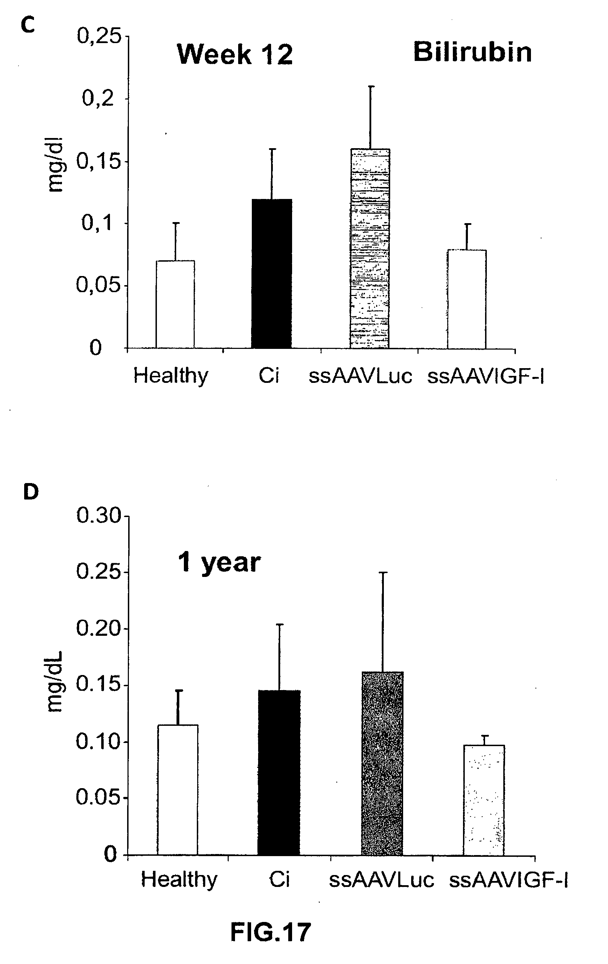

**Figure 17****. Analysis of serum bilirubin.** Bilirubin levels quantified in the serum 4 days (A), 8 weeks (B), 12 weeks (C), and 1 year (D) after vector administration. For week 12, represented levels correspond to the low dose of vector (4.8 x 10¹⁰ vp/rat). For 1 year, represented levels correspond to the high dose of vector (1.2 x 10¹² vp/rat). Triangles indicate doses in decreasing order.

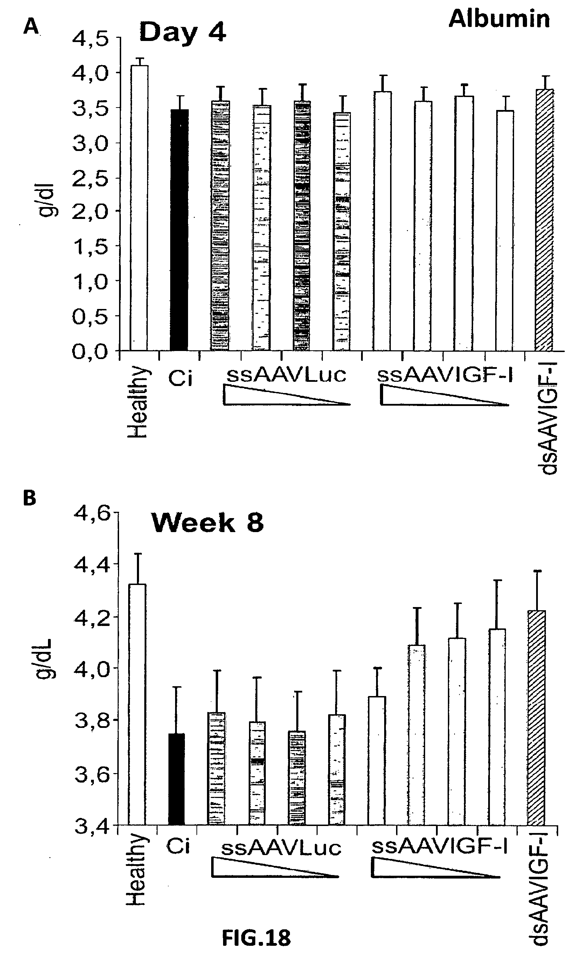

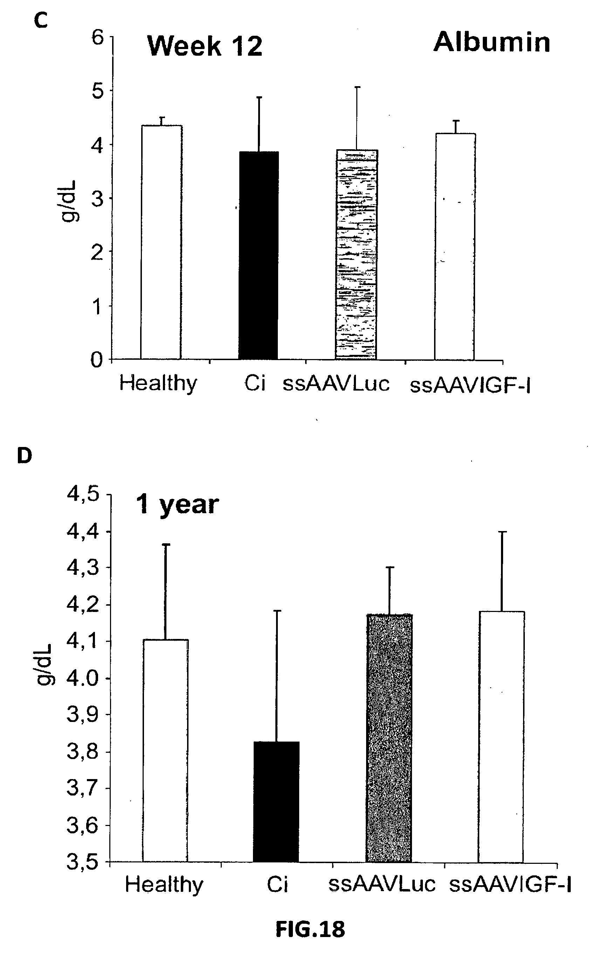

**Figure 18****. Analysis of serum Albumin.** Albumin levels quantified in the serum 4 days (A), 8 weeks (B), 12 weeks (C), and 1 year (D) after vector administration. For week 12, represented levels correspond to low dose of vector (4.8 x 10¹⁰ vp/rat). For 1 year, represented levels correspond to high dose of vector (1.2 x 10¹² vp/rat). Triangles indicate doses in decreasing order.

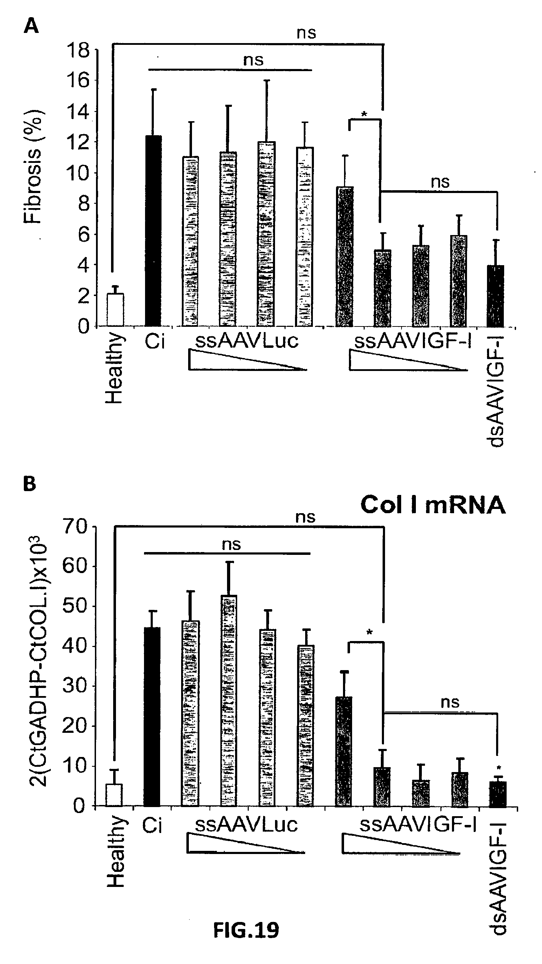

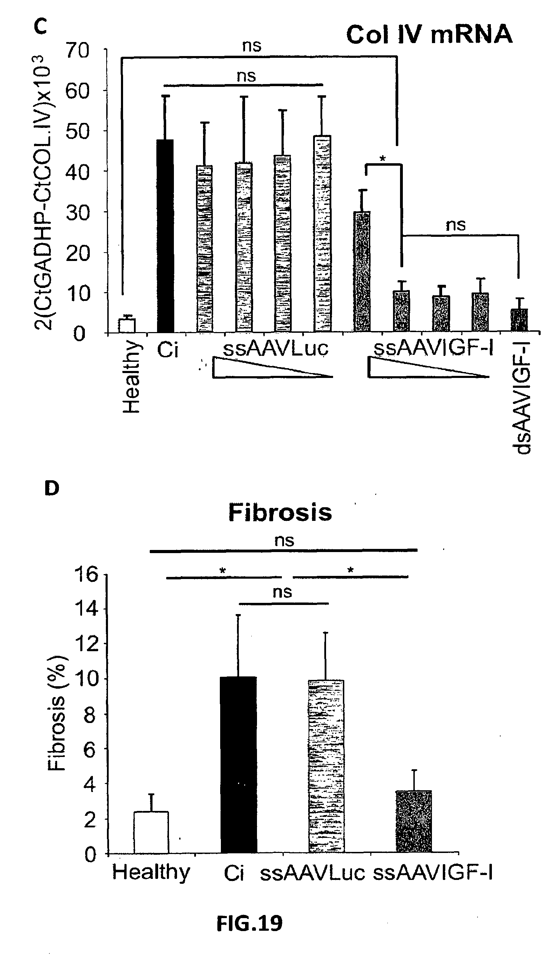

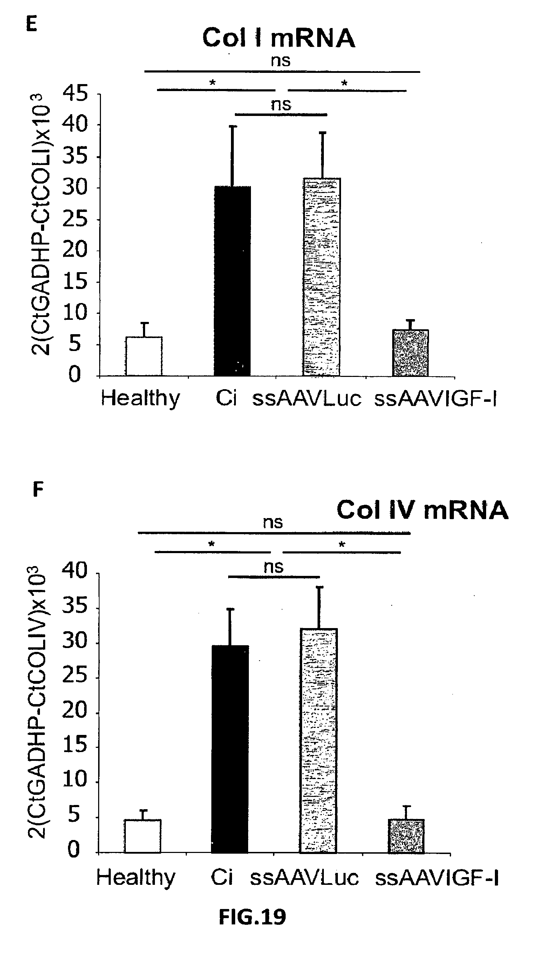

**Figure 19****. Assessment of liver fibrosis.** Liver fibrosis was evaluated by: image analysis quantification of extracellular deposition in tissue samples stained with Sirius Red (A and C); and quantification of Collagen I (B and D) and IV (C and F) mRNA expression levels in liver tissue by qRT-PCR. Represented data correspond to samples taken 8 weeks after treatment (A-C), and 16 weeks after treatment (D-F). For the later case, represented data correspond to the low dose of ssAAVLuc and ssAAVIGF-I. Triangles indicate doses in decreasing order.

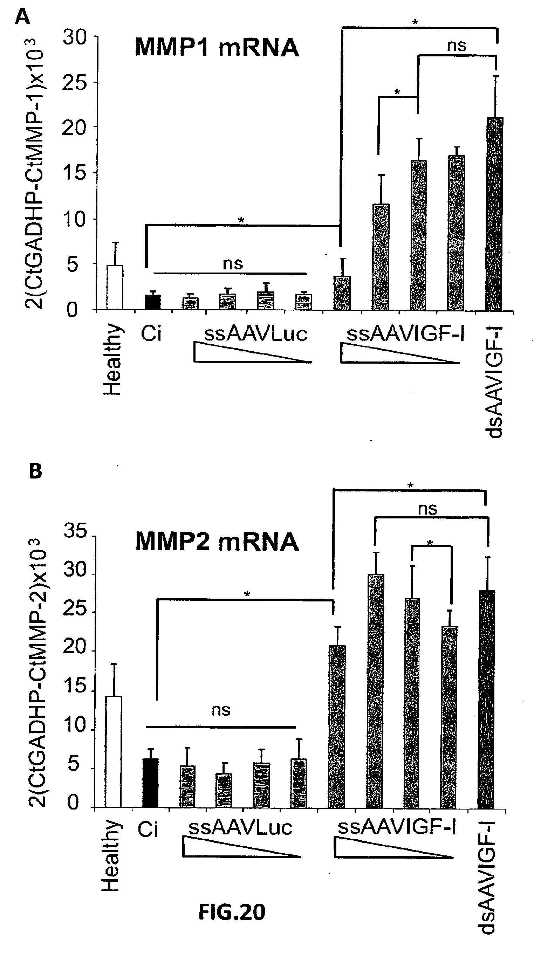

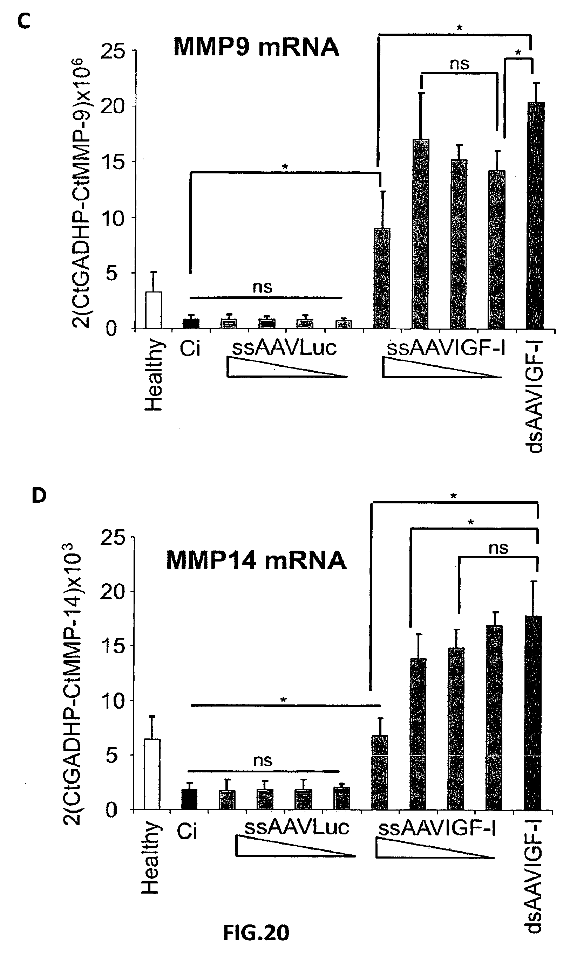

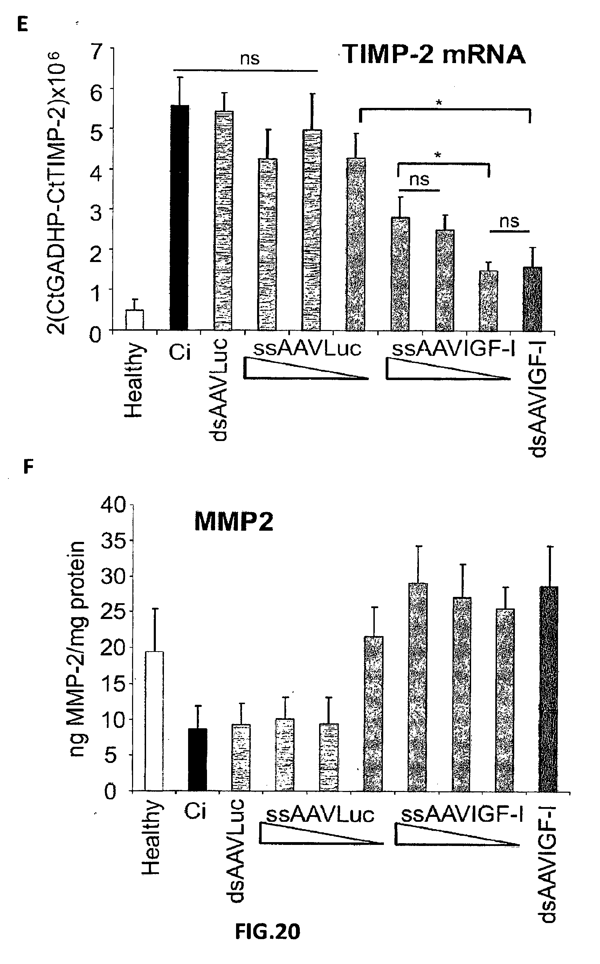

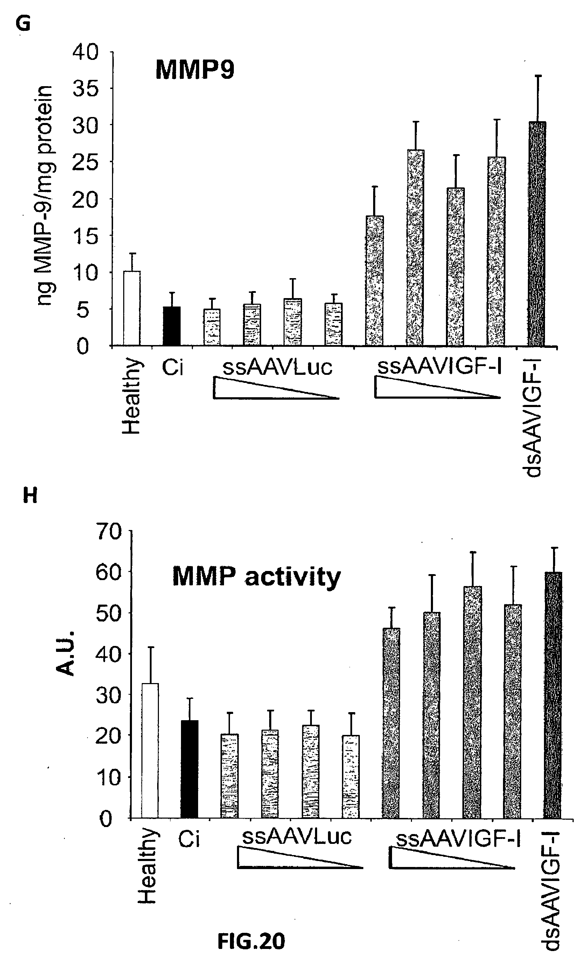

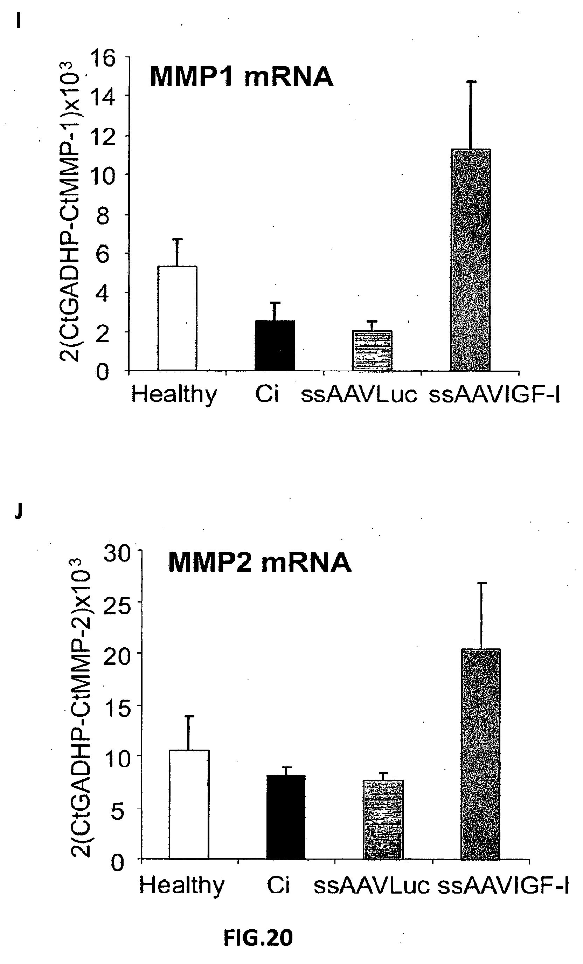

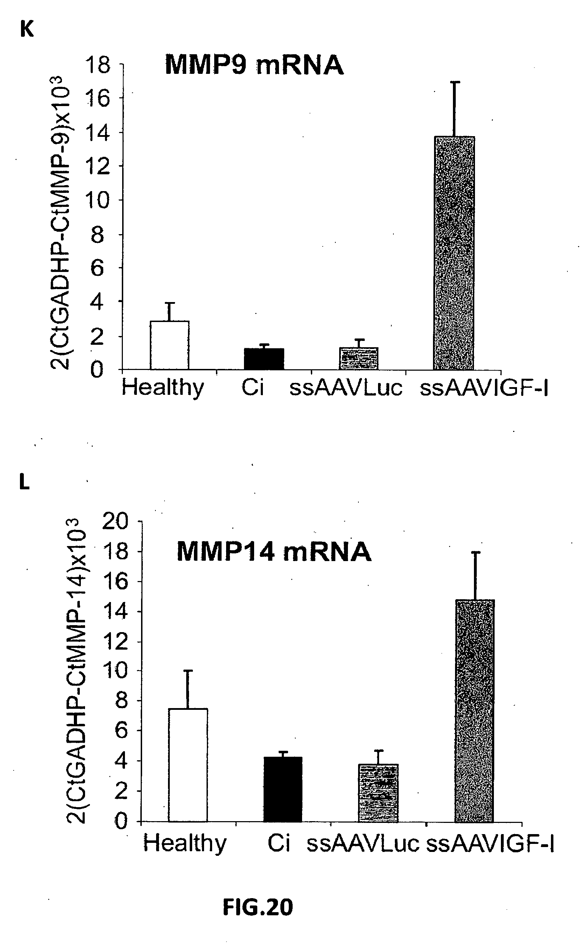

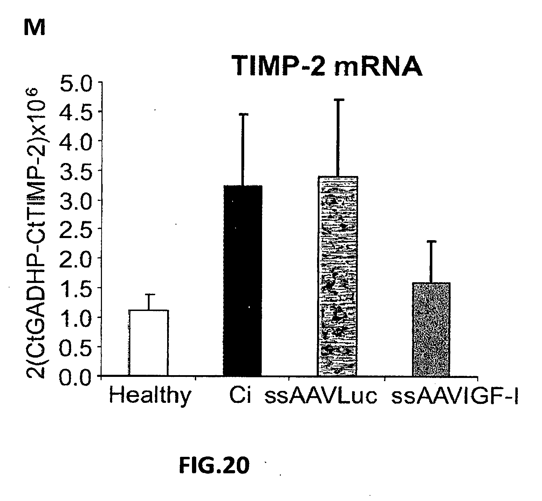

**Figure 20****. Analysis of MMPs and MMP inhibitors.** Liver extract samples were obtained 8 weeks (A-H), or 16 weeks after treatment (I-M). For E and F represented data for ssAAVIGF-I correspond to the high dose and the very low dose. For the later case, represented data correspond to the low dose (4.8 x 10¹⁰ vp/rat) of ssAAVLuc and ssAAVIGF-I. mRNA expression for MMP1 (A, I) MMP2 (B, J), MMP9 (C, K), MMP14 (D, L) and TIMP-2 (E, M) was quantified by qRT-PCR. MMP2 (F) and MMP9 (G) protein levels were quantified by ELISA. Total MMP activity was also evaluated (H). Triangles indicate doses in decreasing order.

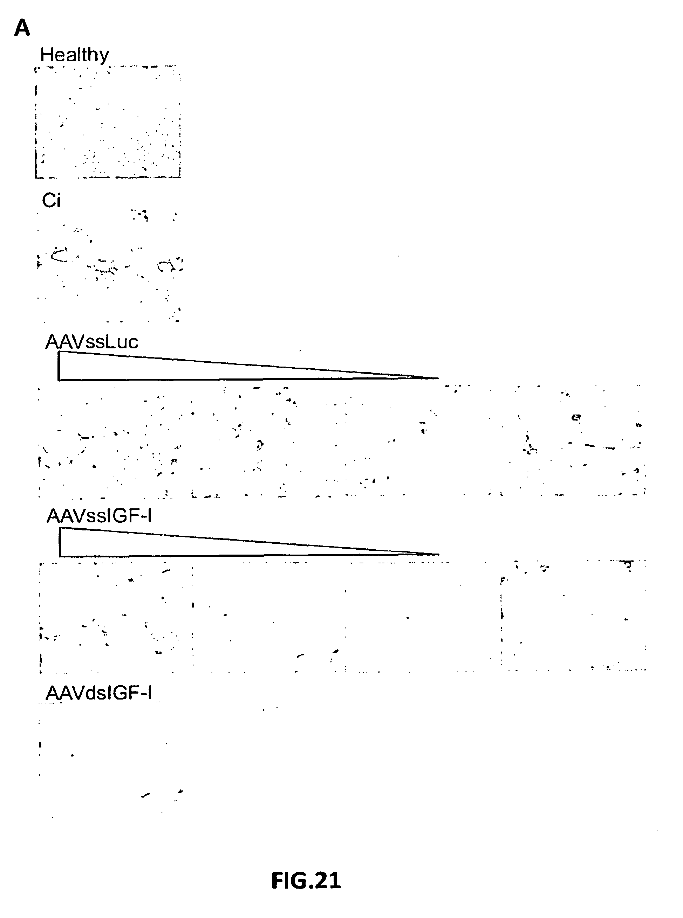

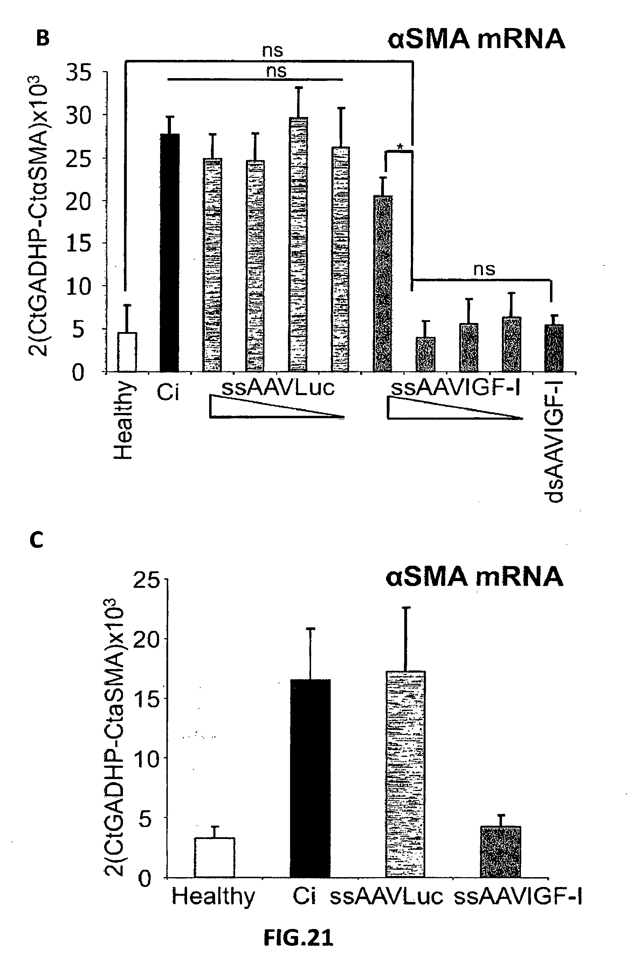

**Figure 21****. Analysis of hepatic stellate cells (HSCs).** αSMA expression was analysed in liver samples obtained 8 weeks (A-B), or 16 weeks after treatment (C). For the later case, represented data correspond to the low dose of ssAA VLuc and ssAA VIGF-I. **A:** Localization of αSMA in hepatic tissue by αSMA specific immunohistochemistry. **B and C:** αSMA mRNA expression quantified by qRT-PCR Triangles indicate doses in decreasing order.

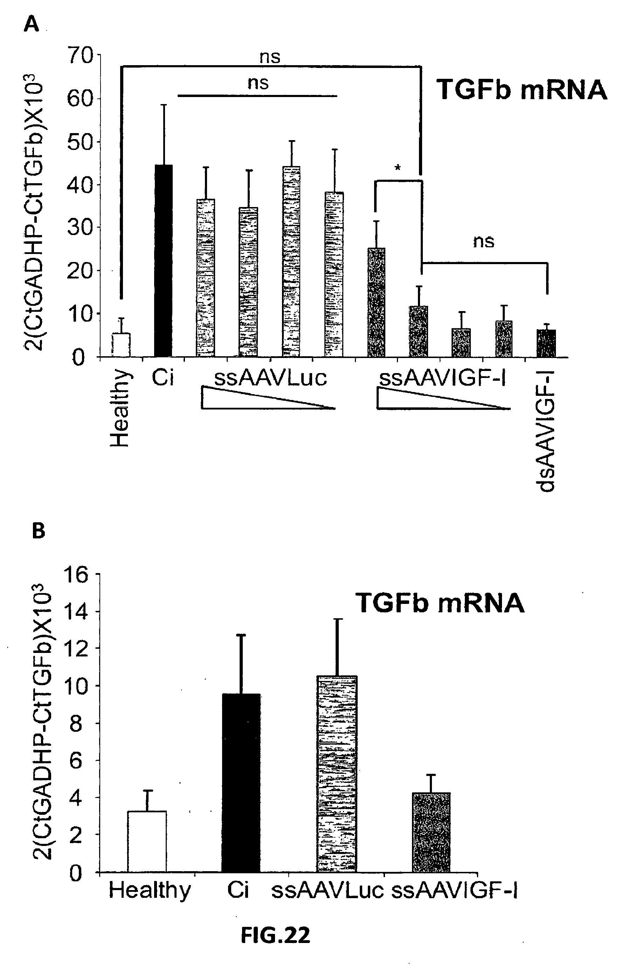

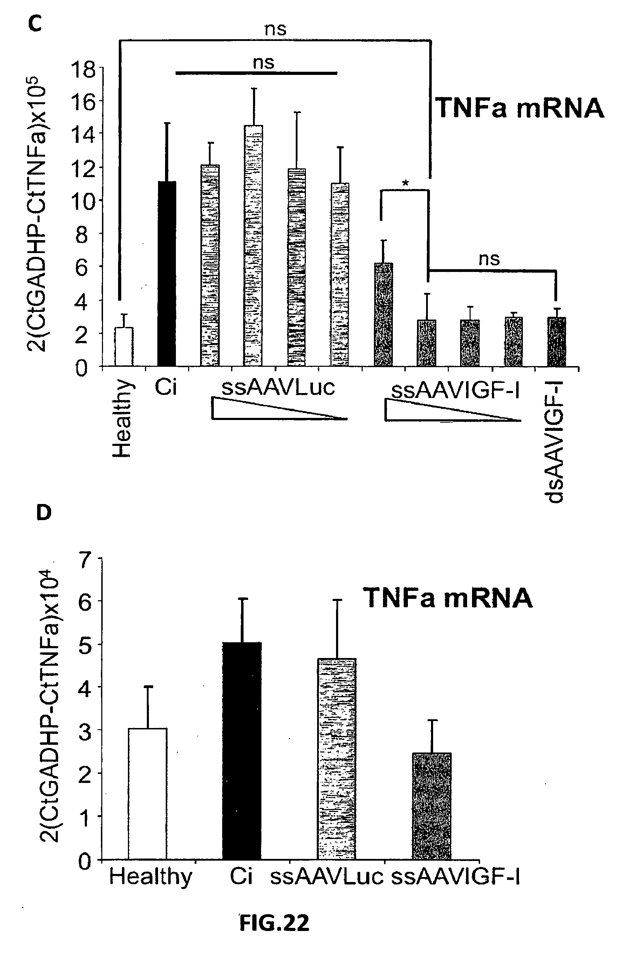

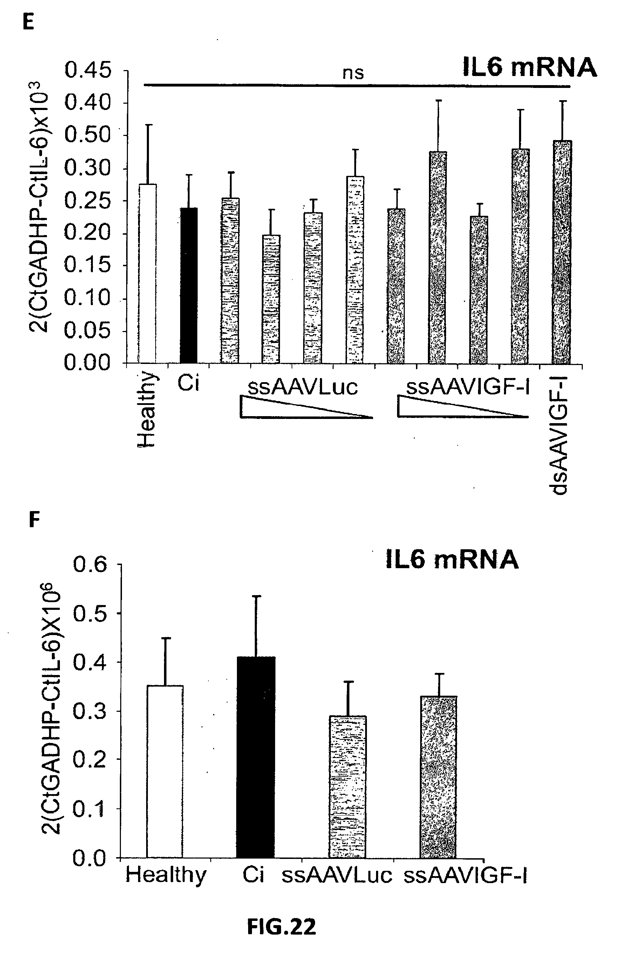

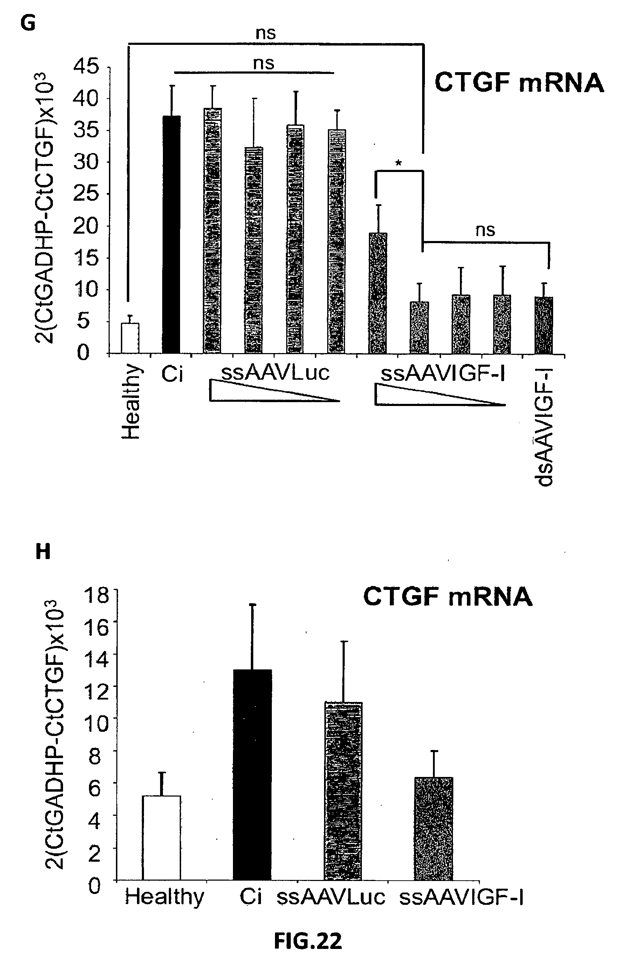

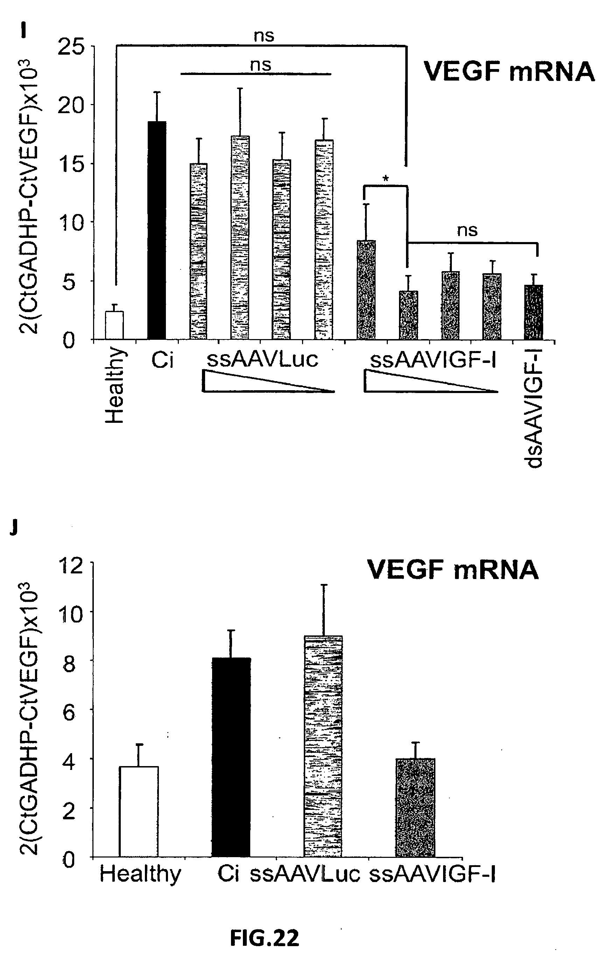

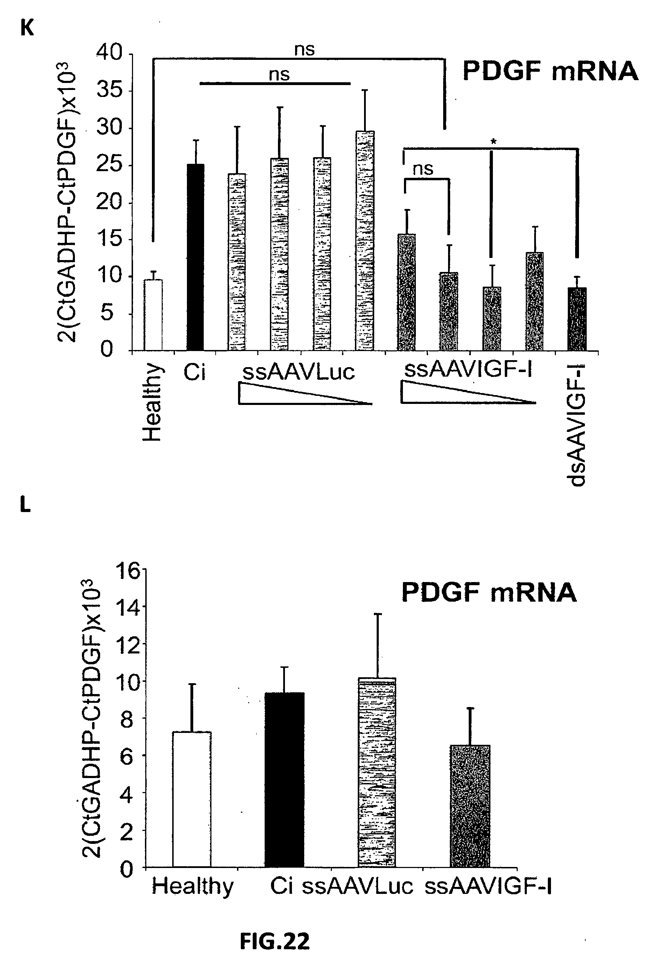

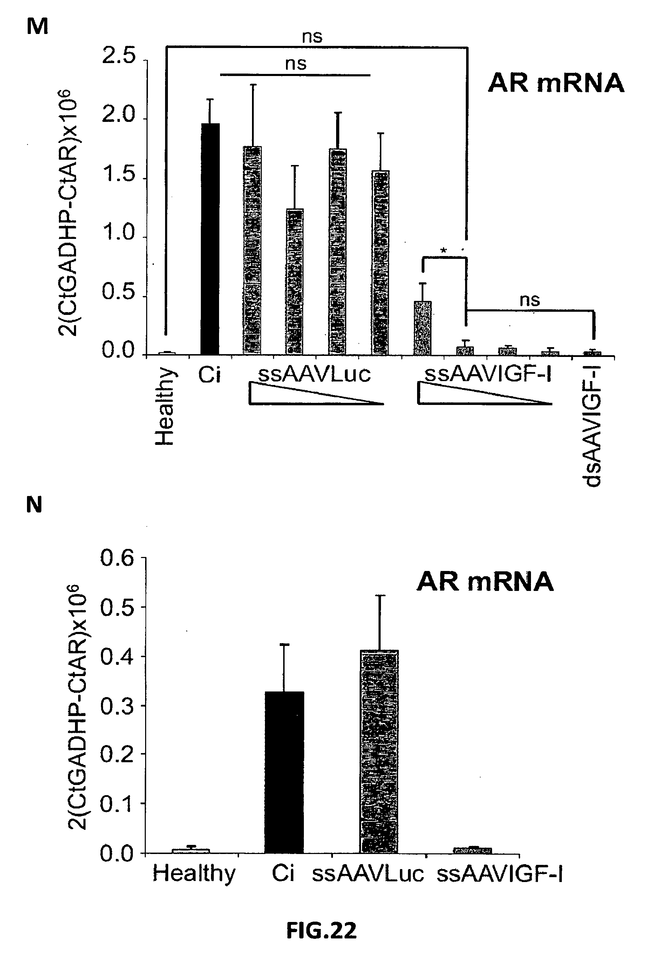

**Figure 22****. Analysis of inflammatory and profibrogenic factors.** Liver extract samples were obtained 8 weeks (A, C, E, G, I, K and M), or 16 weeks after treatment (B, D, F, I, J, L and N). For the later case, represented data correspond to the low dose of ssAAVLuc and ssAAVIGF-I TGFβ, TNFα, IL-6, CTGF, VEGF, PDGF, and amphiregulin (AR) mRNA expression was quantified by qRT-PCR Triangles indicate doses in decreasing order.

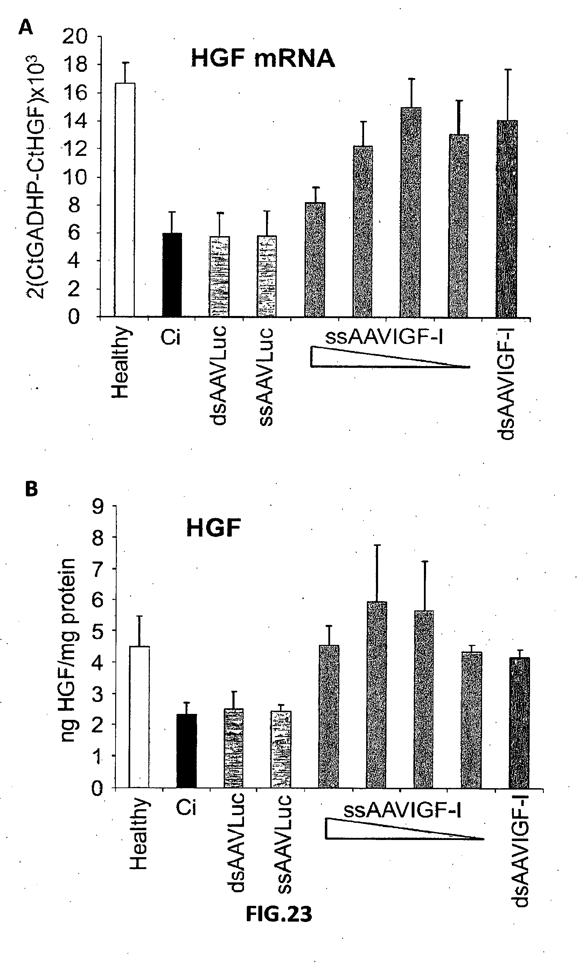

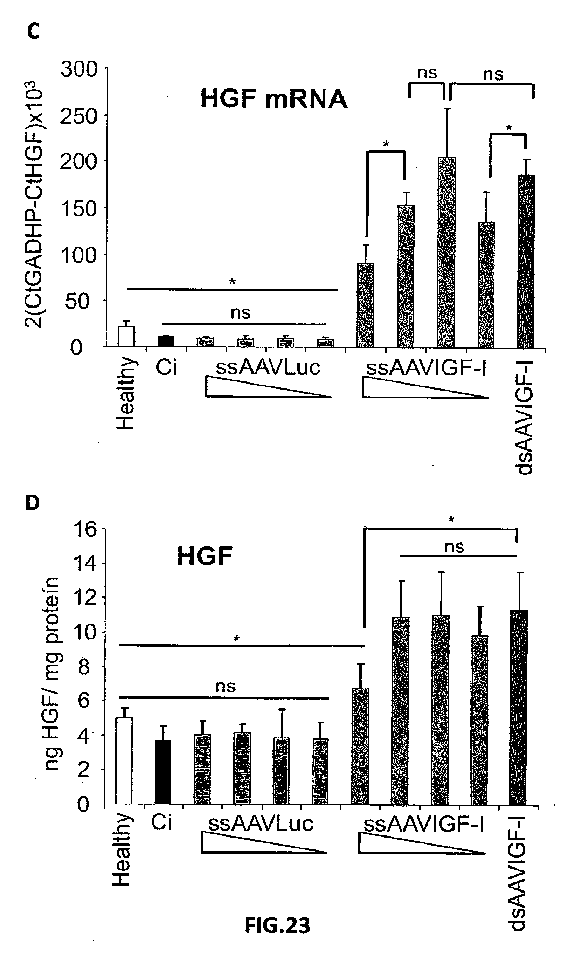

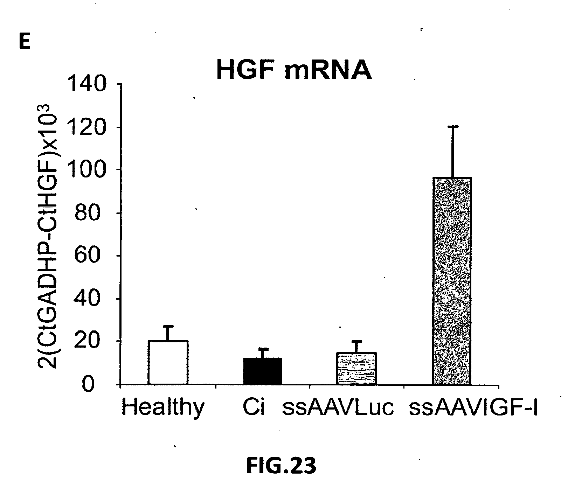

**Figure 23****. Analysis of hepatocytes growth factor HGF.** Liver extract samples were obtained 4 days (A-B), 8 weeks (C-D), or 16 weeks after treatment (E). For A and B represented data for ssAAVIGF-I correspond to the high dose and the very low dose. For the later case, represented data correspond to the low dose of ssAAVLuc and ssAAVIGF-I. HGF mRNA expression was quantified by qRT-PCR (A, C and E); HGF protein levels were quantified by ELISA (B and D). Triangles indicate doses in decreasing order.

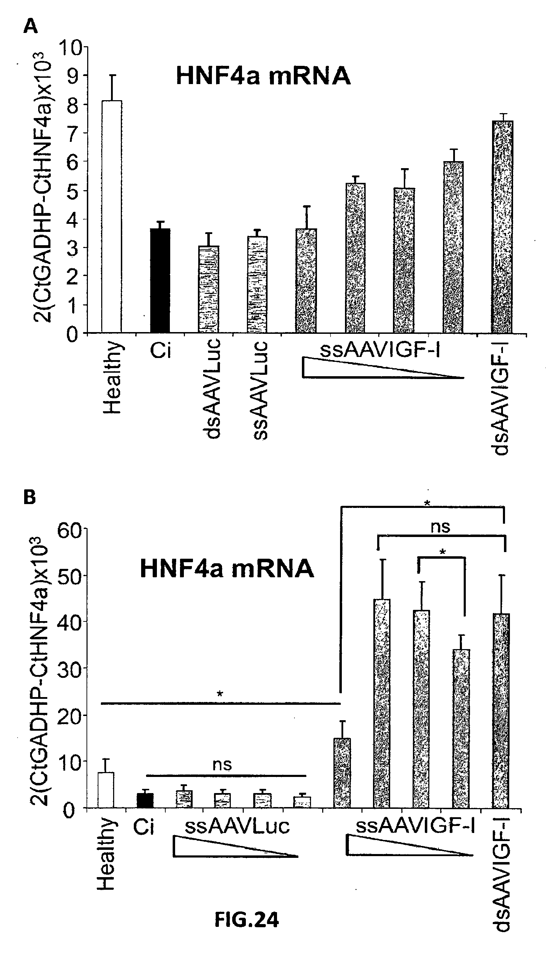

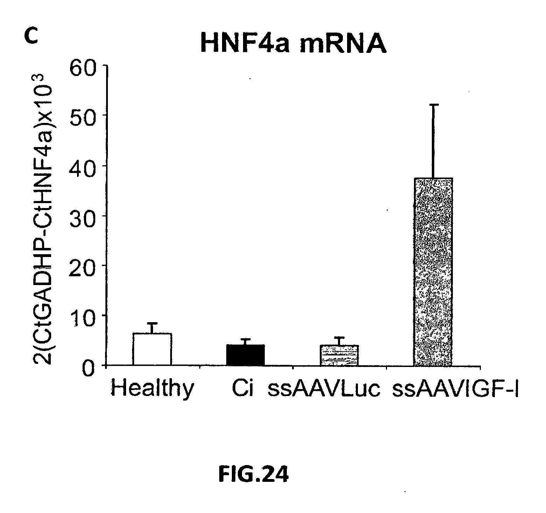

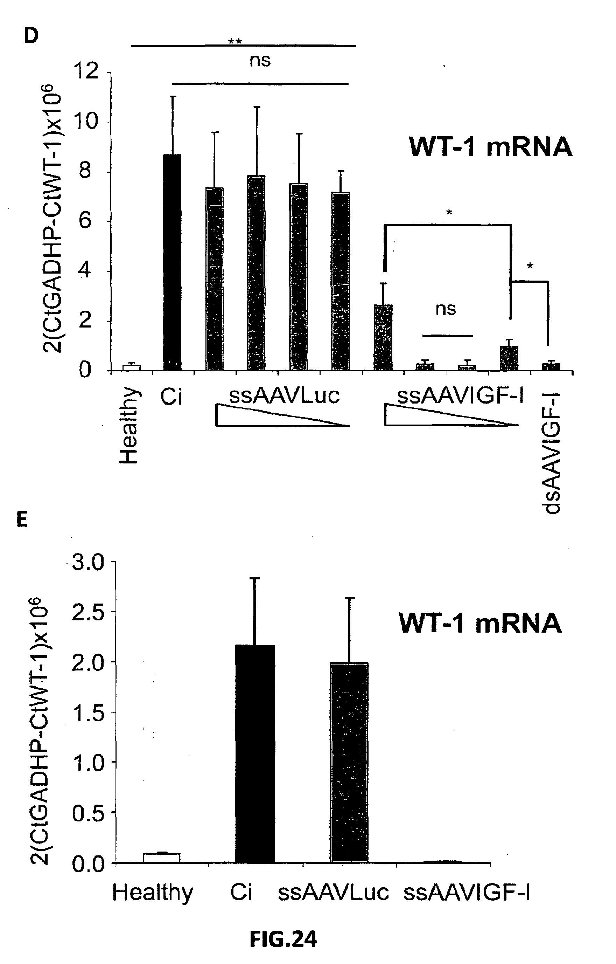

**Figure 24****. Analysis of differentiation factors.** Liver extract samples were obtained 4 days (A), 8 weeks (B, D), or 16 weeks after treatment (C, E). For 4 days, represented dose for ssAAVLuc correspond to the very low dose. For 16 weeks, represented data correspond to the low dose of ssAA VLuc and ssAA VIGF-I. Maturation factor HNF4a (A, B and C) and differentiation factor WT-1 (D and E) mRNA expression levels were quantified by qRT-PCR Triangles indicate doses in decreasing order.

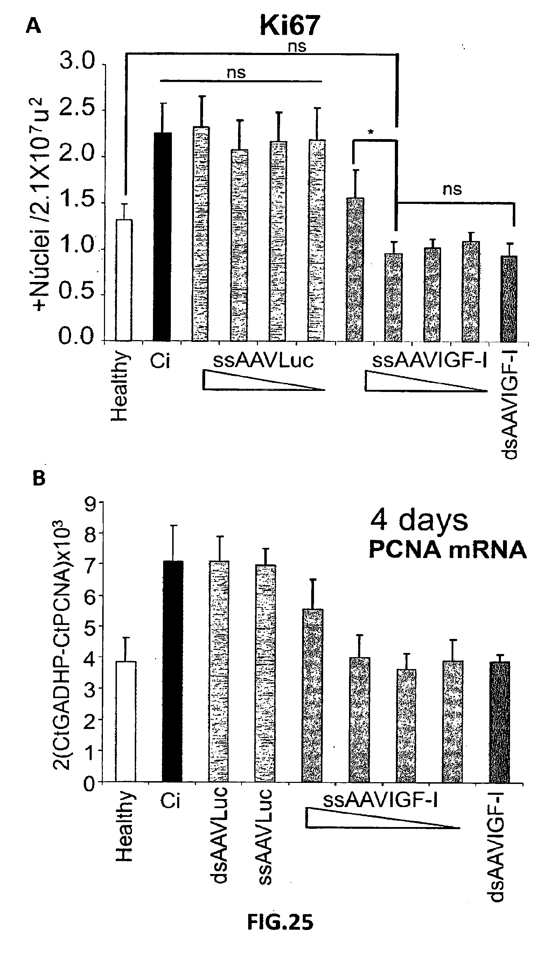

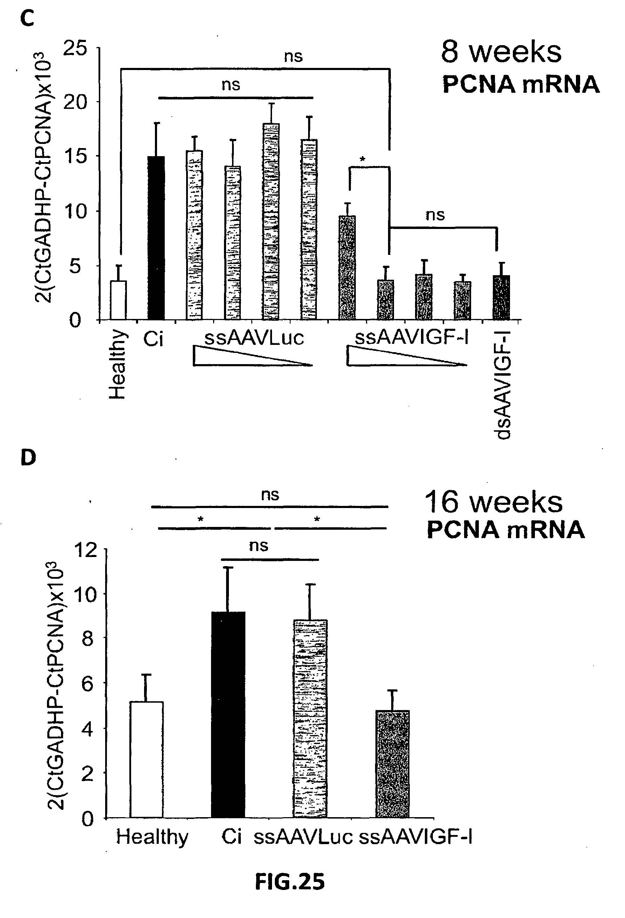

**Figure. 25****. Analysis of proliferation. A:** Quantification of Ki67 stained nuclei in liver tissue samples obtained 4 days after treatment **B, C and D:** Proliferation factor PCNA mRNA expression levels quantified by qRT-PCR in liver extract samples obtained 4 days (B), 8 weeks (C) or 16 weeks (D) after treatment For 4 days, represented dose for ssAA VLuc correspond to the very low dose. For 16 weeks, represented data correspond to the low dose of ssAAVLuc and ssAA VIGF-I. Triangles indicate doses in decreasing order.

### DESCRIPTION OF THE INVENTION

The invention in its broadest sense is as characterized in the independent claims.

The authors of the present invention have observed that the administration to rats with established liver cirrhosis of a viral vector encoding IGF-I wherein the polynucleotide encoding IGF-I is under the control of a liver-specific promoter activates a robust tissue repair program characterized by stimulation of fibrolysis, downregulation of profibrogenic factors and induction of cytoprotective molecules. These changes are associated with a marked improvement of liver structure and hepatocellular function.

These findings suggest that IGF-I gene transfer with AAV vectors to the cirrhotic liver may be a potential therapeutic option for patients with advanced liver cirrhosis whom cannot be offered liver transplant or who deteriorate on the waiting list for transplantation.

### VIRAL GENOMES

The authors of the present invention have observed that the transfer of IGF-I to the cirrhotic liver using a viral vector wherein the sequence encoding IGF-I is under the control of a liver-specific promoter results in an improvement in liver function and a reduction of liver fibrosis (see examples 3 and 10 of the present invention). Thus, in a first aspect, the disclosure relates to a viral genome comprising a nucleotide sequence encoding IGF-I or a functionally equivalent variant thereof which is operably linked to a liver-specific promoter.

As used herein, the term "viral genome" refers to a recombinant viral genome (i.e., viral DNA) that comprises one or more heterologous nucleotide sequences. Preferably, all other structural and non-structural coding sequences are not present in the viral vector since they can be provided in *trans* by a vector, such as a plasmid, or by stably integrating the sequences into a packaging cell line. The vectors may be utilized for the purpose of transferring DNA into cells either *in vitro, in vivo* or *ex vivo.* Viral genomes include, without limitation, an adenoviral vector, a retroviral vector, a vaccinia viral vector, including poxviral-based vectors, an adeno-associated viral vector, a polyoma viral vector, an alphaviral vector, a rhabdoviral vector, a picornavirus vector, a herpesviral vector, including EBV vectors, including lentiviral vectors, MML V-based vectors.

The term "nucleotide sequence", is used herein interchangeably with "polynucleotide", and relates to any polymeric form of nucleotides of any length and composed of ribonucleotides and/or deoxyribonucleotides. The term includes both single-stranded and double-stranded polynucleotides as well as modified polynucleotides (methylated, protected and the like).

The term "IGF-I", as used herein, is used interchangeably with the terms "insulin-like growth factor I" and somatomedin C and relates to a family of polypeptides characterised in that they show insulin-like effects and insulin-like structure, sharing nearly 50% of amino acid homology with insulin. Furthermore, by three dimensional modelling, it has been shown that the structures of IGF's are similar to proinsulin being a single chain peptide, cross-linked by three disulfide bridges and consisting of a B-chain-like amino-terminal part (B domain), a connecting peptide (C domain), and an A-chain-like part (A domain). In addition, a carboxyl-terminal extension not found in proinsulin is present (D domain). The IGF-I polypeptide comprise yet another carboxyl-terminal extension not found in proinsulin which has been given an E domain designation.

Suitable IGF-I molecules useful for the invention include, without limitation,
- Amino acids 1-158, 49-158 or 49-116 of the polypeptide described under NCBI under accession number NP_001104753 (SEQ ID NO:1), corresponding, respectively, to human isoform 1 of prepro-IGF-I, pro-IGF-I or the mature IGF-I.
- Amino acids 1-137, 33-137 or 33-102 of the polypeptide described under NCBI under accession number NP_001104754 (SEQ ID NO:2), corresponding, respectively, to human isoform 2 of prepro-IGF-I, pro-IGF-I or mature IGF-I.
- Amino acids 1-195, 49-195 or 49-118 of the polypeptide described under NCBI under accession number NP_001104755 (SEQ ID NO:3), corresponding, respectively, to isoform 3 of human prepro-IGF-I, pro-IGF-I or mature IGF-I.

The invention also contemplates the use of polynucleotides encoding IGF- 1 from different animal species such as, without limitation: *Cervus elaphus* insulin-like growth factor I (IGF-I) mRNA (GenBank Accession No. U62106); *Equus caballus* insulin-like growth factor I precursor (IGF-I) mRNA, (GenBank Accession No. U28070); Goat mRNA for insulin-like growth factor-I, (GenBank Accession No. D11378); *Oryctolagus cumiculus* insulin-like growth factor 1 precursor (IGF-1) mRNA, (GenBank Accession No. U75390); Pig insulin-like growth factor I (pIGF-I) mRNA, (GenBank Accession No. M31175); *Ovis aries* insulin-like growth factor I (IGF-I) mRNA, (GenBank Accession No. M89787); Human insulin-like growth factor (IGF-I) IA and IB gene, exon 1, (GenBank Accession Nos. M12659 and M77496;) Rat insulin-like growth factor I (IGF-I) mRNA, (GenBank Accession No. M15480); Chicken insulin-like growth factor (IGF-I) mRNA, ((GenBank Accession Nos. M32791 and M29720); Salmon insulin-like growth factor I (IGF-I) mRNA, (GenBank accession no. M32792); X. laevis insulin-like growth factor I (IGF-I) mRNA, (GenBank Accession No. M29857).

The skilled person will appreciate that IGF-I is synthesized as a precursor form which undergoes a first cleavage step by the signal peptidase upon accession to the secretory pathway to produce pro-IGF-I which is then processed to mature IGF-I by endoproteolytic processing of its C-terminal region. Thus, the nucleotide sequence present in the vector of the invention may encode for the full-length precursor form, which should then be processed by the target cell machinery based on the presence of the IGF-I endogenous signal peptide. Alternatively, it is also possible to include a polynucleotide encoding the pro-IGF-I fused to a heterologous signal sequence. The expression "signal sequence", as used herein, refers to a DNA sequence at the 5' end of a structural gene which is transcribed and translated along with the gene. The leader usually results in the protein having an n-terminal peptide extension sometimes called a pro-sequence. For proteins destined for either secretion to the extracellular medium or the membrane, this signal sequence directs the protein into endoplasmic reticulum from which it is discharged to the appropriate destination. The leader sequence normally is encoded by the desired nucleic acid, synthetically derived or isolated from a different gene sequence. Suitable heterologous sequences suitable as signal sequences for promoting secretion of the polynucleotide of the invention include the signal sequences of gelsolin, albumin, fibrinogen, among others, the signal peptides from tissue plasminogen activator, insulin, and neuron growth factor (NGF).

The skilled person will also appreciate that, as long as the length of the viral genome does not exceed the packaging size limit of the viral capsid, the viral genome of the invention may comprise part or all of the genomic sequence encoding IGF-I, in which case, the coding region of IGF-I will be interrupted by intronic regions.

The invention also contemplates viral genomes which comprise sequences encoding IGF-I variants and fragments known in the art such as those described by Sara, V.R. et al (Proc.Natl.Acad.Sci. USA, 1986, 83: 4904-4907), Ballard, F.J. et al. (Biochem. Biophys. Res. Commun. 1987, 149: 398-404); Bayne et al (J. Biol. Chem. 1988, 263:6233-6239); Sara V. R. et al. (Biochem. Biophys. Res. Commun., 1989, 165:766-771); Forsberg et al., 1990, Biochem. J. 271:357-363); US. Patent Nos. 4, 876,242 and 5,077,276; and International Patent Publication Nos. WO87/01038 and WO89/05822. Representative analogues include one with a deletion of Glu-3 of the mature molecule, analogues with up to 5 amino acids truncated from the N-terminus, an analogue with a truncation of the first 3 N-terminal amino acids (referred to as des (1-3)-IGF-I, des-IGF-I, tIGF-I, or brain IGF), and an analogue including the first 17 amino acids of the B chain of human insulin in place of the first 16 amino acids of human IGF-1.

Thus, the invention should be construed to include DNA encoding functional equivalent variants of IGF-I. The term "functional equivalent variant", as used herein relates to any polypeptide which sequence can be obtained from the sequence of IGF-I as defined above by means of insertion of one or more nucleotides in the sequence, the addition of one or more nucleotides in any end or inside the sequence, or the deletion of one or more nucleotides in any end or inside the sequence and which substantially preserves the biological activity of IGF-I. Methods for determining whether a variant preserves the biological activity of the native IGF-I are widely known to the skilled person and include, the determination of DNA and protein synthesis in cultured rat calvaria as described by Canalis et al (J.Clin.Invest., 1980, 66:709-719), the stimulation of sulphate and thymidine uptake in chick cartilage as described by Jennings et al. (J. Clin. Endocrinol. Metab., 1980, 51:1166-70) or the stimulation of DNA synthesis in the rat clonal aortic smooth muscle cell line A10 as described by Bayne et al (J.Biol.Chem., 1988, 263:6233-6239).

Variants of IGF-I may be obtained by substituting nucleotides within the polynucleotide accounting for codon preference in the host cell that is to be used to produce the IGF-I. Such "codon optimization" can be determined via computer algorithms which incorporate codon frequency tables such as "Ecohigh. Cod" for codon preference of highly expressed bacterial genes as provided by the University of Wisconsin Package Version 9.0, Genetics Computer Group, Madison,Wis. Other useful codon frequency tables include "Celegans_ high.cod", "Celegans _low.cod", Drosophila_high.cod", "Human_ high.cod", "Maize_high.cod", and "Yeast_ high.cod".

Variants of IGF-I may be generated by making conservative amino acid changes and testing the resulting variant in one of the functional assays described above or another functional assay known in the art. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, and asparagine-glutamine.

The functionally equivalent variants of IGF-I include polypeptides which are substantially homologous to the native IGF-I. The expression "substantially homologous", as used herein, relates to any of the nucleotide sequences describe above when its nucleotide sequence has a degree of identity with respect to the nucleotide sequence of the invention of at least 60%, advantageously of at least 70%, preferably of at least 85%, and more preferably of at least 95%. A nucleotide sequence that is substantially homologous to the nucleotide sequence of the invention can typically be isolated from a producer organism of the polypeptide of the invention based on the information contained in said nucleotide sequence, or it is constructed based on the DNA sequence described above. The degree of identity between two polynucleotides is determined using computer algorithms and methods that are widely known for the persons skilled in the art. The identity between two amino acid sequences is preferably determined by using the BLASTN algorithm [BLAST Manual, Altschul, S., et al., NCBI NLM NIH Bethesda, Md. 20894, Altschul, S., et al., J. Mol. Biol. 215: 403-410 (1990)]. BLAST and BLAST 2.0 are used, with the parameters described herein, to determine percent sequence identity. Software for performing BLAST analyses is publicly available through the National Center for Biotechnology Information. This algorithm involves first identifying high scoring sequence pairs (HSPs) by identifying short words of length W in the query sequence, which either match or satisfy some positive- valued threshold score T when aligned with a word of the same length in a database sequence. T is referred to as the neighborhood word score threshold (Altschul et al, supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always 0) and N (penalty score for mismatching residues; always 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff, Proc. Natl. Acad. Sd. USA, 1989, 89:10915) alignments (B) of 50, expectation (E) of 10, M=5, N=-4, and a comparison of both strands.

As those skilled in the art will appreciate, variants or fragments of IGF-I can be generated using conventional techniques, such as mutagenesis, including creating discrete point mutation(s), or by truncation. For instance, mutation can give rise to variants which retain substantially the same, or merely a subset, of the biological activity of a polypeptide growth factor from which it was derived.

As used herein, the term "operably linked" refers to a linkage of polynucleotide (or polypeptide) elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame.

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter, including e.g. attenuators or enhancers, but also silencers. A "constitutive" promoter is a promoter that is active in most tissues under most physiological and developmental conditions. An "inducible" promoter is a promoter that is physiologically or developmentally regulated, e.g. by the application of a chemical inducer. A "tissue specific" promoter is a promoter only active in specific types of tissues or cells. That is to say a tissue specific promoter, is one which is more active in one or several (for example two, three or four) particular tissues than other tissues (i.e. is capable of driving higher a promoter that allows expression of a coding sequence to which is it operably linked in the tissue(s) for which it is specific as compared to any others). Typically, the down-stream gene in a "tissue specific" promoter is one which is active to a much higher degree in the tissue(s) for which it is specific than in any other. In this case, there may be little or substantially no activity of the promoter in any tissue other than the one(s) for which it is specific.

In the context of this invention, a liver specific promoter is a promoter which is more active in liver as compared to its activity in any other tissue in the body. Typically, the activity of a liver specific promoter will be considerably greater in the liver than in other tissues. For example, such a promoter may be at least 2, at least 3, at least 4, least 5 or least 10 times more active (for example as determined by its ability to drive the expression in a given tissue while preventing expression in other cells or tissues. Accordingly, a liver specific promoter allows an active expression of the linked gene in the liver and prevents expression in other cells or tissues.

Liver-specific promoters include, without limitation, an α1-anti-trypsin (AAT) promoter, a thyroid hormone-binding globulin promoter, an alpha fetoprotein promoter, an alcohol dehydrogenase promoter, an IGF-II promoter, the factor VIII (FVIII) promoter, a HBV basic core promoter (BCP) and PreS2 promoter, an albumin promoter, a thyroxin-binding globulin (TBG) promoter, an Hepatic Control Region (HCR)-ApoCII hybrid promoter, an HCR-hAAT hybrid promoter, an AAT promoter combined with the mouse albumin gene enhancer (Ealb) element, an apolipoprotein E promoter, a low density lipoprotein promoter, a pyruvate kinase promoter, a phosphenol pyruvate carboxykinase promoter, a lecithin-cholesterol acyl transferase (LCAT) promoter, an apolipoprotein H (ApoH) promoter, the transferrin promoter, a transthyretin promoter, an alpha-fibrinogen and beta-fibrinogen promoters, an alpha 1-antichymotrypsin promoter, an alpha 2-HS glycoprotein promoter, an haptoglobin promoter, a ceruloplasmin promoter, a plasminogen promoter, promoters of the complement proteins (CIq, CIr, C2, C3, C4, C5, C6, C8, C9, complement Factor I and Factor H), C3 complement activator and the α1-acid glycoprotein promoter. Additional tissue-specific promoters may be found in the Tissue-Specific Promoter Database, TiProD (Nucleic Acids Research, J4:D104-D107 (2006).

A liver-specific promoter may be a hybrid promoter comprising a liver-specific enhancer and a liver-specific promoter such as an Hepatic Control Region (HCR)-ApoCII hybrid promoter, an HCR-hAAT hybrid promoter, an AAT promoter combined with the mouse albumin gene enhancer (Ealb) element and an apolipoprotein E promoter. In a preferred embodiment, the hybrid promoter comprises the mouse albumin gene enhancer (Ealb) and the mouse alphal-antitrypsin (AAT) promoter (Ealb-AATp). In a still more preferred embodiment, the promoter region corresponds to the sequence of SEQ ID NO:4.

A liver-specific promoter may be an inducible liver specific promoter, for example a tetracycline-inducible liver-specific promoter such as the promoter described by Wang et al. (Nature Biotech., 1997;15:239-43), the adenovirus mediated regulatable liver-specific promoter described by Burcin et al., (Proc. Natl. Acad. Sci. USA, 1999, 96:355-60), the tetracycline-regulatable liver-specific promoter described by Manickan et al (J. Biol. Chem., 2001, 276:13989-13994), the promoters described by Han et al. (Molecular Therapy, 2005, 11, S161), the tetracycline-regulated adenoviral expression system for in vivo delivery to liver as described by Tietge et al. (J.Gen.Medicine, 2003, 5:567-575), the mifepristone (RU-486)-inducible liver specific promoter as described by Crettaz et al. (Molecular Therapy (2006) 13, S224) and the like.

Additional elements that can be inserted into the viral genomes of the invention include a Kozak consensus sequence around the initiation codon of the nucleotide sequence encoding the IGF-I or the variant thereof. The Kozak consensus sequence is herein defined as GCCRCC(AUG)A (SEQ ID NO: 5), wherein R is a purine (i.e. A, adenosine or G, guanosine) and wherein (AUG) stands for the initiation codon of the porphobilinogen deaminase coding sequence. The Kozak consensus sequence may be preceded by another GCC triplet.

The parvo viral genomes of the invention may also comprise polyadenylation signals operably linked to the nucleic acid encoding IGF-I or the functionally equivalent variant thereof. The term "polyadenylation signal", as used herein, relates to a nucleic acid sequence that mediates the attachment of a polyadenine stretch to the 3' terminus of the mRNA. Suitable polyadenylation signals include the SV40 early polyadenylation signal, the SV40 late polyadenylation signal, the HSV thymidine kinase polyadenylation signal, the protamine gene polyadenylation signal, the adenovirus 5 EIb polyadenylation signal, the bovine growth hormone polydenylation signal, the human variant growth hormone polyadenylation signal and the like.

Also described is a polyoma virus genome. Polyoma viruses, such as SV40, are known to infect non-dividing as well as actively dividing cells and are also known to be non-immunogenic allowing repeated administration to the same individual. Moreover, it allows long- term expression of the transgene. Polyoma viruses include any vector based on viruses of the genus Polyoma and includes JC virus, BK virus, KI virus, Wu virus, Merkel cell polyomavirus and Simian vacuolating virus 40 (hereinafter SV40). In the disclosure, the polyoma viral genome is a SV40 genome.

SV40 comprises a 5.25 kilobases, long circular double stranded DNA genome which consists of two regulatory regions, the promoter/origin region and the polyadenylation region. The promoter/origin region is 500 base pairs long and comprises two oppositely-directed promoters, the early and late promoter (SVEP and SVLP respectively) that flank the central origin of replication and packaging signal. The polyadenylation region is 100 base pairs long and contains the polyadenylation signals of both the early and the late transcripts. The early promoter drives expression of the small, medium and large T antigens (stag, mtag and Tag, respectively) necessary for virus replication and activation of the late promoter. The late promoter drives expression of the viral capsid proteins VP1, 2 and 3.

The disclosure contemplates the replacement of at least one expression cassette of SV40 by a polynucleotide comprising a liver-specific promoter and a sequence encoding IGF-I or a functionally equivalent variant thereof. The skilled person will appreciate that the polynucleotide comprising the liver-specific promoter and the IGF-I coding sequence can be inserted by replacing the early promoter and the small, medium and large T antigens. Alternatively, the polynucleotide comprising the liver-specific promoter and the IGF-I coding sequence may be inserted by replacing the late promoter region and the sequence coding for the viral capsid proteins VP1, 2 and 3. Also contemplated are SV40 vectors lacking all coding sequences (a gutless SV40 genome), lacking all viral genome except the regions comprising the control elements necessary for replication and packaging of the vector. Thus, minimal SV40 genome is derived from this region and contains at least a complete origin of replication. Suitable SV40 vectors include pSVT7 and pMT2.

The viral genome of the invention is a parvoviral genome. The term "parvovirus" as used herein encompasses the family Parvoviridae, including autonomously-replicating parvoviruses and dependoviruses. The autonomous parvoviruses include members of the genera Parvovirus, Erythrovirus, Densovirus, Iteravirus, and Contravirus. Exemplary autonomous parvoviruses include, but are not limited to, minute virus of mouse, bovine parvovirus, canine parvovirus, chicken parvovirus, feline panleukopenia virus, feline parvovirus, goose parvovirus, H1 parvovirus, muscovy duck parvovirus, B19 virus, and any other autonomous parvovirus now known. Other autonomous parvoviruses are known to those skilled in the art. See, e.g., BERNARD N. FIELDS et al., VIROLOGY, volume 2, chapter 69 (4th ed., Lippincott-Raven Publishers).

On the other hand, and as may be deduced from the name of their genus, members of the Dependovirus are unique in that they usually require co-infection with a helper virus such as adenovirus or herpes virus for productive infection in cell culture. The genus Dependovirus includes AAV, which normally infects humans (e.g., serotypes 1, 2, 3A, 3B, 4, 5, and 6) or primates (e.g., serotypes 1 and 4), and related viruses that infect other warm-blooded animals (e.g., bovine, canine, equine, and ovine adeno-associated viruses). Further information on parvoviruses and other members of the Parvoviridae is described in Kenneth I. Berns, "Parvoviridae: The Viruses and Their Replication," Chapter 69 in Fields Virology (3d Ed. 1996).

In a still more preferred embodiment, the parvoviral genome is an adeno-associated virus (AAV) genome. As used herein, the term "adeno-associated virus" (AAV) includes any AAV serotype. Generally, the AAV serotypes have genomic sequences of significant homology at the amino acid and the nucleic acid levels, provide an identical set of genetic functions, produce virions which are essentially physically and functionally equivalent, and replicate and assemble by practically identical mechanisms. In particular, the invention may be carried out using to AAV serotype 1 (AAV1), AAV2, AAV3 (including types 3A and 3B), AAV4, AAV5, AAV6, AAV7, AAV8, AAV9, AAV10, AAV11, avian AAV, bovine AAV, canine AAV, equine AAV, ovine AAV, and any other AAV now known or later discovered. See, e.g., Fields et al., Virology, volume 2, chapter 69 (4th ed., Lippincott-Raven Publishers). Recently, a number of putative new AAV serotypes and clades have been identified (see, e.g., Gao et al., (2004) J. Virology 78:6381-6388; Moris et al., (2004) Virology 33-:375-383; and Table 1). The genomic sequences of the various serotypes of AAV and the autonomous parvoviruses, as well as the sequences of the terminal repeats, Rep proteins, and capsid subunits are known in the art. Such sequences may be found in the literature or in public databases such as GenBank. See, e.g., GenBank Accession Numbers NC_002077, NC_001401, NC_001729, NC_001863, NC_001829, NC_001862, NC_000883, NC_001701, NC_001510, NC_006152, NC_006261, AF063497, U89790, AF043303, AF028705, AF028704, J02275, J01901, J02275, X01457, AF288061, AH009962, AY028226, AY028223, NC_001358, NC_001540, AF513851, AF513852, AY530579; See also, e.g., Srivistava et al., (1983) J. Virology 45:555; Chiorini et al., (1998) J. Virology 71:6823; Chiorini et al" (1999) J. Virology 73:1309; Bantel-Schaal et al., (1999) J. Virology 73:939; Xiao et al., (1999) J. Virology 73:3994; Muramatsu et al., (1996) Virology 221: 208; Shade et al., (1986) J. Virol. 58:921; Gao et al., (2002) Proc. Nat. Acad. Sci. USA 99:11854; Moris et al., (2004) Virology 33-: 375-383; international patent publications WO 00/28061, WO 99/61601, WO 98/11244; and U.S. Patent No. 6,156,303.

For convenience the present invention is further exemplified and described herein by reference to AAV. It is however understood that the invention is not limited to AAV but may equally be applied to other parvoviruses.

The genomic organization of all known AAV serotypes is very similar. The genome of AAV is a linear, single-stranded DNA molecule that is less than about 5,000 nucleotides (nt) in length. Inverted terminal repeats (ITRs) flank the unique coding nucleotide sequences for the non-structural replication (Rep) proteins and the structural (VP) proteins. The VP proteins (VP1, -2 and -3) form the capsid. The terminal 145 nt are self-complementary and are organized so that an energetically stable intramolecular duplex forming a T-shaped hairpin may be formed. These hairpin structures function as an origin for viral DNA replication, serving as primers for the cellular DNA polymerase complex. Following wtAAV infection in mammalian cells the Rep genes (i.e. Rep78 and Rep52) are expressed from the P5 promoter and the P19 promoter, respectively and both Rep proteins have a function in the replication of the viral genome. A splicing event in the Rep ORF results in the expression of actually four Rep proteins (i.e. Rep78, Rep68, Rep52 and Rep40). However, it has been shown that the unspliced mRNA, encoding Rep78 and Rep52 proteins, in mammalian cells are sufficient for AAV vector production. Also in insect cells the Rep78 and Rep52 proteins suffice for AAV vector production.

A "recombinant parvoviral or AAV genome" (or "rAAV genome") herein refers to a vector comprising one or more polynucleotide sequences of interest, genes of interest or "transgenes" that are flanked by at least one parvoviral or AAV inverted terminal repeat sequences (ITRs). Such rAAV vectors can be replicated and packaged into infectious viral particles when present in an insect host cell that is expressing AAV rep and cap gene products (i.e. AAV Rep and Cap proteins). When an rAAV vector is incorporated into a larger nucleic acid construct (e.g. in a chromosome or in another vector such as a plasmid or baculovirus used for cloning or transfection), then the rAAV vector is typically referred to as a "pro-vector" which can be "rescued" by replication and encapsidation in the presence of AAV packaging functions and necessary helper functions.

Thus, in another aspect the invention relates to a nucleic acid construct comprising a nucleotide sequence encoding a IGF-I or a functionally equivalent variant thereof as herein defined above, wherein the nucleic acid construct is a recombinant parvoviral or AAV vector and thus comprises at least one parvoviral or AAV ITR, as recited in the claims. Preferably, in the nucleic acid construct the nucleotide sequence encoding the IGF-I or a functionally equivalent variant thereof is flanked by parvoviral or AAV ITRs on either side. Any parvoviral or AAV ITR may be used in the constructs of the invention, including ITRs from AAV1, AAV2, AAV4, and/or AAV5. ITRs of AAV2 are most preferred.

AAV sequences that may be used in the present invention can be derived from the genome of any AAV serotype. Generally, the AAV serotypes have genomic sequences of significant homology at the amino acid and the nucleic acid levels, provide an identical set of genetic functions, produce virions which are essentially physically and functionally equivalent, and replicate and assemble by practically identical mechanisms. For the genomic sequence of the various AAV serotypes and an overview of the genomic similarities see e.g. GenBank Accession number U89790; GenBank Accession number J01901; GenBank Accession number AF043303; GenBank Accession number AF085716; Chlorini et al. (1997, J. Vir. 71: 6823-33); Srivastava et al. (1983, J. Vir. 45:555-64); Chlorini et al. (1999, J. Vir. 73:1309-1319); Rutledge et al. (1998, J. Vir. 72:309-319); and Wu et al. (2000, J. Vir. 74: 8635-47). AAV serotypes 1, 2, 3, 4 and 5 are preferred source of AAV nucleotide sequences for use in the context of the present invention. Preferably the AAV ITR sequences for use in the context of the present invention are derived from AAV1, AAV2, and/or AAV4.

Although it is preferred that the nucleic acid sequences encoding the capsid genes are provided in *trans* by the packaging cell or by a second vector, the invention also contemplates AAV genomes which further comprise a sequence encoding one or more capsid proteins which package the above mentioned polynucleotide sequence. The sequences coding for the VP1, VP2, and VP3 capsid proteins for use in the context of the present invention may however be taken from any of the known 42 serotypes, more preferably from AAV1, AAV2, AAV3, AAV4, AAV5, AAV6, AAV7, AAV8 or AAV9 or newly developed AAV-like particles obtained by e.g. capsid shuffling techniques and AAV capsid libraries. When the sequences encoding the capsid proteins derive from a different AAV serotype as the ITRs, the AAV genome is known as a "hybrid" parvovirus genome (i.e., in which the AAV capsid and the AAV terminal repeat(s) are from different AAV) as described in international patent publication WO 00/28004 and Chao et al., (2000) Molecular Therapy 2:619. As described herein, the rAAV vector can be any suitable rAAV vector now known. Alternatively, the sequences encoding the capsid genes may be provided in trans by co-transfecting into the packaging cell a polynucleotide encoding said capsid proteins. In a preferred embodiment, the viral vector comprises ITRs from AAV1, AAV2 and/or AAV4 and one or more or all capsid genes from AAV1, AAV2, AAV5, AAV6 or AAV8.

If the viral vector comprises sequences encoding the capsid proteins, these may be modified so as to comprise an exogenous targeting sequence. Suitable exogenous targeting sequences are described in detail below in the context of the virions of the invention.

Optionally, the AAV genomes of the invention may comprise additional sequences coding for the Rep proteins. The Rep (Rep78/68 and Rep52/40) coding sequences are preferably derived from AAV1, AAV2, and/or AAV4. AAV Rep and ITR sequences are particularly conserved among most serotypes. The Rep78 proteins of various AAV serotypes are e.g. more than 89% identical and the total nucleotide sequence identity at the genome level between AAV2, AAV3A, AAV3B, and AAV6 is around 82% (Bantel-Schaal et al., 1999, J. Virol., 73:939-947). Moreover, the Rep sequences and ITRs of many AAV serotypes are known to efficiently cross-complement (i.e., functionally substitute) corresponding sequences from other serotypes in production of AAV particles in mammalian cells. US2003148506 reports that AAV Rep and ITR sequences also efficiently cross-complement other AAV Rep and ITR sequences in insect cells.

The AAV VP proteins are known to determine the cellular trophicity of the AAV virion. The VP protein-encoding sequences are significantly less conserved than Rep proteins and genes among different AAV serotypes. The ability of Rep and ITR sequences to cross-complement corresponding sequences of other serotypes allows for the production of pseudotyped rAAV particles comprising the capsid proteins of one serotype (e.g., AAV5) and the Rep and/or ITR sequences of another AAV serotype (e.g., AAV2). Such pseudotyped rAAV particles are a part of the present invention as recited in the claims.

Typically, the AAV genome of the invention comprises, in addition to the expression cassette comprising the liver-specific promoter and the sequence encoding IGF-I or the functionally equivalent variant thereof, one or more of the following elements:
- Inverted terminal repeats
- Non resolvable terminal repeats
- Sequences encoding capsid genes
- Stuffer sequences to complete the minimal packageable genome size

The inverted terminal repeats (ITR) are typically present in at least two copies of the AAV vector, typically flanking the expression cassette containing the heterologous sequence. The ITRs typically will be at the 5' and 3' ends of the heterologous nucleotide sequence(s), but need not be contiguous thereto. The terminal repeats can be the same or different from each other. The term "terminal repeat" includes any viral terminal repeat and/or partially or completely synthetic sequences that form hairpin structures and function as an inverted terminal repeat, such as the "double-D sequence" as described in United States Patent No. 5,478,745 to Samulski et al. An "AAV terminal repeat" may be from any AAV, including but not limited to serotypes 1, 2, 3, 4, 5, 6, 7, 8, 9, 10, 11 or 12 or any other AAV now known or later discovered. The AAV terminal repeat need not have a wild- type sequence (e.g., a wild-type sequence may be altered by insertion, deletion, truncation or missense mutations), as long as the terminal repeat mediates the desired functions, e.g., replication, nicking, virus packaging, integration, and/or provirus rescue, and the like. The vector genome can comprise one or more (e.g., two) AAV terminal repeats, which may be the same or different. Further, the one or more AAV terminal repeats can be from the same AAV serotype as the AAV capsid, or can be different. In particular embodiments, the vector genome comprises an AAV1, AAV2, AAV3, AAV4, AAV5, AAV6, AAV7, AAV8, AAV9, AAV10, AAV11 and/or AAV 12 terminal repeat, in particular from AAV1, AAV2 and/or AAV4.. In a preferred embodiment, the ITRs may derive from AAV2 and may be defined by SEQ ID NO:6 (5' ITR) and SEQ ID NO:7 (3'-ITR).

The AAV genomes of the invention may also contain non-resolvable terminal repeats. The expression "non-resolvable terminal repeat", as used herein, relates to terminal repeats which are not recognized by and resolved (i.e., "nicked") by the AAV Rep proteins, such that resolution of the terminal repeat is substantially reduced (e.g., by at least about 50%, 60%, 70%, 80%. 90%, 95%, 98% or greater as compared with a resolvable terminal repeat) or eliminated. Such non-resolvable terminal repeats may be naturally-occurring terminal repeat sequences (including altered forms thereof) and, for example, can be derived from a parvovirus, including an AAV, or can be from another virus or, as a further alternative, can be partially or completely synthetic. The non-resolvable terminal repeat may be a non-AAV viral sequence that is not recognized by the AAV Rep proteins, or it can be an AAV terminal repeat that has been modified (e.g., by insertion, substitution and/or deletion) so that it is no longer recognized by the AAV Rep proteins. Further, a non- resolvable terminal repeat can be any terminal repeat that is non-resolvable under the conditions used to produce the virus vector. For example, the non- resolvable terminal repeat may not be recognized by the Rep proteins used to replicate the vector genome. To illustrate, the non-resolvable terminal repeat can be an autonomous parvovirus terminal repeat or a virus terminal repeat other than a parvovirus terminal repeat that is not recognized by AAV Rep proteins. In a preferred embodiment, the resolvable terminal repeat and Rep proteins may be from one AAV serotype (e.g. AAV8) and the non- resolvable terminal repeat is from another AAV serotype (e.g., AAV2) that is not recognized by the AAV8 Rep proteins, such that resolution is substantially reduced or eliminated. Further, an AAV terminal repeat can be modified so that resolution by the AAV Rep proteins is substantially reduced or eliminated. The non-resolvable terminal repeat can be any inverted repeat sequence that forms a hairpin structure and cannot be nicked by the AAV Rep proteins.

Parvoviral ITR nucleotide sequences are typically palindromic sequences, comprising mostly complementary, symmetrically arranged sequences also referred to as "A," "B," and "C" regions. The ITR functions as an origin of replication, a site having a "cis" role in replication, i.e., being a recognition site for trans acting replication proteins such as e.g. Rep 78 (or Rep68) which recognize the palindrome and specific sequences internal to the palindrome. One exception to the symmetry of the ITR sequence is the "D" region of the ITR. It is unique (not having a complement within one ITR). Nicking of single-stranded DNA occurs at the junction between the A and D regions. It is the region where new DNA synthesis initiates. The D region normally sits to one side of the palindrome and provides directionality to the nucleic acid replication step. A parvovirus replicating in a mammalian cell typically has two ITR sequences. It is, however, possible to engineer an ITR so that binding sites are on both strands of the A regions and D regions are located symmetrically, one on each side of the palindrome. On a double-stranded circular DNA template (e.g., a plasmid), the Rep78- or Rep68- assisted nucleic acid replication then proceeds in both directions and a single ITR suffices for parvoviral replication of a circular vector. Thus, one ITR nucleotide sequence can be used in the context of the present invention. Preferably, however, two or another even number of regular ITRs are used. Most preferably, two ITR sequences are used. Accordingly, in the invention, at least one ITR may be used, i.e. one ITR may be used, although more typically two ITRs will be used.

In a preferred embodiment, the AAV genome of the invention comprises a polynucleotide which comprises an expression cassette formed by the liver-specific promoter, the sequence encoding IGF-I or a functionally equivalent variant thereof, the polyadenylation signal, wherein said expression cassette is flanked by AAV ITRs. The liver-specific promoter is a hybrid promoter comprising the albumin enhancer and the alpha 1-antitrypsin promoter region.

The viral genome of the invention can be a single-stranded parvovirus vector, such as an AAV vector. In a preferred embodiment, the AAV vector is a single-stranded AAV (ssAAV). The expression "single-stranded parvovirus vector", as used herein, relates to a single-stranded polynucleotide (typically, DNA) packaged within an AAV capsid. As used herein, the term "single-stranded", when used in reference to a nucleic acid molecule, refers to a nucleic acid molecule which is not hybridized to another nucleic acid molecule and has no regions which will hybridize intramolecularly either under physiological conditions or stringent conditions. This is in contrast to double-stranded target which exists as two strands of nucleic acid which are held together by inter-strand base pairing interactions. The single stranded nucleic acid molecule is either sense strand or antisense strand, as both strands are equally infectious.

The viral genome of the invention can further be a duplexed parvovirus vector as described in international patent publication WO 01/92551 and McCarty et al., (2003) Gene Therapy 10:2112-2118. In the context of the present invention the terms "double-stranded parvovirus vector" and "duplexed parvovirus vector" have the same meaning and they are indistinctly used along the description. In a particular embodiment the parvoviral vector is an AAV vector, preferably a double-stranded AAV. In addition, the AAV capsid or vector genome can contain other modifications, including insertions, deletions and/or substitutions. The rAAV vector comprises an AAV capsid derived from, without limitation, an AAV1, AAV2, AAV3, AAV4, AAV5, AAV6, AAV7, AAV8, AAV9, AAV10, AAV11 or AAV12 capsid, including modified forms thereof. Optionally, the capsid can be an AAV2, AAV3 or AAV6 capsid or a modified form thereof e.g. modified capsids generated using shuffling techniques and AAV capsid libraries.

In representative embodiments of the invention, the viral genome is a duplexed parvovirus vector, wherein the recombinant vector genome comprises 5' and 3 AAV terminal repeats (that are resolvable), the heterologous nucleotide sequence encoding IGF-I or a functional variant thereof and a non-resolvable terminal repeat as recited in the claims. Duplexed parvovirus vectors and their production are described in international patent publication WO 01/92551 and McCarty et al., (2003) Gene Therapy 10:2112-2118.

Typically, the rAAV vector genome only retains the minimal terminal repeat sequence(s) (each 145 bases) so as to maximize the size of the transgene that can be efficiently packaged by the vector.

In general, duplexed parvovirus vectors are dimeric self-complementary polynucleotides (typically, DNA) packaged within an AAV capsid. In some aspects, the recombinant viral genome that is packaged within the capsid is essentially a "trapped" AAV replication intermediate that cannot be resolved to produce the plus and minus polarity strands. Duplexed parvovirus vectors appear to circumvent the need for host cell mediated synthesis of complementary DNA inherent in conventional rAAV vectors, thereby addressing one of the limitations of rAAV vectors.

The duplexed parvovirus vectors are fundamentally different from conventional rAAV vectors, and from the parent AAV, in that the viral DNA may form a double-stranded hairpin structure due to intrastrand base pairing, and the DNA strands of both polarities are encapsidated. Thus, the duplexed parvovirus vector is functionally similar to double-stranded DNA virus vectors rather than the AAV from which it was derived. This feature addresses a previously recognized shortcoming of rAAV mediated gene transfer, which is the limited propensity of the desired target cell to synthesize complementary DNA to the single-stranded genome normally encapsidated by AAV.

While not wishing to be held to any particular theory it is possible that the virion genome is retained in a single-stranded form while packaged within the viral capsid. Upon release from the capsid during viral infection, it appears that the dimeric molecule "snaps back" or anneals to form a double-stranded molecule by intra-strand base pairing, with the non- resolvable TR sequence forming a covalently-closed hairpin structure at one end. This double-stranded viral DNA obviates the need for host cell mediated second-strand synthesis, which has been postulated to be a rate-limiting step for AAV transduction.

In the case of liver tissue cells, duplexed parvovirus vectors may be advantageous because they may provide a faster onset of gene expression and/or higher levels of gene expression, thereby permitting lower dosages, which in turn may result in a reduced likelihood and/or extent of inflammation in target tissues.

The duplexed parvovirus vector genome generally comprises in the 5' to 3' direction, (i) a resolvable AAV terminal repeat, (ii) a heterologous nucleotide sequence of interest (coding or noncoding strand), (iii) a non- resolvable terminal repeat, (iv) a complementary sequence or substantially complementary (e.g., at least about 90%, 95%, 98%, 99% or more) sequence to the heterologous nucleotide sequence of interest of (ii), and (v) a resolvable AAV terminal repeat. Those skilled in the art will appreciate that the vector genome can comprise other sequences (e.g., intervening sequences between the sequences specifically described above).

In particular embodiments, the sequences in each half of the vector genome (e.g., the entire sequence or the sequences between the AAV terminal repeat and the non-resolvable terminal repeat) are substantially complementary (i.e., at least about 90%, 95%, 98%, 99% nucleotide sequence complementarity or more), so that the vector genome may form double-stranded molecules due to base-pairing between the complementary sequences. In other words, the vector genome is essentially an inverted repeat with the two halves joined by the non-resolvable terminal repeat. In particular embodiments, the two halves of the vector genome (i.e., the entire sequence or the sequences between the AAV terminal repeats and the non- resolvable terminal repeat) are essentially completely self-complementary (i.e., contain an insignificant number of mismatched bases) or completely self- complementary.

In other embodiments, the two strands of the heterologous nucleotide sequence of interest (with or without regulatory elements) are substantially complementary (i.e., at least about 90%, 95%, 98%, 99% nucleotide sequence complementarity or more). In particular embodiments, the two strands of the heterologous nucleotide sequence(s) are essentially completely self-complementary (i.e., contain an insignificant number of mismatched bases) or completely self-complementary.

In general, the vector genome of the duplexed parvoviruses can contain positions or regions of non-complementarity to the extent that expression of the heterologous nucleotide sequence(s) from the duplexed parvovirus vector is enhanced (e.g., earlier onset and/or higher level of expression) than from a corresponding rAAV vector. The duplexed parvoviruses of the present invention provide the host cell with a double-stranded molecule that addresses one of the drawbacks of rAAV vectors, i.e., the need for the host cell to convert the single-stranded rAAV virion DNA into a double-stranded DNA. The presence of any substantial regions of non- complementarity within the virion DNA, in particular, within the heterologous nucleotide sequence(s) may be recognized by the host cell, and may result in DNA repair mechanisms being recruited to correct the mismatched bases, thereby counteracting the advantageous characteristics of the duplexed parvovirus vectors, e.g., reduction or elimination of the need for the host cell to process the viral template.

A non-resolvable AAV terminal repeat can be produced by any method - known in the art. For example, insertion into the terminal repeat will displace the nicking site (i.e., trs) and result in a non-resolvable terminal repeat. The designation of the various regions or elements within the terminal repeat are known in the art (see, e.g., BERNARD N. FIELDS et al., VIROLOGY, volume 2, chapter 69, Figure 5, 3d ed., Lippincott- Raven Publishers and Figure 6 of WO01/925551). An insertion can also be made into the sequence of the terminal resolution site (trs). Alternatively, an insertion can be made at a site between the Rep Binding Element (RBE) within the A element and the trs (see, Figure 6 of WO 01/925551). The core sequence of the AAV trs site is known in the art and has been described (Snyder et al., (1990) Cell, 60:105; Snyder et al., (1993) J. Virology 67:6096; Brister and Muzyczka, (2000) J. Virology 74:7762; Brister and Muzyczka, (1999) J. Virology 73:9325. For example, Brister and Muzyczka, (1999) J. Virology 73:9325, describes a core trs sequence of 3'- CCGGT/TG-5 adjacent to the D element. Snyder et al., (1993) J. Virology 67:6096, identified the minimum trs sequence as 3'-GGT/TGA-5' which substantially overlaps the sequence identified by Brister and Muzyczka.

The insertion can be of any suitable length that substantially reduces (e.g., by at least about 50%, 60%, 70%, 80%, 90%. 95%, 98% or greater) or eliminates resolution of the terminal repeat. The insertion can be at least about 3, 4, 5, 6, 10, 15, 20 or 30 nucleotides or more. There are no particular upper limits to the size of the inserted sequence, as long as suitable levels of viral replication and packaging are achieved (e.g., the insertion can be as long as 50, 100, 200 or 500 nucleotides or longer).

As another approach, the terminal repeat can be rendered non- resolvable by deletion of the trs site. The deletions may extend 1, 3, 5, 8, 10, 15, 20, 30 nucleotides or more beyond the trs site, as long as the template retains the desired functions. In addition to the trs site, some or all of the D element can be deleted (see, e.g., McCarty et al. (2003) Gene Therapy 10:2112-2118; and WO 01/92551). Deletions can further extend into the A element; however those skilled in the art will appreciate that it may be advantageous to retain the RBE in the A element, e.g., to facilitate efficient packaging. Deletions into the A element can be 2, 3, 4, 5, 8, 10, or 15 nucleotides in length or more, as long as the non-resolvable terminal repeat retains any other desired functions: Further, some or all of the viral sequences going beyond the D element outside the terminal repeat sequence (e.g., to the right of the D element in Figure 6 of PCT Publication No. WO01/925551) can be deleted to reduce or prevent the process of gene conversion to correct the altered terminal repeat.

As still a further alternative, the sequence at the nicking site can be mutated so that resolution by Rep protein is reduced or substantially eliminated. For example, A and/or C bases can be substituted for G and/or T bases at or near the nicking site. The effects of substitutions at the terminal resolution site on Rep cleavage have been described by Brister and Muzyczka, (1999) J. Virology 73:9325.

As a further alternative, nucleotide substitutions in the regions surrounding the nicking site, which have been postulated to form a stem-loop structure, can also be used to reduce Rep cleavage at the terminal resolution site. Those skilled in the art will appreciate that the alterations in the non- resolvable terminal repeat can be selected so as to maintain desired functions, if any, of the altered terminal repeat (e.g., packaging, Rep recognition, and/or site-specific integration, and the like).

Further, the non-resolvable terminal repeat can be rendered resistant to the process of gene conversion as described by Samulski et al., (1983) Cell 33:135. Gene conversion at the non-resolvable terminal repeat will restore the trs site, which will generate a resolvable terminal repeat. Gene conversion results from homologous recombination between the resolvable terminal repeat and the altered terminal repeat. One strategy to reduce gene conversion is to produce virus using a cell line (e.g., mammalian) that is defective for DNA repair, as known in the art, because these cell lines will be impaired in their ability to correct the mutations introduced into the viral template.

Alternatively, templates that have a substantially reduced rate of gene conversion can be generated by introducing a region of non-homology into the non-resolvable terminal repeat. Non-homology in the region surrounding the trs element between the non-resolvable terminal repeat and the unaltered terminal repeat on the template will reduce or even substantially eliminate gene conversion. Any suitable insertion or deletion may be introduced into the non- resolvable terminal repeat to generate a region of non-homology, as long as gene conversion is reduced or substantially eliminated. Strategies that employ deletions to create non-homology are preferred. It is further preferred that the deletion does not unduly impair replication and packaging of the template. In the case of a deletion, the same deletion may suffice to impair resolution of the trs site as well as to reduce gene conversion.

As a further alternative, gene conversion may be reduced by insertions into the non-resolvable terminal repeat or, alternatively, into the A element between the RBE and the trs site. The insertion is typically at least about 3, 4, 5, 6, 10, 15, 20 or 30 nucleotides or more nucleotides in length. There is no particular upper limit to the size of the inserted sequence, which may be as long as 50, 100, 200 or 500 nucleotides or longer, however, generally, the insertion is selected so that it does not unduly impair replication and packaging of the vector genome.

Non-resolvable terminal repeats and duplexed parvovirus vectors are described in international patent publication WO 01/92551 and McCarty et al., (2003) Gene Therapy 10:2112-2118).

The rAAV vector of the invention may also comprise a transcription termination signal. While any transcription termination signal may be included in the vector of the invention, preferably, the transcription termination signal is the SV40 transcription termination signal.

Modified "AAV" sequences also can be used in the context of the present invention, e.g. for the production of rAAV vectors in insect cells. Such modified sequences e.g. include sequences having at least about 70%, at least about 75%, at least about 80%, at least about 85%, at least about 90%, at least about 95%, or more nucleotide and/or amino acid sequence identity (e.g., a sequence having about 75-99% nucleotide sequence identity) to an AAV1, AAV2, AAV3, AAV4, AAV5, AAV6, AAV7, AAV8 or AAV9 ITR, Rep, or VP can be used in place of wild-type AAV ITR, Rep, or VP sequences.

Although similar to other AAV serotypes in many aspects, AAV5 differs from other human and simian AAV serotypes more than other known human and simian serotypes. In view thereof, the production of rAAV5 can differ from production of other serotypes in insect cells. Where methods of the invention are employed to produce rAAV5, it is preferred that one or more constructs comprising, collectively in the case of more than one construct, a nucleotide sequence comprising an AAV5 ITR, a nucleotide sequence comprises an AAV5 Rep coding sequence (i.e. a nucleotide sequence comprises an AAV5 Rep78). Such ITR and Rep sequences can be modified as desired to obtain efficient production of rAAV5 or pseudotyped rAAV5 vectors in insect cells. E.g., the start codon of the Rep sequences can be modified, VP splice sites can be modified or eliminated, and/or the VP1 start codon and nearby nucleotides can be modified to improve the production of rAAV5 vectors in the insect cell.

### RECOMBINANT VIRIONS

Also described are virions obtainable by expressing a viral genome of the invention in a suitable packaging cell.

The term "virion", "recombinant virus particle" and "viral vector" are used herein interchangeably and relate to an infectious, replication-defective virus particle comprising the viral genome packaged within a capsid and, as the case may be, a lipidic envelope surrounding the capsid.

The virions may be a polyoma virion and, more preferably, an SV40 virion. An SV40 virion comprises a double-stranded circular DNA genome of 5.2 kb and a viral capsid, surrounding the viral mini-chromosome, composed of three viral-coded proteins, VP1, VP2, and VP3.

In another embodiment, if the virion is obtained by packaging of an AAV vector of the invention, the virion of the invention is a "recombinant AAV virion". The term, "recombinant AAV virion" or "rAAV virion", as used herein, refers to an infectious, replication-defective virus composed of an AAV protein shell encapsidating a heterologous nucleotide sequence of interest that is flanked on both sides by AAV ITRs and one or more Rep proteins.

The term "Cap protein", as used herein, refers to a polypeptide having at least one functional activity of a native AAV Cap protein (e.g., VP1, VP2, VP3). Examples of functional activities of Cap proteins (e.g., VP1, VP2, VP3) include the ability to induce formation of a capsid, facilitate accumulation of single-stranded DNA, facilitate AAV DNA packaging into capsids (i.e., encapsidation), bind to cellular receptors, and facilitate entry of the virion into host cells. In a preferred embodiment, the polynucleotide sequence encoding the cap gene corresponds to the AAV8 cap gene. The shell of an AAV virion shows icosahedral symmetry and usually contain a major Cap protein, usually the smallest of the Cap protein and one or two minor Cap protein or proteins.

The term "Rep protein", as used herein, refers to a polypeptide having at least one functional activity of a native AAV Rep protein (e.g., Rep 40, 52, 68, 78). A "functional activity" of a Rep protein (e.g., Rep 40, 52, 68, 78) is any activity associated with the physiological function of the protein, including facilitating replication of DNA through recognition, binding and nicking of the AAV origin of DNA replication as well as DNA helicase activity. Additional functions include modulation of transcription from AAV (or other heterologous) promoters and site-specific integration of AAV DNA into a host chromosome. In a preferred embodiment, the polynucleotide sequence encoding the rep gene corresponds to the AAV1 rep gene.

The skilled person will understand that the AAV virions of the invention may comprise capsid proteins from any AAV serotype. However, due to the different tropism of the known AAV serotypes for different cells, the AAV virions will contain a capsid protein which is more adequate for delivery to the liver cells. For transduction of liver cells rAAV virions with AAV1, AAV8 and AAV5 capsid proteins are preferred (Nathwani et al., 2007, Blood 109: 1414-1421; Kitajima et al., 2006, Atherosclerosis 186:65-73).

Additionally, the AAV genomes of the invention include also AAV genomes which have been prepared by DNA shuffling as described by Stemmer, W. P. C., (Nature 270:389-391, 1994); Schmidt-Dannert et al., (Nat. Biotech. 18:750-753, 2000) and Oreneis et al., (Nat. Struct. Biol. 9:238-242, 2001). DNA or gene shuffling involves the creation of random fragments of members of a gene family and their recombination to yield many new combinations. To shuffle AAV capsid genes, several parameters are to be considered, including: involvement of the three capsid proteins VP1, VP2, and VP3 and different degrees of homologies between 8 serotypes. To increase the likelihood of obtaining a viable rcAAV vector with a cell- or tissue-specific tropism, for example, a shuffling protocol yielding a high diversity and large number of permutations is preferred. An example of a DNA shuffling protocol for the generation of chimeric rcAAV is random chimeragenesis on transient templates (RACHITT), Coco et al., Nat. Biotech. 19:354-358, 2001.

The RACHITT method can be used to recombine two PCR fragments derived from AAV genomes of two different serotypes (e.g., AAV 1 and AAV2). For example, conservative regions of the cap gene, segments that are 85% identical, spanning approximately 1 kbp and including initiating codons for all three genes (VP1, VP2, and VP3) can be shuffled using a RATCHITT or other DNA shuffling protocol, including in vivo shuffling protocols (U.S. Pat. No. 5,093,257; Volkov et al., NAR 27:e18, 1999; and Wang P. L., Dis. Markers 16:3-13, 2000). A resulting combinatorial chimeric library can be cloned into a suitable AAV TR-containing vector (e.g., pTR-AAV2) to replace the respective fragment of the WT AAV genome. Random clones can be sequenced and aligned with parent genomes using AlignX application of Vector NTI 7 Suite Software. From the sequencing and alignment, the number of recombination crossovers per 1 Kbp gene can be determined. Alternatively, the variable domain of AAV genomes can be shuffled. For example, mutations can be generated within two amino acid clusters (amino acids 509-522 and 561-591) of AAV that likely form a particle surface loop in VP3. To shuffle this low homology domain, recombination protocols can be utilized that are independent of parent's homology (Ostermeier et al., Nat. Biotechnol. 17:1205-1209, 1999; Lutz et al., Proc. Nat. Acad. Sci. 98:11248-11253, 2001) and Lutz et al., (NAR 29:E16, 2001) or a RACHITT protocol modified to anneal and recombine DNA fragments of low homology.

Combinatorial libraries can also be constructed using insertions of short randomized oligonucleotides into certain positions of capsid genes that likely form a loop and are exposed at a particle surface to interact with a cell surface receptor (e.g., amino acids 509-522 and 561-591 in AAV2) (Xie et al, 2002, Proc. Natl.Acad.Sci.USA, 99:10405-10410). Such libraries can be used to select for virions with new cell/tissue tropisms. Selection of virions involves the protocol described in FIGS. 1B and 1C.

Methods of making AAV capsid mutants in addition to degenerate oligonucleotide synthesis, random peptide insertion, and RATCHITT methods might also be used. Examples of alternative methods include site-directed mutagenesis (Wu et al., J. Virol. 72:5919-5926); molecular breeding, nucleic acid, exon, and DNA family shuffling (Soong et al., Nat. Genet. 25:436-439, 2000; Coco et al., Nature Biotech. 2001; 19:354; and U.S. Pat. Nos. 5,837,458; 5,811,238; and 6,180,406; Kolkman and Stemmer, Nat. Biotech. 19:423-428, 2001; Fisch et al., Proceedings of the National Academy of Sciences 93:7761-7766, 1996; Christians et al., Nat. Biotech. 17:259-264, 1999); ligand insertions (Girod et al. Nat. Med. 9:1052-1056, 1999); and cassette mutagenesis (Rueda et al. Virology 263:89-99, 1999; Boyer et al., J. Virol. 66:1031-1039, 1992). For mutational analyses of the AAV capsid gene, see Wu et al., J. Virol. 74:8635-8647, 2000 and Rabinowitz et al., Virology 265;274-285, 1999.

Modified "AAV" sequences also can be used in the context of the present invention, e.g. for the production of rAAV vectors in insect cells. Such modified sequences e.g. include sequences having at least about 70%, at least about 75%, at least about 80%, at least about 85%, at least about 90%, at least about 95%, or more nucleotide and/or amino acid sequence identity (e.g., a sequence having about 75-99% nucleotide sequence identity) to an AAV1, AAV2, AAV3, AAV4, AAV5, AAV6, AAV7, AAV8 or AAV9 ITR, Rep, or VP can be used in place of wild-type AAV ITR, Rep, or VP sequences.

The Rep (Rep78/68 and Rep52/40) coding sequences may be from any AAV serotype, but preferably derived from AAV1, AAV2, and/or AAV4. The sequences coding for the VP1, VP2, and VP3 capsid proteins for use in the context of the present invention may however be taken from any of the known 42 serotypes, more preferably from AAV1, AAV2, AAV5, AAV6 or AAV8.

The invention also contemplates virions/comprising a capsid and a recombinant viral genome, wherein an exogenous targeting as recited in the claims, sequence has been inserted or substituted into the native capsid. The virion is preferably targeted (i.e., directed to a particular cell type or types) by the substitution or insertion of the exogenous targeting sequence into the capsid. Alternatively stated, the exogenous targeting sequence preferably confers an altered tropism upon the virion. As yet a further alternative statement, the targeting sequence increases the efficiency of delivery of the targeted vector to a cell.

The exogenous targeting sequence(s) may replace or substitute part or all of a capsid subunit, alternatively, more than one capsid subunit. As a further alternative, more than one exogenous targeting sequence (e.g., two, three, four, five or more sequences) may be introduced into the virion capsid. In alternative embodiments, insertions and substitutions within the minor capsid subunits (e.g., Vp1 and Vp2 of AAV) are preferred. For AAV capsids, insertions or substitutions in Vp2 or Vp3 are also preferred.

In more preferred embodiments, the exogenous targeting sequence may be any amino acid sequence encoding a peptide or protein, which is inserted or substituted into the virion capsid to alter the tropism of the virion. The native virion tropism may be reduced or abolished by insertion or substitution of the amino acid sequence. Alternatively, the insertion or substitution of the exogenous amino acid sequence may target the virion to a particular cell type(s). The exogenous targeting sequence may be any amino acid sequence encoding a protein or peptide that alters the tropism of the virion. In particular embodiments, the targeting peptide or protein may be naturally occurring or, alternately, completely or partially synthetic. Exemplary peptides and proteins include ligands and other peptides that bind to cell surface receptors present in liver cells include ligands capable of binding the Sr-B1 receptor for Apoliprotein E, galactose- and lactose-specific lectins, low density lipoprotein receptor ligands, asialoglycoprotein (galactose-terminal) ligands and the like.

Alternatively, the exogenous targeting sequence may be an antibody or an antigen-recognizing moiety thereof The term "antibody" as used herein refers to all types of immunoglobulins, including IgG, IgM, IgA, IgD, and IgE. The antibodies may be monoclonal or polyclonal and may be of any species of origin, including (for example) mouse, rat, rabbit, horse, or human, or may be chimeric antibodies. Also encompassed by the term "antibody" are bispecific or "bridging" antibodies as known by those skilled in the art. Antibody fragments include, for example, Fab, F(ab')2, and Fc fragments, and the corresponding fragments obtained from antibodies other than IgG. Such fragments may be produced by known techniques.

The exogenous amino acid sequence inserted into the virion capsid may be one that facilitates purification or detection of the virion. It is not necessary that the exogenous amino acid sequence also alters the virion of the modified parvovirus. For example, the exogenous amino acid sequence may include a poly-histidine sequence that is useful for purifying the virion over a nickel column, as is known to those skilled in the art or an antigenic peptide or protein that may be employed to purify the virion by standard immunopurification techniques. Alternatively, the amino acid sequence may encode a receptor ligand or any other peptide or protein that may be used to purify the modified virion by affinity purification or any other techniques known in the art (e.g., purification techniques based on differential size, density, charge, or isoelectric point, ion-exchange chromatography, or peptide chromatography).

It is preferred to insert the exogenous amino acid sequence within the parvovirus minor Cap subunits, e.g., within the AAV Vp1 and Vp2 subunits. Alternately, insertions in Vp2 or Vp3 are preferred.

Preferred AAV virions are modified to reduce the host response as reviewed by Russell (2000, J. Gen. Virol. 81:2573-2604), or as described in US20080008690 and by Zaldumbide and Hoeben (Gene Therapy, 2008:239-246).

The virions of the invention comprise a parvoviral genome which comprises a nucleotide sequence encoding IGF-I or a functionally equivalent variant thereof having the biological activity of IGF-I which is operably linked to a liver-specific promoter comprising the albumin gene enhancer region and the alphal-antitrypsin promoter. In another embodiment, the IGF-I corresponds to a human IGF-I.

### THERAPEUTIC METHODS

The authors of the present invention have observed that the administration of the viral vectors to animals suffering from CCl₄-induced cirrhosis results in significant improvement of liver function as measured by biochemical liver tests (decrease in serum AST, ALT, ALP and bilirubin and increase in serum albumin) and histochemical observation (see examples 3 and 10). Moreover, the virions of the invention results in a induction of fibrolysis in the cirrhotic liver (see examples 4 and 11) and in a reduction of profibrogenic factors (see examples 5 and 12).

Thus, in another aspect, the invention relates to a virion of the invention for use as a medicament. In another aspect, the invention pertains to a pharmaceutical composition comprising a virion as herein defined above. The pharmaceutical composition further preferably comprises a pharmaceutically acceptable carrier. Any suitable pharmaceutically acceptable carrier or excipient can be used in the present compositions (See e.g., Remington: The Science and Practice of Pharmacy, Alfonso R. Gennaro (Editor) Mack Publishing Company, April 1997). Preferred pharmaceutical forms would be in combination with sterile saline, dextrose solution, or buffered solution, or other pharmaceutically acceptable sterile fluids. Alternatively, a solid carrier, may be used such as, for example, microcarrier beads.

In yet another aspect, the invention relates to a virion as claimed for use in a method for the treatment and/or prevention or prophylaxis of hepatic cirrhosis or hepatic fibrosis.

In another aspect, the invention relates to the use of a virion according to the invention for the manufacture of a medicament for the prevention and/or treatment of hepatic cirrhosis or hepatic fibrosis.

In yet another aspect, the invention relates to a virion according to the invention for use in the treatment and/or prevention of hepatic cirrhosis or hepatic fibrosis.

The term "hepatic cirrhosis", as used herein, relates to a condition in which the liver slowly deteriorates and malfunctions because liver tissue is replaced by fibrous scar tissue and regenerative nodules. This results in a partial block in the flow of blood through the liver as well as in an impairment in the liver's ability to control infections, remove bacteria and toxins from the blood, process nutrients, hormones, and drugs, make proteins that regulate blood clotting and produce bile to help absorb fats-including cholesterol-and fat-soluble vitamins. The therapeutic method of the invention is suitable for the treatment of cirrhosis of different causes, including alcohol-related cirrhosis, chronic hepatitis C, B or D, non-alcoholic fatty liver disease (NAFLD), autoimmune hepatitis, primary or secondary biliary cirrhosis, primary sclerosing cholangitis, inherited diseases such as Cystic fibrosis, alpha-1 antitrypsin deficiency, hemochromatosis, Wilson disease, galactosemia, and glycogen storage diseases.

The term "liver fibrosis", as used herein, relates to a condition characterized by an increase accumulation in the liver of extracellular matrix proteins, including collagen and includes fibrosis scores of 1 (minimal scarring), 2 (scarring has occurred and extends outside the areas in the liver that contains blood vessels), 3 (bridging fibrosis is spreading and connecting to other areas that contain fibrosis) or 4 (cirrhosis or advanced scarring of the liver) according to the metavir scoring system; or an score of 1-4, 5-8, 9-12 or 13-18 according to the Knodell score.

The amount of virions and the time of administration of such compositions will be within the purview of the skilled artisan having benefit of the present teachings. In fact, the inventors contemplate that the administration of therapeutically-effective amounts of the virions of the invention may be achieved by a single administration, such as for example, a single injection of sufficient numbers of infectious particles to provide therapeutic benefit to the patient undergoing such treatment. Alternatively, in some circumstances, it may be desirable to provide multiple, or successive administrations of the virion compositions, either over a relatively short, or a relatively prolonged period of time, as may be determined by the medical practitioner overseeing the administration of such compositions. For example, the number of infectious particles administered to a mammal may be on the order of about 10⁷, 10⁸, 10⁹, 10¹⁰, 10¹¹, 10¹², 10¹³, or even higher, infectious particles/ml given either as a single dose, or divided into two or more administrations as may be required to achieve therapy of the particular disease or disorder being treated. In fact, in certain embodiments, it may be desirable to administer two or more different virion vector compositions, either alone, or in combination with one or more other therapeutic drugs to achieve the desired effects of a particular therapy regimen. In most virion-based gene therapy regimens, the inventors believe that the use of a liver-specific promoter to control the expression of IGF-I or of the functional equivalent variant thereof will result in that a lower titer of infectious particles will be required when using the virions according to the invention than compared to conventional gene therapy protocols.

In particularly preferred embodiments of the invention, the nucleotide sequence of interest is formulated for delivery to the liver of the subject. Administration to the liver may be achieved by any method known in the art, including, but not limited to intravenous administration, intraportal administration, intrabiliary administration, intra-arterial administration, and direct injection into the liver parenchyma. In a preferred embodiment, the virion is formulated for administration intra-arterially. In a still more preferred embodiment, the virion is formulated for intra-arterial administration through the hepatic artery.

### METHODS FOR THE PREVENTION AND/OR TREATMENT OF HEPATIC CIRRHOSIS AND FIBROSIS USING PARVOVIRAL VECTORS ENCODING IGF-I

The results provided by the authors of the present invention have shown that the administration of recombinant parvoviral virions comprising the IGF-I coding sequence results in an improvement of liver function in an animal model of cirrhosis (see examples 3-6 and 10-13). Moreover, the results observed with other viral vectors indicate that IGF-I may delay the progression of disease if applied before the development of the disease. Thus, the virions of the present invention are also suitable for preventing the development of cirrhosis or liver fibrosis.

Thus, in another aspect, the invention relates to a recombinant parvovirus as claimed for use in medicine, and more preferably for use in the treatment and/or prevention of hepatic cirrhosis or fibrosis.

In another aspect, the invention relates to the use of a recombinant parvovirus as claimed for the preparation of a medicament for the treatment and/or prevention of hepatic cirrhosis or fibrosis.

The term "treatment", as used herein, refers to the act of reversing, alleviating, or inhibiting the progress of the disorder or condition to which such term applies, or one or more symptoms of such disorders or condition.

The term "prevention", as used herein, refers to the act of keeping from happening, existing, or alternatively delaying the onset or recurrence of a disease, disorder, or condition to which such term applies, or of one or more symptoms associated with a disease, disorder, or condition.

The terms "liver cirrhosis" and "liver fibrosis" have been previously described in detail.

The term "parvovirus" has also been described in detail above in connection with the viral vectors of the invention. Preferably, the parvovirus is an adenoassociated virus. Still more preferably, the AAV comprises a genome comprising the sequence encoding IGF-I or the functionally equivalent variant thereof flanked by ITRs, wherein said ITRs are from AAV1, AAV2 and/or AAV4. In a still more preferred embodiment, the AAV is an AAV1, AAV5, AAV6 or AAV8-pseudotyped with AAV1, AAV2 and/or AAV4, i.e. it contains the cap proteins from AAV1, AAV5, AAV6 or AAV8. In a preferred embodiment the recombinant parvovirus is a single-stranded parvovirus, preferably, a single-stranded AAV. In another preferred embodiment the recombinant parvovirus is a double-stranded parvovirus, preferably, a double-stranded AAV.

The term "IGF-I" has been described in detail before when referring to the viral vectors of the invention. In a preferred embodiment, the IGF-I corresponds to human IGF-I.

The sequence encoding IGF-I or the functionally equivalent variant thereof may be operably linked to a promoter region. Suitable promoters for use in the virions for use in the therapeutic methods include any promoter which is able to active transcription of downstream sequences in liver cells, including constitutive promoters as well as liver specific promoters. Constitutive promoters suitable for expression of heterologous sequences in liver include, without limitation, a promoter of hypoxanthine phosphoribosyl transferase (HPTR), a promoter of the adenosine deaminase, a promoter of the pyruvate kinase, a promoter of β-actin, an elongation factor 1 alpha (EF1) promoter, a phosphoglycerate kinase (PGK) promoter, a ubiquitin (Ubc) promoter, an albumin promoter, promoters of the intermediate filaments (desmin, neurofilaments, keratin, GFAP, and the like) and other constitutive promoters. Exemplary viral promoters which function constitutively in eukaryotic cells include, for example, the SV40 early promoter region (Bernoist and Chambon, 1981, Nature 290:304-310), the promoter contained in the 3' long terminal repeat of Rous sarcoma virus (Yamamoto et al., 1980, Cell 22:787-797), the herpes thymidine kinase promoter (Wagner et al., 1981, Proc. Natl. Acad. Sci. U.S.A. 78:1441-1445).

The promoter which is operably linked to the sequence encoding IGF-I or the functionally equivalent variant thereof is a liver specific promoter comprising the albumin gene enhancer region and the alpha1-antitrypsin promoter.

The therapeutic method involves the administration of parvoviral virions encoding IGF-I or a functionally equivalent variant thereof by transducing the cells by incubation with the virions/viral particles. The cells may be present in an organism, in which case the cells are reachable by needle injection, jet injection or particle gun. On the other hand, the cells to be transduced can also be isolated from an organism, be infected outside the organism and then be returned to the organism again. Such cells are referred to as autologous cells. Moreover, as to the organism it is also possible to use allogenic cells for the transduction. In this connection, it is favourable for these cells to belong to an HLA type corresponding to the organism. The person skilled in the art knows methods of providing cells with a certain HLA type. Preferable titer of the virion preparation is usually between 10⁷ and 10⁹ infectious viruses/ml.

The present invention finds use in both veterinary and medical applications. Suitable subjects include both avians and mammals, with mammals being preferred. The term "avian" as used herein includes, but is not limited to, chickens, ducks, geese, quail, turkeys and pheasants. The term "mammal" as used herein includes, but is not limited to, humans, bovines, ovines, caprines, equines, felines, canines, lagomorphs, etc. Human subjects are the most preferred. Human subjects include fetal, neonatal, infant, juvenile and adult subjects.

Therefore, the subject matter of the present invention also relates to a medicament which contains a parvoviral particle according to the invention, more preferably an AAV particle. Here, the medicament may additionally contain a pharmaceutically acceptable carrier. Suitable carriers and the formulation of such medicaments are known to the person skilled in the art. Suitable carriers comprise e.g. phosphate-buffered saline solutions, water, emulsions, e.g. oil/water emulsions, wetting agents, sterile solutions, etc. The kind of carrier depends on how to administer the parvoviral vector packaging plasmid and/or parvoviral particle according to the invention. A suitable dosage is determined by the attending physician and depends on various factors, e.g. the patient's age, sex and weight, the severity of the disease, the kind of administration, etc. It has turned out that by means of inventive parvoviral vector packaging plasmids and/or particles it is possible to obtain high transduction rates with the most different cells, e.g. primary cells of the cornea epithelium or muscle cells.

Administration of the parvovirus particles of the present invention to a human subject or an animal in need thereof can be by any means known in the art for administering virus vectors. Exemplary modes of administration include oral, rectal, transmucosal, topical, transdermal, inhalation, parenteral (e.g., intravenous, subcutaneous, intradermal, intramuscular, and intraarticular) administration, and the like, as well as direct tissue or organ injection, alternatively, intrathecal, direct intramuscular, intraventricular, intravenous, intraperitoneal, intranasal, or intraocular injections. Injectables can be prepared in conventional forms, either as liquid solutions or suspensions, solid forms suitable for solution or suspension in liquid prior to injection, or as emulsions. Alternatively, one may administer the virus in a local rather than systemic manner, for example, in a depot or sustained-release formulation.

In particularly preferred embodiments of the invention, the virion is formulated for delivery to the liver of the subject. Administration to the liver may be achieved by any method known in the art, including, but not limited to intravenous administration, intraportal administration, intrabiliary administration, intra-arterial administration, and direct injection into the liver parenchyma. In a preferred embodiment, the virion is formulated for intra-arterial administration. In a still more preferred embodiment, the virion is formulated for intra-arterial administration through the hepatic artery.

Dosages of the inventive parvovirus particles will depend upon the mode of administration, the individual subject's condition, the particular virus vector, and the gene to be delivered, and can be determined in a routine manner. Exemplary doses for achieving therapeutic effects are virus titers of at least about 10⁵, 10⁶, 10⁷, 10⁸, 10⁹, 10¹⁰, 10¹¹, 10¹², 10¹³, 10¹⁴ transducing units or more, preferably about 10⁸ - 10¹³ transducing units, yet more preferably 10¹² transducing units.

In particular embodiments of the invention, the virion is formulated for more than one administration (e.g., two, three, four, or more administrations) to achieve therapeutic levels of gene expression. According to this embodiment, and as described above, it is preferred to use parvovirus vectors having different antigenic properties for each administration to obviate the effects of neutralizing antibodies.

### METHODS FOR PREPARING RECOMBINANT AAV VIRIONS

In yet another aspect, the invention provides an in vitro method as claimed for producing recombinant AAV virions.

The elements forming the first nucleic acid sequence are essentially as described previously in the context of the viral vectors of the invention. In a preferred embodiment, the first nucleic acid sequence further comprises a polyadenylation signal downstream of the sequence encoding IGF-I or the functional equivalent thereof. Suitable polyadenylation signals have been described previously. By way of an example the polyadenylation signals is the SV40 polyadenylation signal.

The liver specific promoter is a hybrid promoter comprising the albumin enhancer region and the promoter of the α1-antitrypsin.

In order to facilitate packaging, the recombinant vector genome is generally about 80% to about 105% of the size of the wild-type genome and comprises an appropriate packaging signal. To facilitate packaging into an AAV capsid, the genome is preferably approximately 5.2 kb in size or less. In other embodiments, the genome is preferably greater than about 3.6, 3.8, 4.0, 4.2, or 4.4 kb in length and/or less than about 5.4, 5.2, 5.0 or 4.8 kb in length. Alternatively stated, the heterologous nucleotide sequence(s) will typically be less than about 5 kb in length (more preferably less than about 4.8 kb, still more preferably less than about 4.4 kb in length, yet more preferably less than about 4.2 kb in length) to facilitate packaging of the recombinant genome by the AAV capsid.

The second and third nucleic acid sequences needed for the production of a virion of the invention are the so-called "AAV helper function" and comprise one, or both of the major AAV ORFS, namely the rep and cap coding regions, or functional homologues thereof. Suitable nucleic acid sequences encoding rep and cap proteins for use in the method of the invention have been described in detailed above in relation to the virions of the invention.

One of skill in the art will appreciate, however, that the first, second and third nucleic acid sequences can be provided on two or more vectors in various combinations. As used herein, the term "vector" includes any genetic element, such as a plasmid, phage, transposon, cosmid, chromosome, artificial chromosome, virus, virion, etc., that is capable of replication when associated with the proper control elements and that can transfer gene sequences between cells. Thus, the term includes cloning and expression vehicles, as well as viral vectors.

The AAV rep and/or cap genes can alternatively be provided by a packaging cell that stably expresses the genes (see, e.g., Gao et al., (1998) Human Gene Therapy 9:2353; Inoue et al., (1998) J. Virol. 72:7024; U.S. Pat. No. 5,837,484; WO 98/27207; U.S. Pat. No. 5,658,785; WO 96/17947).

In a preferred embodiment, the second and third polynucleotides may be provided in a single vector, which is usually referred to as an AAV helper function vector. Examples of vectors suitable for use with the present invention include pHLP19, described in U.S. Pat. No. 6,001,650 and pRep6cap6 vector, described in U.S. Pat. No. 6,156,303.

In other particular embodiments, the second and third nucleic acid sequences are in the form of an adenovirus helper virus which may be a hybrid helper virus that encodes AAV Rep and/or capsid proteins. Hybrid helper Ad/AAV vectors expressing AAV rep and/or cap genes and methods of producing AAV stocks using these reagents are known in the art (see, e.g., U.S. Pat. No. 5,589,377; and U.S. Pat. No. 5,871,982, U.S. Pat. No. 6,251,677; and U.S. Pat. No. 6,387,368). Preferably, the hybrid Ad of the invention expresses the AAV capsid proteins (i.e., VP1, VP2, and VP3). Alternatively, or additionally, the hybrid adenovirus can express one or more of AAV Rep proteins (i.e., Rep40, Rep52, Rep68 and/or Rep78). The AAV sequences can be operatively associated with a tissue-specific or inducible promoter.

In another particular embodiment, the cell used in the preparation of a recombinant AAV virion is an insect cell and the first, second and third nucleic acid sequences are comprised in a baculoviral vector.

In a preferred embodiment the recombinant AAV virion produced by this method is a single-stranded AAV virion. In another preferred embodiment the recombinant AAV virion produced is a double-stranded AAV virion.

The different nucleic acid sequences are contacted with the cell under conditions adequate for entry of said nucleic acid sequences in the cell. A number of transfection techniques suitable for this purpose are generally known in the art and include calcium phosphate co-precipitation, direct micro-injection into cultured cells, electroporation, liposome mediated gene transfer, lipid-mediated transduction and nucleic acid delivery using high-velocity microprojectiles.

Component (d) may comprise a nucleic acid sequence encoding for non-AAV derived viral and/or cellular functions upon which AAV is dependent for replication (i.e., "accessory functions"). The accessory functions include those functions required for AAV replication, including, without limitation, those moieties involved in activation of AAV gene transcription, stage specific AAV mRNA splicing, AAV DNA replication, synthesis of cap expression products, and AAV capsid assembly. Viral-based accessory functions can be derived from any of the known helper viruses such as adenovirus, herpesvirus (other than herpes simplex virus type-1), and vaccinia virus.

Alternatively, the nucleic acid sequences of component (d) may be carried by the packaging cell, either episomally and/or integrated into the genome of the packaging cell. Accessory functions may be distributed between a fourth nucleic acid (component (d)) as described above and the packaging cell.

The adenoviruses encompass a number of different subgroups, although Adenovirus type 5 of subgroup C (Ad5) is most commonly used. Numerous adenoviruses of human, non-human mammalian and avian origin are known and available from depositories such as the ATCC.

It is also possible to provide the fourth nucleotide sequence as a viral DNA sequence, which is usually referred to as "helper virus". Suitable helper viruses are described in German patent application 196 44 500.0-41, for example, and they comprise e.g. also the DNA sequences disclosed in this patent application of the plasmid pTG9585 which as a helper virus DNA sequence comprises the complete adenovirus 5 sequence with the exception of the E1 region.

The sequence forming the herpes virus DNA may be incorporated into a vector, which is usually referred to as "the AAV vector packaging plasmid". The packaging plasmid can also contain helper virus DNA sequences which differ from those in pTG 9585 in that they have a deletion in the structural gene L1 of Ad5 sequence, in particular in the region of nucleotides 16614-18669.

The accessory virus DNA sequences are preferably derived from herpes virus or adenovirus, with adenovirus 5 (Ad5) being preferred.

In a part of the disclosure, the AAV vector packaging plasmid contains as helper virus DNA sequences the Ad5 genes E2A, E4 and VA, which may be derived from the pDG plasmid described in German patent application 196 44 500.0-41, for example, and which are controlled by the respective original promoter or by heterologous promoters.

In addition, the AAV vector packaging plasmid may contain a gene coding for a detectable phenotypic marker so as to prove the successful introduction of the AAV vector packaging plasmid into the target cell. In a part of the disclosure the AAV vector packaging plasmid thus contains additionally an expression cassette for the expression of a marker protein, preferably a fluorescent protein. In this connection, the term "expression cassette" refers to a combination of a gene coding e.g. for a fluorescent gene and a suitable promoter which controls this gene and a polyadenylation signal. This readily proves a transfection of the desired target cell. Examples of suitable genes coding for fluorescent proteins are RFP-(red), GFP-(green), CFP-(cyan) and YFP-(yellow) gene, RFP-(red) (Dsred-cDNA; Clontech) being preferred. Examples of suitable promoters are RSV (rous sarcoma virus) promoter, CMV (cytomegalovirus) promoter and HSV (herpes simplex virus) tk promoters, the RSV promoter being preferred. This expression cassette is inserted in the AAV vector packaging plasmid at a suitable site which can easily be determined by the person skilled in the art, preferably between the 3' end of the cap gene and the beginning of the adenoviral VA gene, e.g. in the ClaI cleavage site. This ClaI cleavage site is present in pDG.

The present disclosure relates to an AAV vector packaging plasmid, the AAV expression vector DNA sequences containing an HPV16-L1-coding DNA sequence under the control of a CMV promoter. Such an AAV vector packaging plasmid referred to as pDS2-Lh1 was deposited under DSM 14406 with DSMZ [German collection of microorganisms and cell cultures], Braunschweig, Germany, in accordance with the provisions of the Budapest Treaty of Jul. 17, 2001. Such a plasmid may carry all required accessory functions or only some of the necessary functions in which case the remaining accessory functions may be provided by the packaging cell.

An alternative method for the production of AAV virions is the use of an insect cell based system. The baculovirus expression system is well known for its use as a eukaryotic cloning and expression vector (King, L. A., and R. D. Possee, 1992, "The baculovirus expression system", Chapman and Hall, United Kingdom; O'Reilly, D. R., et al., 1992. Baculovirus Expression Vectors: A Laboratory Manual. New York: W. H. Freeman.). Advantages of the baculovirus expression system are, *inter alia,* that the expressed proteins are almost always soluble, correctly folded and biologically active. Further advantages include high protein expression levels, faster production, suitability for expression of large proteins and suitability for large-scale production.

The baculovirus expression system has successfully been used for the production of recombinant Adeno-associated virus (AAV) vectors (Urabe et al., 2002, Hum. Gene Ther. 13: 1935-1943; US 6,723,551 and US 20040197895). This system was described by *Urabe et al.* (2002, *sura*) who developed an AAV production system in insect cells.

In this system, the following nucleic acid sequences are generated:
(i) a first nucleic acid sequence comprising an expression cassette comprising a sequence encoding IGF-I or a functionally equivalent variant thereof which is operably linked to liver-specific promoter and an AAV 5'-ITR and a 3'-ITR flanking the said expression cassette,
(ii) a second nucleic acid sequence encoding an AAV rep protein and
(iii) a third nucleic acid sequence encoding an AAV cap protein.

The three nucleic acid sequences are typically carried on vectors which are insect cell-compatible vectors. The sequences may be carried out one, two or three vectors. As set out above, the first nucleotide sequence comprises at least one AAV inverted terminal repeat (ITR) nucleotide sequence. The second nucleotide sequence will typically comprise an open reading frame (ORF) comprising nucleotide sequences encoding AAV VP1, VP2, and VP3 capsid proteins operably linked to at least one expression control sequence for expression in an insect cell. The third nucleotide sequence typically comprises an open reading frame encoding Rep52 or a Rep40 coding sequence and a Rep78 or a Rep68 coding sequence operably linked to at least one expression control sequence for expression in an insect cell. The Rep proteins may be encoded by a single open reading frame.

The method for generating AAV virions comprises introducing the nucleic acid sequences, for example comprised in one, two or three insect cell-compatible vectors (typically baculoviruses), into an insect cell and maintaining the insect cell under conditions such that AAV is produced. AAV may then be recovered.

For production of AAV in insect cells some modifications may be necessary in order to achieve the correct stoichiometry of the three AAV capsid proteins (VP1, VP2 and VP3), which relies on a combination of alternate usage of two splice acceptor sites and the suboptimal utilization of an ACG initiation codon for VP2 that is not accurately reproduced by insect cells. To mimic the correct stoichiometry of the capsid proteins in insect cells Urabe et al. (2002, *supra*) proposed the use of a construct that is transcribed into a single polycistronic messenger that is able to express all three VP proteins without requiring splicing and wherein the most upstream initiator codon is replaced by the suboptimal initiator codon ACG. WO2007/046703 discloses further improvement of the infectivity of baculovirus-produced rAAV vectors based production by optimisation of the stoichiometry of AAV capsid proteins as produced in insect cells.

For expression of the AAV Rep proteins in the AAV insect cell expression system as initially developed by Urabe *et al.* (2002, *supra*), a recombinant baculovirus construct is used that harbours two independent Rep expression units (one for Rep78 and one for Rep52), each under the control of a separate insect cell promoter, the δIE1 and PolH promoters, respectively.

Kohlbrenner et al. (2005, MoI. Ther. 12: 1217-25; WO 2005/072364) reported that the baculovirus construct for expression of the two Rep protein, as used by Urabe et al., suffers from an inherent instability. By splitting the palindromic orientation of the two Rep genes in Urabe's original vector and designing two separate baculovirus vectors for expressing Rep52 and Rep78, Kohlbrenner et al. (2005, supra) increased the passaging stability of the vector. However, despite the consistent expression of Rep78 and Rep52 from the two independent baculovirus-Rep constructs in insect cells over at least 5 passages, rAAV vector yield is 5 to 10-fold lower as compared to the original baculovirus-Rep construct designed by Urabe *et al.* (2002, *supra*)*.*

In WO2007/148971, a significantly improved stability of rAAV vector production in insect cells was achieved by using a single coding sequence for the Rep78 and Rep52 proteins wherein a suboptimal initiator codon is used for the Rep78 protein that is partially skipped by the scanning ribosomes to allow for initiation of translation to also occur further downstream at the initiation codon of the Rep52 protein.

All of the modifications described above may be used in a method of the invention as described herein.

In the insect cells the first, second and third nucleic acid sequences are preferably comprised within nucleic acid vectors - they may be on the same or different vector. The insect cell may comprise three separate nucleic acid constructs, one for each of the first and second and third nucleotide sequences, or the insect cell may comprise a single type of nucleic acid construct or two vectors comprising the first, second and third nucleotide sequences distributed appropriately (for example, the first nucleic acid sequence may be located on a first vector and the second and third nucleic acid sequences may be located on a second vector).

Preferably, the second and third nucleic acid sequences are operably linked to expression control sequences for expression in an insect cell. These expression control sequences will at least include a promoter that is active in insect cells. Techniques known to one skilled in the art for expressing foreign genes in insect host cells can be used to practice the invention. Methodology for molecular engineering and expression of polypeptides in insect cells is described, for example, in Summers and Smith. 1986. A Manual of Methods for Baculovirus Vectors and Insect Culture Procedures, Texas Agricultural Experimental Station Bull. No. 7555, College Station, Tex.; Luckow. 1991. In Prokop et al., Cloning and Expression of Heterologous Genes in Insect Cells with Baculovirus Vectors' Recombinant DNA Technology and Applications, 97-152; King, L. A. and R. D. Possee, 1992, The baculovirus expression system, Chapman and Hall, United Kingdom; O'Reilly, D. R., L. K. Miller, V. A. Luckow, 1992, Baculovirus Expression Vectors: A Laboratory Manual, New York; W. H. Freeman and Richardson, C. D., 1995, Baculovirus Expression Protocols, Methods in Molecular Biology, volume 39; US 4,745,051; US2003148506; and WO 03/074714. Suitable promoters for transcription of the first and second nucleotide sequences of the invention include e.g. the polyhedron (PoIH), p10, p35, IE-I or δIE-1 promoters and further promoters described in the above references. Since it is known that in mammalian cells a less abundant expression of Rep78 as compared to Rep52 favours high vector yields (Li et al., 1997, J Virol. 71: 5236-43; Grimm et al., 1998, Hum Gene Ther. 9, 2745-2760), preferably a weaker promoter is used for driving expression of the Rep78 or 68 protein than the promoter used for expression of the Rep52 or 40 protein. E.g. the stronger polyhedron promoter may be used for expression of the Rep52 or 40 protein, the δIE1 promoter, a much weaker promoter than the PoIH promoter, may be chosen for driving expression of the Rep78 or 68 protein. Preferably, the choice of promoters for the Rep52 or 40 protein and Rep78 or 68 protein, respectively, is such that in an insect cell so as to produce in the insect cell a molar ratio of Rep78/68 to Rep52/40 in the range of 1:10 to 10:1, 1:5 to 5:1, or 1 :3 to 3:1, preferably at about 20 - 40 hours post infection, more preferably at about 30 - 40 hours post infection, using a baculovirus expression. The molar ratio of the Rep78 and Rep52 may be determined by means of Western blotting, preferably using a monoclonal antibody that recognizes a common epitope of both Rep78/68 and Rep52/40, or using e.g. a mouse anti-Rep antibody (303.9, Progen, Germany; dilution 1:50).

Preferably the nucleic acid constructs for expression of the second and third nucleotide sequences of the invention in insect cells are insect cell-compatible vectors. An "insect cell-compatible vector" or "vector" is understood to be a nucleic acid molecule capable of productive transformation or transfection of an insect or insect cell. Exemplary biological vectors include plasmids, linear nucleic acid molecules, and recombinant viruses. Any vector can be employed as long as it is insect cell- compatible. The vector may integrate into the insect cells genome but the vector may also be episomal. The presence of the vector in the insect cell need not be permanent and transient episomal vectors are also included. The vectors can be introduced by any means known, for example by chemical treatment of the cells, electroporation, or infection. In a preferred embodiment, the vector is a baculovirus, a viral vector, or a plasmid. In a more preferred embodiment, the vector is a baculovirus, i.e. the construct is a baculoviral vector. Baculoviral vectors and methods for their use are described in the above cited references on molecular engineering of insect cells.

The insect cell may be any cell that is suitable for the production of heterologous proteins. Preferably the insect cell allows for replication of baculoviral vectors and can be maintained in culture. More preferably the insect cell also allows for replication of recombinant parvoviral vectors, including rAAV vectors. For example, the cell line used can be from *Spodoptera frugiperda,* Drosophila cell lines, or mosquito cell lines, e.g., *Aedes albopictus* derived cell lines. Preferred insect cells or cell lines are cells from the insect species which are susceptible to baculovirus infection, including e.g. Se301, SeIZD2109, SeUCR1, Sf9, Sf900+, Sf21, BTI-TN-5B1-4, MG-I, Tn368, HzAmI, Ha2302, Hz2E5, High Five (Invitrogen, CA, USA) and expresSF+^{®} (US 6,103,526; Protein Sciences Corp., CT, USA).

A preferred insect cell for use in the method of the invention is an insect cell for production of recombinant parvoviral vectors. This insect cell further comprises, in addition to the above described "second " and "third" nucleic acid sequences, a first nucleic acid construct comprising at least one parvoviral inverted terminal repeat (ITR) nucleotide sequence and a sequence encoding IGF-I or a functionally equivalent variant thereof which is operably linked to liver-specific promoter.

The number of nucleic acid constructs employed (to deliver the three nucleic acid sequences described above) in the insect cell for the production of the recombinant parvoviral (rAAV) vector is not limiting in the invention. For example, one, two, three, four, five, or more separate constructs can be employed to produce rAAV in insect cells in accordance with the methods of the present invention. If five constructs are employed, one construct encodes AAV VP 1, another construct encodes AAV VP2, yet another construct encodes AAV VP3, still yet another construct encodes the Rep protein as defined above and a final construct comprises at least one AAV ITR. If fewer than five constructs are used, the constructs can comprise various combinations of the at least one AAV ITR and the VP1, VP2, VP3, and the Rep protein coding sequences.

"Packaging" as used herein refers to a series of subcellular events that result in the assembly and encapsidation of a viral vector, particularly a rAAV vector. Thus, when a suitable vector is introduced into a packaging cell line under appropriate conditions, it can be assembled into a viral particle. Functions associated with packaging of viral vectors, particularly rAAV vectors, are described herein and in the art.

Step (ii) of the method for producing AAV virions comprises the recovery of the virions from the packaging cells. For this purpose, the virus-containing sample is subjected to one or more purification steps, including density gradient separation and chromatography.

An example of a method for purifying rAAV virions includes several steps. First, a plurality of cells infected with rAAV virions is provided. From these infected cells, rAAV virions are collected. These virions are then subjected to a density gradient separation step such as one using an iodixanol gradient. A typical iodixanol step gradient contains a 15% iodixanol step, a 25% iodixanol step, a 40% iodixanol step, and a 60% iodixanol step. The iodixanol step can further include 1M NaCl. The virion-containing iodixanol step is centrifuged, and the resultant virion-containing sample is collected from the iodixanol gradient step. This sample is then subjected to a chromatography step, such as an ion exchange or hydroxyapatite chromatography step.

Purification methods are particularly useful for purifying virions having capsids containing proteins from AAV serotypes 1 and 5 because these serotypes do not bind to heparin columns. To purify rAAV1 and rAAV5 virions, purification protocols are employed that use iodixanol density gradient centrifugation followed by anion exchange or hydroxyapatite chromatography. Iodixanol is an iodinated density gradient media originally produced as an X-ray contrast compound for injection into humans. Unlike the hyper-osmotic inorganic salt (CsCl) and sucrose gradients commonly used for fractionating macromolecules, iodixanol solutions can be made iso-osmotic at all densities. This property makes iodixanol an ideal media for analysis and downstream purification steps. In addition, iodixanol has the capacity to separate free capsid proteins and empty capsids from vector genome-containing (full) capsids. Although the use of iodixanol is preferred in the invention, other suitable density gradient media might be substituted.

Following density gradient centrifugation, rAAV vectors are purified by column chromatography. Any chromatography method that allows purification of rAAV virions may be used. For example, ion exchange chromatography can be used. Ion exchange chromatography is a method that relies on charge interactions between the protein of interest and the ion exchange matrix, which is generally composed of resins, such as agarose, dextran, and cross-linked cellulose and agarose, which are covalently bound to a charged group. Charged groups are classified according to type (cationic and anionic) and strength (strong or weak). Ion exchange chromatographic techniques generally take place in several steps: equilibration of the column to pH and ionic conditions ideal for target protein binding, reversible adsorption of the sample to the column through counterion displacement, introduction of elution conditions that change the buffer's pH or ionic strength in-order to displace-bound proteins, and elution of substances from the column in order of binding strength (weakly-bound proteins are eluted first). Ion exchange chromatography is directly upgradable from a small-scale to a bulk-scale level. Anionic exchange chromatography is a type of ionic exchange chromatography in which a negatively charged resin will bind proteins with a net positive charge. Examples of commercially available anion-exchange resins include HiTrapQ by Pharmacia; MonoQ, MonoS, MiniQ, Source 15Q, 30Q, Q Sepharose, DEAE, and Q Sepharose High Performance by Amersham Biosciences (Piscataway, N.J.); WP PEI, WP DEAM, and WP QUAT by J. T. Baker (St. Louis, Mo.); Hydrocell DEAE, and Hydrocell QA by Biochrom Labs (Terre Haute, Ind.); UNOsphere Q, Macro-Prep DEAE, and Macro-Prep HighQ by Bio-Rad (Hercules, Calif); Ceramic HyperD Q, Ceramic HyperD S, Ceramic HyperD DEAE, Trisacryl M DEAE, Trisacryl LS. DEAE, Spherodex LS DEAE, QMA Spherosil, and QMA M Spherosil by Ciphergen (Fremont, Calif.); DOWEX MONOSPHERE by Dow Liquid Separations (Midland, Mich.); Matrex Q500, Matrex A500, Matrex Q800, Matrex A800, and Matrex A200 by Millipore (Bedford, Mass.); Fractogel EMD TMAE, Fractogel EMD DEAE, and Fractogel EMD DMAE by Novagen (Madison, Wis.); Amberlite Strong Anion Exchangers Type I, Amberlite Strong Anion Exchangers Type II, DOWEX Strong Anion Exchangers, Type I, DOWES Strong Anion Exchangers Type II, Diaion Strong Anion Exchangers Type I, Diaion Strong Anion Exchangers Type I, Diaion Strong Anion Exchangers Type II, Amberlite Weak Anion Exchangers, and DOWEX Weak Anion Exchangers by Sigma-Aldrich (St. Louis, Mo.); TSK Gel DEAE-5PW-HR, TSK Gel DEAE-5PW, TSK Gel Q-5PW-HR, and TSK Gel Q-5PW by Tosoh Biosep (Montgomeryville, PA); and QA52, DE23, DE32, DE51, DE52, DE53, Express-Ion D and Express-Ion Q by Whatman (Kent, UK). For the purification of rAAV1 and AAV5 virions, anion-exchange chromatography is preferred.

Hydroxyapatite chromatography is another example of a suitable chromatography technique. Hydroxyapatite is a crystalline form of calcium phosphate. The mechanism of hydroxyapatite chromatography involves nonspecific interactions between negatively charged protein carboxyl groups and positively charged calcium ions on the resin, and positively charged protein amino groups and negatively charged phosphate ions on the resin. Examples of commercially available hydroxyapatite resins include Bio-Gel HT and CHT ceramic resins by Bio-Rad (Hercules, Calif); hydroxyapatite high resolution and hydroxyapatite fast flow by Calbiochem (San Diego, Calif.); HA Ultrogel by Ciphergen (Fremont, Calif.); and hydroxyapatite by Sigma-Aldrich (St. Louis, Mo.). In addition to anion exchange chromatography; rAAV5 virions,: were purified using hydroxyapatite chromatography (FIG. 3B). An example of a preferred hydroxyapatite resin is ceramic hydroxyapatite by Bio-Rad, Hercules, Calif., as this is a, stable, porous form of hydroxyapatite with an improved calcium:phosphate ration, which overcomes low binding capacity due to excess phosphate.

For the purification of rAAV2 virions, heparin-agarose chromatography is preferred See, e.g, U.S. Pat. No. 6,146,874.

A combination of iodixanol step gradient followed by either affinity heparin (for purifying rAAV2), hydroxyapatite, or anion exchange chromatography (for purifying AAV1, 2 and 5) is used to facilitate the high-throughput of several viruses for direct comparison of transduction efficiency and specificity in animal models and cell culture. Scaled-up production of the viruses in tissue culture is facilitated by the use of cell factories, e.g., plastic trays with large culture surface areas (Nunc, Rochester, N.Y.). More importantly, purification of rAAV1, 2 and 5 virions on Q-Sepharose allows the comparison of virions purified using the same method. Furthermore, the cell-factory based protocol results in virion stocks with titers of 1 × 10 ¹² -1 × 10 ¹³ vg/ml purified from 1 × 10 ⁹ cells. These chromatographic methods have the added benefit that they can be readily scaled up to purify virus from 1 × 10 ¹⁰ cells.

By optimizing the transfection protocol and the method of purification, 100-200 infectious units (IU) per cell can routinely be obtained. For a preparation from 1 × 10⁹ cells, for example, the final yield of rAAV is approximately 1-5×10 ¹¹ IU or approximately 1 ×10¹²-1 × 10⁻¹³ vector genomes.

Virions are also purified using chromatography in the absence of density gradient centrifugation. As an example, lysates from infected cells can be directly subjected to chromatography for purification of rAAV virions. For large-scale production methods of rAAV vectors involving chromatography, see Potter et al. (Methods Enzymol., 2002, 346:413-430).

The recombinant virions may be carried out or may include an additional step of affinity-purification of the virions vector using an anti-AAV antibody, preferably an immobilised antibody. The anti-AAV antibody preferably is a monoclonal antibody. A particularly suitable antibody is a single chain camelid antibody or a fragment thereof as e.g. obtainable from camels or llamas (see e.g. Muyldermans, 2001, Biotechnol. 74: 277-302). The antibody for affinity-purification of rAAV preferably is an antibody that specifically binds an epitope on an AAV capsid protein, whereby preferably the epitope is an epitope that is present on capsid protein of more than one AAV serotype. E.g. the antibody may be raised or selected on the basis of specific binding to AAV2 capsid but at the same time also it may also specifically bind to AAV1, AAV3 and AAVS capsids.

### METHODS FOR PREPARING RECOMBINANT SV40 VIRIONS

Also described is a method for preparing a recombinant SV40 virion comprising
(i) contacting a cell with a polynucleotide comprising a replication-defective SV40 genome comprising an expression cassette comprising a sequence encoding IGF-I or a functionally equivalent variant thereof which is operably linked to liver-specific promoter and wherein the cell expresses the SV40 genes which complement the replication defect in said polynucleotide under conditions adequate for entry of said polynucleotide into the cell and
(ii) recovering the recombinant SV40 virion from the cells.

In step (i), a cell is contacted with a polynucleotide comprising a replication-defective SV40 genome comprising an expression cassette comprising a sequence encoding IGF-I or a functionally equivalent variant thereof which is operably linked to liver-specific promoter. Preferably, the SV40 genome lacks the region encoding the large T antigen.

The SV40 genome, which lacks the sequence coding the large T antigen but still contains the sequences encoding the capsid proteins under the control of the late promoter can then be propagated in a conventional cloning vector. A transgene under the control of a liver-specific promoter can be then cloned in the region of the cloning vector previously occupied by the large T antigen. The viral DNA sequence can be excised and purified from the cloning vector and be religated to form a circular DNA. The virions can be produced and amplified by transfecting a packaging cell which express the large T antigen with the religated circular viral vector DNA. A particularly preferred packaging cell is a cell line which constitutively expresses T antigens that can complement the SV40 vector. A suitable cell is the Cos-1 cell line. However, other cell lines can be used after the introduction of the gene for the SV40 large T-antigen.

The liver-specific promoter can be any promoter as defined above. In a part of the disclosure, the liver specific promoter is a hybrid promoter comprising the albumin enhancer region and the promoter of the α1-antitrypsin. In another part of the disclosure, the liver specific promoter is an inducible liver specific promoter.

The invention is hereby explained by the following examples which are to be construed as merely illustrative and not limitative of the scope of the invention.

### EXAMPLES

### EVALUATION OF THE EFFECTS OF A DOUBLE-STRANDED AAV VECTOR ENCODING IGF-I (dsAAVIGF-I) IN LIVER CIRRHOSIS

### MATERIAL AND METHODS

**Double stranded AAV vector construction and production.** The AAV plasmids used in this study contain an expression cassette flanked by two ITRs from the AAV2 and an appropriate stuffer sequence to adjust the size of the AAV genome to the optimal packaging capacity described for AAV. The transgene expression cassette has the following elements: the 5'ITR from AAV2, a liver-specific promoter EalbAATp with regulatory sequences from the albumin enhancer (Kramer et al., 2003, Mol Ther. 7:375-85), the rat IGF-I cDNA, the SV40 polyadenylation, and the 3'ITR from AAV2. In order to generate a double stranded AAV vector the 3'ITR lacked the terminal resolution site, as described by McCarty et al. (Gene Therapy 2003; 10: 2112-2118). This AAV plasmid was named pAAVIGF-I. A similar construct was made with a PBGD promoter, which is ubiquitous and a Luciferase reporter gene (GenBank acc # M15077). This AAV plasmid was named pAAVLuc. Double stranded dsAAV2/1 vectors were produced by calcium phosphate-mediated co-transfection in 293 cells of three different plasmids pAdDeltaF6, p5E18-VD2/8 and the therapeutic (pAAVIGF-I) or reporter gene (pAAVLuc) (Hermens et al, 1999 Hum Gene Ther. 10:1885-91 and Gao et al 2002, Proc Natl Acad Sci USA, 99:11854-9). Briefly, 293 cells were cotransfected with pAdDeltaF6, p5E18-VD2/8 and target vector by calcium phosphate and the virus was harvested by freeze thawing of the cells, 48h after transfection. The virus was purified by ion exchange column chromatography and iodixanol gradient centrifugation followed by filtration and further concentration against phosphate buffered saline (PBS)-5% sucrose. dsAAVIGF-I virus titres in terms of genome copies/ml were determined by Q-PCR performed in triplicate, TaqMan (AppliedBiosystems) analysis using primers pr300fw 5'CCCTGTTTGCTCCTCCGATAA3' (SEQ I D N O :8) pr301rv 5' GTCCGTATTTAAGCAGTGGATCCA 3' (SEQ ID NO:9) amplifying a 95 bp fragment of the hAAT promoter region. Protein composition and purity was determined by sodium dodecyl sulfate-polyacrylamide gel electrophoresis (SDS-PAGE).

**Model of established liver cirrhosis.** Cirrhosis was induced in male Sprague-Dawley rats of (180-200 g), with weekly intragastric administrations of carbon tetrachloride (CCl₄, Riedel-de Haën) for 8 weeks, as described and 400mg/l of phenobarbital in the drinking water (Runyon BA et al., Gastroenterology. 1991;100:489-493.). In brief, the initial CCl₄ dose was 20 µl per rat. Subsequent doses were adjusted based on the change in body weight 48 hours after the last dose (Runyon et al., *supra*). All rats were observed at least twice daily until death. Following this protocol, ascites was apparent in some of the animals. Blood samples were collected from the retro-orbital plexus 3, 5, and 8 weeks after the first administration of CCl₄. Serum transaminases (alanine aminotransferase and aspartate and alkaline phosphatase), albumin and bilirubin, were measured (ABX diagnostics) in a Hitachi autoanalyzer (Roche). The results show that transaminases reached highest levels after 8 weeks of CCl₄ administration.

**Administration of viral vectors.** Four experimental groups of animals were analyzed: healthy rats (n=19), cirrhotic rats injected with saline (Ci) (n=16), treated with 3.4 x 10⁹ viral particles of dsAAVLuc (Ci+Luc) (n=19) or dsAAVIGF-1 (Ci+IGF-I) (n=23). Vector was administered by injection in the hepatic artery. Animals were sacrificed 4 days (healthy n = 5, Ci n = 4, Ci+Luc n = 4, Ci+IGF-I n = 6), 2 weeks (healthy n = 4, Ci n = 3, Ci+Luc n = 5, Ci+IGF-I n = 6), 8 weeks (healthy n = 5, Ci n = 4, Ci+Luc n = 5, Ci+IGF-I n = 5), 16 weeks (healthy n = 5, Ci n = 5, Ci+Luc n = 5, Ci+IGF-I n = 6) or one year (healthy n = 3, saline n = 4, Ci+AAVLuc n = 2, Ci+IGF-I n = 4) after vector administration. Healthy rats were also sacrificed as controls. Animals treated with 10¹¹ viral particles of SVIGF-I and sacrificed 4 days and 8 weeks after the last administrations of CCl₄ were also included as positive controls and are included in the supplementary information. Blood samples were collected before sacrifice and analyzed as indicated above. Liver samples were processed for histology, and purification of RNA and proteins for further analysis.

**Analysis of serum markers and IGF-I in serum.** Serum transaminases (alanine aminotransferase and aspartate and alkaline phosphatase), albumin and bilirubin, were measured (ABX diagnostics) in a Hitachi autoanalyzer (Roche).
IGF-I in serum was quantified with OCTEIA Rat/Mouse IGF-I (IDS) by ELISA.

**Liver histology and immunohistochemistry.** Liver collagen content was assessed and quantified as described (Vera M. et al., Gene Ther. 2007; 14:203-210). Immunohistochemical staining for α-smooth muscle actin (αSMA) was done with antibody 1A4 (M0851, Dako) diluted 1:100 or 1:400, and for Ki-67 with antibody SP6 (RM-9106, NeoMarkers) diluted 1:50.

**Liver proteins and RNA analysis.** Total liver IGF-I (OCTEIA Rat/mouse IGF-I, Vitro) was measured in liver extracts by ELISA.
HGF (Institute of immunology Co, Ldt),
MMP2 (Matrix Metalloproteinase-2 Activity Assay Biotrak System) and MMP9 (Matrix Metalloproteinase-9 Activity Assay Biotrak System) were measured in liver extracts by ELISA.

Total MMP activity was also evaluated in liver extracts with a fluorigenic peptide substrate (Mca-Pro-Leu-Gly-Leu-Dpa-Ala-Arg-NH2; R&D systems) (Knight CG et al.. FEBS Lett. 1992; 296:263-266).
Recombinant human MMP-2 (Calbiochem) was used as positive control.

Total RNA was extracted as described previously (Vera et al., *supra.*). qRT-PCRs were done as described using the primers shown in Table 1 (Vera et al., *supra.*).

| **Gene** | **Forward primers** | **SEQ ID NO:** | **Reverse primers** | **SEQ ID NO:** | **Tm (°C)** | **72 °C (s)** |
|---|---|---|---|---|---|---|
| GADPH | CTTCCACGATGCCAAAGTTG | 10 | GATGGTGAAGGTCGGTGTG | 11 | 60.0 | 30 |
| IGF-I | GTGTCGATAGGGGCTGGGAC | 12 | TTCAGTTCGTGTGTGGACAAGG | 13 | 64.0 | 25 |
| IGF-I receptor | GACAGTGAATGAGGCTGCAA | 54 | TCTCCACCTCTGGCCTTAGA | 55 | 64.0 | 25 |
| TGF-β | CGGCAGCTGTACATTGAC | 14 | TCAGCTGCACTTGCAGGAGC | 15 | 59.0 | 25 |
| Collagen I | CAAGAATGGCGACCGTGGTGA | 16 | GGTGTGACTCGTGCAGCCATC | 17 | 59.0 | 25 |
| Collagen IV | GCAGGTGTGCGGTTTGTGAAG | 18 | AGCTCCCCTGCCTTCAAGGTG | 19 | 62.0 | 35 |
| CTGF | ATCCCCTGCGAGGGACACAAG | 20 | CAACTGCTTTGGAAGGACTCGC | 21 | 70.0 | 25 |
| PDGF | GAAGCCAGTCTTCAAGAAGGCCAC | 22 | AACGGTCACCCGAGTTTGAGGTGT | 23 | 67.0 | 30 |
| VEGF | AAGCCAGCACATAGGAGAGATGAG | 24 | TCACCGCCTTGGCTTGTCACATCT | 25 | 68.0 | 25 |
| αSMA | ACTGGGACGACATGGAAAAG | 26 | CATCTCCAGAGTCCAGCACA | 27 | 64.5 | 15 |
| HGF | AGGGAATCCTCTCGTTCCTTGG | 28 | GAGGCGAGGCGAAACGCAAAC | 29 | 60.0 | 25 |
| HNF4α | CCTTGGACCCAGCCTACA | 30 | GCTTGAGGCTCCGTAGTGT | 31 | 62.0 | 25 |
| MMP-1 | TTGTTGGTGCCCATGAGCTT | 32 | ACTTTGTCGCCAATTCCAGG | 33 | 55.0 | 25 |
| MMP-2 | CTATTCTGTCAGCACTTTGG | 34 | CAGACTTTGGTTCTCCAACTT | 35 | 59.1 | 25 |
| MMP-9 | AAGGATGGTCTACTGGCAC | 36 | AGAGATTCTCACTGGGGC | 37 | 59.7 | 25 |
| MMP-14 | AAAGGGAACAAATACTGGAA | 38 | ATGTAGTTAGGGGGATGGAA | 39 | 59.7 | 25 |
| AR | CTGCTGGTCTTAGGCTCAGG | 40 | CCAGGTTCTCGATGTATCTGC | 41 | 60.0 | 25 |
| WT-1 | GGAACCAGATGAACCTCGGAG | 42 | CGCTTCTCACTGGTTTCAGATGCTG | 43 | 66.0 | 30 |
| TIMP-2 | ATTTATCTACAGGGCCCC | 44 | CAAGAACCATCACTTCTGTTG | 45 | 59 | 25 |
| TNFα | GAGCACAGAAAGCATGATCC | 46 | AGCCCATTTGGGAACTTCTC | 47 | 59 | 25 |
| IL-6 | CAGTTGCCTTCTTGGGACTG | 48 | GTTCATACAATCAGAATTGC | 49 | 62.0 | 25 |
| IGFBP3 | CCTCCGAGTCTAAGCGGGAGAC | 50 | GCATTGCCTCAGCGTGCAGAG | 51 | 61 | 25 |
| PCNA | TTTGAGGCACGCCTGATCC | 52 | GGAGACGTGAGACGAGTCCAT | 53 | 64 | 25 |

**Liver cells isolation.** Liver cells were isolated after perfusion with pronase and liberase (to isolate stellate, kupffer and endothelial cells) or collagenase (to isolate hepatocytes) (Wang SR et al. In Vitro Cell Dev Biol. 1985; 21:526-530). For hepatocyte isolation, liver cells were centrifuged at 500 rpm for 4 minutes at 4 °C. The hepatocyte pellet was washed twice in serum free William's medium and centrifuged at 500 rpm for 2 minutes at 4 °C. When the cell viability was lower than 70 %, viable hepatocytes were purified in an 80% Percoll gradient. To isolate stellate, kupffer and endothelial cells, hepatocytes were first discarded after centrifugation at 50 g for 5 minutes at 4 °C. The supernatant was washed three times in serum free DMEM-F12 by centrifugation at 450 g for 7 minutes at 4 °C. Then, cells were separated by centrifugation in a Nycodenz gradient at 1450g for 20 minutes at 4 °C. Hepatic stellate cells were removed from the upper layer of the gradient and were washed in DMEM and centrifuged at 450 g for 5 minutes at 4 °C. Kupffer and endothelial cells were recovered from the interphase of the Nicodenz gradient and were washed in serum free DMEM.

**Statistical analysis.** Data are expressed as means ± standard deviation. Statistical significance was estimated with Student's *t*-test. A *P*-value of <0.05 was considered significant (*). All statistical analyses were carried out with SPSS v11.0 (SPSS Inc).

### Example 1. Analysis of the model of established liver cirrhosis

Blood samples were collected from the retro-orbital plexus 8, 16, 25 and 33 weeks after the first administration of CCl₄ (Fig. 1A). Serum transaminases (alanine aminotransferase and aspartate and alkaline phosphate), albumin and bilirubin, were measured (ABX diagnostics) in a Hitachi autoanalyzer (Roche). The results show that transaminases reached highest levels after 8 weeks of CCl₄ administration (Fig. 1B). Then, transaminases decreased but levels were higher than healthy animals even 33 weeks after the first administration of CCl₄ (Fig. 1B). The same result was obtained with bilirubin (data not shown). Also, albumin levels decreased after 8 weeks of CCl₄ administration and remained lower than healthy controls 33 weeks after the initiation of the protocol (data not shown).

Animals were sacrificed 16, 21 or 33 weeks after the first administration of CCl₄ (Fig. 1A) Liver samples were fixed in 4% paraformaldehyde, paraffin embedded and stained with hematoxylin-eosin to evaluate liver morphology (data not shown). Liver collagen content was assessed by Sirius red staining and scored by imaging analysis (AnalySIS 3.1, Soft Imaging System) (Vera et al., *supra.*). Liver fibrosis was apparent at 16 weeks and remained 21 and 33 weeks after the initiation of the protocol (Fig. 1C and D). Similar levels were observed in all cases as shown by quantification of Sirius red staining (Fig. 1D). We did not observed significant changes in the size of parenchymal nodules in the different samples.

### Example 2. IGF-I gene transfer to the cirrhotic liver.

To evaluate IGF-I effect in rat cirrhotic livers, cirrhosis was induced with CCl₄ for 8 weeks (Fig. 2). Cirrhotic rats were treated with saline or with recombinant double-stranded adenoassociated (dsAAV) vectors encoding Luciferase (dsAAVLuc) or IGF-I (dsAAVIGF-I) by intra-arterial administration. We chose this route as previous experiments with dsAAVLuc showed good expression levels following intra-arterial injection of the vector. Also, this route following catheterism of the hepatic artery is possibly the most adequate procedure to be used in patients. Treated animals were sacrificed 4 days, 2 weeks, 8 weeks, 16 weeks, and 1 year after virus injection. Healthy rats were also sacrificed as controls.

To assess transgene expression from dsAAVIGF-I in rat liver, we performed qRT-PCR and ELISA of IGF-I in liver and serum samples from all groups. As expected, both mRNA and protein levels of IGF-I were significantly increased in the Ci+IGF-I group and decreased in control cirrhotic livers (Ci and Ci+Luc) as compared to healthy rats (Fig. 3A and B). As has been described, IGF-I was decreased in serum in cirrhotic animals compared to healthy controls 4 days after the last dose of CCl₄ (Fig. 3C). However, IGF-I levels in cirrhotic and healthy animals were similar 2 weeks after the last dose of hepatotoxant. dsAAVIGF-I administration did not lead to an increase of IGF-I in serum at any analyzed time (Fig. 3C). Taken together, these data indicate that dsAAVIGF-I vector was able to transduce the cirrhotic liver and to express IGF-I protein, which was retained into the liver. IGF-IBP3 mRNA levels were evaluated in liver extracts by qRT-PCR (Fig. 3D). The results show that IGF-I is increased in dsAAVIGF-I treated animals compared to controls. The IGF-I expressed is active, as it can activate expression of IGF-IBP3. Thus, increased levels of IGF-I and IGF-IBP3 can still be detected one year after injection of dsAAVIGF-I. However, the levels of these factors are lower than the ones observed at earlier times post vector administration.

### Example 3. IGF-I gene transfer to the cirrhotic liver improves liver function and causes a marked reduction of liver fibrosis.

Cirrhotic rats treated with dsAAVIGF-I showed a significant improvement in biochemical liver tests: serum AST, ALT, ALP and bilirubin were significantly lower than in control cirrhotic rats and similar to healthy controls (Fig. 4A-B). Also, serum albumin was significantly increased in Ci+IGF-I rats, as compared to Ci and Ci+Luc animals, reaching levels similar to healthy controls (Fig. 4C). These alterations are significant from 2 weeks after vector administration.

These findings were accompanied by a marked histological improvement with intense reduction of fibrosis and resolution of cirrhosis in dsAAVIGF-I treated rats (Fig. 5A). Quantification of fibrosis and measurement of collagen I and IV mRNA expression by qRT-PCR, corroborated the reduction of fibrosis observed in dsAAVIGF-I treated animals compared to cirrhotic controls (Fig. 5B-D). The decrease in fibrosis is significant from 2 weeks after vector administration and after 16 weeks of treatment fibrosis reverts resulting in healthy livers. Besides, histopathological analysis of the liver 1 year after vector administration did not revealed liver cirrhosis. This indicates that cirrhosis is regressed even in cirrhotic controls one year after the last dose of hepatotoxant. Sirius red staining of liver sections revealed some fibrotic lesions in cirrhotic controls. Quantification of sirius red staining indicated that cirrhotic controls show more fibrosis than healthy animals or animals treated with dsAAVIGF-I (Fig. 5B). Not surprisingly, the levels of collagen I and IV mRNA are similar in all experimental groups after 1 year (Fig. 5C and D).

In addition, (data not shown) a full necropsy and a complete histopathological analysis was carried in some of the animals 1 year after vector administration. The results show that the animals treated with dsAAVIGF-I have no significant differences with healthy rats or cirrhotic controls.

### Example 4. IGF-I gene expression within the cirrhotic liver induces fibrolysis.

The virtual resolution of cirrhosis observed in animals given IGF-I would require the activation of enzymes capable of removing collagen, such as MMPs. Using a functional assay to measure MMP activity, we observed that in liver extracts from Ci and Ci+Luc groups, MMP activity was reduced as compared to healthy controls (Fig. 6A). In contrast, MMP activity in IGF-I treated animals was significantly higher than in healthy animals (Fig. 6A). MMP activity correlated with a significant increase in the expression of MMP1, 2, 9 and 14 mRNAs and MMP2 and MMP9 protein in the livers that received dsAAVIGF-I (Fig. 6B to G). Activation of MMP expression and activity in dsAAVIGF-I treated animals is detected from 2 weeks after vector administration. At the end of the study, MMP1, MMP2, MMP9 and MMP14 showed similar levels in all groups (Fig. 6B to G). On the contrary, IGF-I treated animals show highest levels of MMPs 2 weeks after vector administration. Then, levels reduce to reach similar levels to healthy and cirrhotic controls at the end of the study.

The inhibitor of MMPs, TIMP-2 also shows similar levels in all groups at the end of the study (Fig. 6E). TIMP-2 levels were constant in healthy animals and decreased with time in all cirrhotic groups. IF-1 treated animals showed a faster decrease than cirrhotic controls, but show similar levels to cirrhotic controls at the end of the study.

### Example 5. IGF-I gene expression within the cirrhotic liver reduces the expression of profibrogenic factors.

Reduced fibrosis could also result from a decrease of activated HSCs. Accordingly, immunohistochemical analysis of liver samples showed that expression of αSMA, a marker of HSC activation, was almost absent in IGF-I treated animals, while it was conspicuous in the septa surrounding nodules in control cirrhotic animals (Ci and Ci+Luc) (Fig. 7A). In line with this observation we found a significant decrease of αSMA mRNA levels in IGF-I treated livers (Fig. 7B), indicating that IGF-I therapy was able to decrease activated HSCs. Surprisingly the decrease in activated HSCs is detected 4 days after vector administration.

After 1 year of vector administration a small increase in αSMA mRNA levels cirrhotic controls can still be detected compared to healthy and IGF-I treated animals.

At this time post-vector administration we also observed reduced TGFβ expression of Ci+IGF-I rats (Fig. 7C-E). Since TGFβ is a powerful activator of HSCs and the most potent accelerator of liver fibrosis, its down-regulation by IGF-I gene therapy might be a key factor underlying the antifibrogenic effect of the treatment. The decrease in TGFβ mRNA and protein can be detected in the liver (Fig. 7C and D) but also in serum (Fig. 7E). In addition to TGFβ, other molecules that promote HSC growth and contribute to liver fibrosis such as connective tissue growth factor (CTGF), platelet-derived growth factor (PDGF) vascular endothelium growth factor (VEGF) and amphiregulin (AR) were up-regulated in control cirrhotic livers but markedly suppressed in those treated with dsAAVIGF-I as compared to control cirrhotic rats (Fig. 7F to I). Other markers of damage and inflammation such as IL6 and TNFα are also decreased in dsAAVIGF-I as compared to control cirrhotic rats (Fig. 7J and K). Many of these markers are significantly decreased at 4 days after vector administration. The levels of these factors were similar in healthy animals at all times evaluated. In cirrhotic animals, the levels decreased with time. IGF-I treated animals showed a faster decrease than cirrhotic controls, but had similar levels to cirrhotic controls at the end of the study.

### Example 6. IGF-I gene expression within the cirrhotic liver increases the expression of antifibrogenic and hepatoprotective factors and does not stimulate proliferation.

Together with these changes we found a significant increase in the expression of hepatocyte growth factor (HGF) in the liver of Ci+IGF-I rats as compared to control animals (Fig 8A). Since HGF displays potent antifibrogenic activities, upregulation of this molecule may contribute to the resolution of liver cirrhosis observed in IGF-I treated rats. HGF also behaves as an hepatoprotective factor and, together with HNF4α, as a marker for hepatocyte differentiation. HNF4a is also upregulated in dsAAVIGF-I treated animals at 4 days after vector administration (Fig. 8B). Conversely, WT-1, a marker of hepatocyte dedifferentiation, decreases in treated animals (Fig. 8C). Finally, in spite of IGF-I being a growth factor, cell proliferation measured by PCNA expression and quantification of Ki67 staining, also decreases in dsAAVIGF-I treated animals (Fig. 9A and B). At the end of the study, HNF4a, WT-1 and PCNA levels were similar in all experimental groups. However, the HGF increase in the presence of IGF-I is still detected one year after IGF-I vector administration.

### Example 7. Comparison between dsAAVIGF-I and SVIGF-I

The effect of SVIGF-I in liver cirrhosis has been studied independently (Vera et al., *supra.*) or in parallel with dsAAVIGF-I. Two independent studies were carried out with SVIGF-I as the only source of IGF-I, that showed reproducible results. These results are very similar to the ones obtained when SVIGF-I was analyzed in parallel with dsAAVIGF-I. In this last case, SVIGF-I was only analyzed 4days and 8 weeks after vector administration. In general, both dsAAVIGF-I and SVIGF-I can revert liver cirrhosis. Using either of the vectors, liver functionality is restored leading to similar levels of transaminases, bilirubin and albumin (data not shown). Transaminases and bilirubin are slightly higher with SVIGF-I than with dsAAVIGF-I but differences are not significant (data not shown). However, liver fibrosis is significantly higher in SVIGF-I treated animals than in rats treated with dsAAVIGF-I 8 weeks after vector administration (Fig. 10). Besides, fibrosis disappears 16 weeks after dsAAVIGF-I vector administration. This reduction has never been observed in SVIGF-I treated animals even when animals were analyzed 25 weeks after vector administration (Fig. 10B). Similar differences are observed by measuring collagen I and IV mRNA (Sup. Fig. 10C and 10D).

Increased efficiency of dsAAVIGF-I compared to SVIGF-I is still a mystery. Expression levels of IGF-I are increased in both SVIGF-I and dsAAVIGF-I treated animals (Fig. 11) and the IGF-I expressed from SV40 is active, as it can activate expression of IGF-IBP3 (data not shown). However, dsAAVIGF-I administration tends to express higher levels of IGF-I than SVIGF-I injection at different time points after vector administration (Fig. 11). These differences in IGF-I expression do not result in different MMP activity in dsAAVIGF-I and SVIGF-I treated animals at 4 days or 8 weeks after vector administration (data not shown). The amount of MMP2 and MMP9 measured by ELISA or the levels of MMP1, MMP2, MMP9 and MMP14 mRNA measured by qRT-PCR are similar in liver extracts isolated from animals treated with dsAAVIGF-I or SVIGF-I (data not shown). However, dsAAVIGF-I treatment shows decreased αSMA protein and mRNA levels 4 days after vector administration, while SVIGF-I shows levels similar to cirrhotic controls (Fig. 12). The decrease in activated HSCs observed in dsAAVIGF-I animals correlates with a higher decrease of profibrogenic factors in these animals compared to SVIGF-I administered rats. Thus, TGFβ, CTGF and VEGF were decreased in dsAAVIGF-I treated animals compared to SVIGF-I treated rats and cirrhotic controls 4 days after vector administration (Fig. 13). Also hepatoprotective factors such as HGF and HNF4α were increased 4 days after dsAAVIGF-I delivery compared to controls or SVIGF-I administration (Fig. 14). No significant differences in proliferation were observed between dsAAVIGF-I and SVIGF-I treated animals as detected by quantification of Ki67 staining (data not shown).

### Example 8. Toxicological assessment of dsAAVIGF-I treated animals after one year of treatment.

Assessment was performed by comparing cirrhotic animals treated with dsAAVIGF-I or dsAAVLuc, or saline, with healthy animals.

A necropsy was performed in animals sacrificed 1 year after virus injection. Blood was collected to evaluate several parameters. Kidney, lung, small intestine, liver, brain, cerebellum, skeletal muscle, testis, spleen, stomach, bone marrow, lymph nodes, thymus and heart were evaluated by histopathological analysis.

Relative weight of spleen, heart, testis, kidney, and liver did not show significant differences among the experimental groups studied.

Urine was collected from all animals in metabolic cages 24 hours before sacrifice. Several parameters were evaluated, such as density, pH, color and turbidity, and molecules including keton bodies, glucose, bilirubin, proteins and urobilinogen. Urine presence of erythrocytes and leukocytes was also quantified. The results show non-significant differences in all these parameters among all experimental groups, even in some quantifications the values obtained show high variability.

Serum was used to quantify red blood cells, platelets, white blood cells, neutrophiles, lymphocytes, monocytes, eosynophiles and basophiles. Monocytes, eosynophiles and basophils showed similar values among all experimental groups. The same was true for red blood cells, platelets, white blood cells and lymphocytes, even if in these cases there was high variability for some of the groups. The amount of neutrophiles was similar in healthy and cirrhotic animals treated with dsAAVLuc, but lower than in animals treated with dsAAVIGF-I.

Several characteristics of hematopoietic cells were analyzed. These include haemoglobin (HGB), hematocrit (HCT), mean corpuscular volume, which is calculated by dividing the total number of packed red blood cells by the total number of red blood cells (MCV), mean corpuscular hemoglobin (MCH), which is a calculation of the average amount of oxygen-carrying hemoglobin inside a red blood cell and mean cell hemoglobin concentration (MCHC). These parameters resulted in values of low variability, with non-significant differences among all experimental groups.

No significant differences among all experimental groups were observed for transaminases, bilirubin and albumin after 1 year of the vector administration. In addition, several parameters were analyzed in serum after 1 year of the administration of the vector (data not shown). Most of these parameters, such as, creatin fosfokinase, triglycerides, sodium, chlorum, creatinin, potassium, calcium, magnesium, and prothrombin time showed values of low variability, very similar among all experimental groups and whose statistical analysis showed non-significant differences. No significant differences among all experimental groups were also observed for transaminases and LDH, but in these cases variability was high. Similar results with non-significant differences were observed for total proteins, glucose, phosphorus and TCA, but these parameters were slightly increased (total proteins and TCA) or decreased (glucose and phosphorus) in dsAAVIGF-I treated animals.

As above indicated, histopathological analysis of the liver did not revealed liver cirrhosis. This indicates that cirrhosis is regressed even in cirrhotic controls one year after the last dose of hepatotoxant. Sirius red staining of liver sections revealed some fibrotic lesions in cirrhotic controls. Quantification of sirius red staining indicated that cirrhotic controls showed more fibrosis than healthy animals or animals treated with dsAAVIGF-I. However, fibrosis percentage in fibrotic controls was 15% at earlier time points and has decreased to approximately 5% one year after the last dose of hepatotoxant and treatment. Not surprisingly, the levels of collagen I and IV mRNA are similar in all experimental groups.

Kidney, lung, small intestine, liver, brain, cerebellum, skeletal muscle, testis, spleen, stomach, bone marrow, lymph nodes, thymus and heart were evaluated by histopathological analysis. Testis, thymus, brain and cerebellum, bone marrow, lymph nodes and stomach did not show significant histopathological changes. Some cirrhotic controls showed alterations in heart, muscle and spleen which were not observed in healthy or dsAAVIGF-I treated animals. All alterations observed in dsAAVIGF-I treated animals were also observed in the control animals. The only exceptions were observed in kidney, lung, liver and small intestine. Half or less than half of the animals show bleeding in lung bronchi, light parenchymal inflammation and small areas of necrosis in the liver or lymphoid depletion in the peyer patches of small intestine. More alterations were observed in the kidneys of dsAAVIGF-I treated animals. Half or less than half of the animals showed increased gromeruli size, occasional acidophilus content in Bowman's capsule, nephritis or proliferation surrounding regenerating glomeruli. All animals showed some tubular necrosis that could probably account for the increased GGT levels in serum. It is difficult to correlate exogenous liver IGF-I expression with the observed alterations. More animals should be evaluated to address whether this result has statistical significance and relevance.

In conclusion, cirrhotic animals expressing exogenous liver IGF-I for 1 year are similar to healthy and cirrhotic controls. Most serum and urine parameters analyzed and histopathological studies show no reproducible significant differences between AAVIGF-I treated animals and controls. However, some parameters such as serum levels of GGT and histopathological changes in kidney should be re-evaluated with larger groups in the toxicological studies that precede clinical trials.

Interestingly, IGF-I expression can still be detected after one year of dsAAVIGF-I administration. IGF-I expression correlates with increased levels of IGFIBP3 and HGF, as observed in healthy animals). However, at this time point changes in metalloproteinases, inflammatory and profibrogenic factors are not detected.

### EVALUATION OF THE EFFECTS OF A SINGLE-STRANDED AAV VECTOR ENCODING IGF-I (ssAAVIGF-I) IN LIVER CIRRHOSIS

### MATERIAL AND METHODS

### Single stranded AAV vector construction and production.

The genome of AAV2 was used and the virus was pseudotyped with AAV1. The ssAAV vectors are identical in gene structure to dsAAV vectors (described above) with the exception of the luciferase cassette, which has an a1AT promoter identical to that of the IGF-I vector. Moreover, 5' and 3' ITR flanking regions of the expression cassette are AAV2 wild type.

***Construction of plasmid pVD204*** For the generation of pVD204, the IGF1 gene sequence was PCR cloned using the Accuprime™ pfx DNA Polymerase kit (Stratagene; ref: 12344-024). The primers used for amplification of the gene sequence were forward primer 373 (5' - GGTA CCAT GTC G TC T TCACATCTC - 3') (SEQ ID NO:56) and reverse primer 374 (5' - GCGGCCGCGAATGTTTACTT - 3') (SEQ ID NO:57). Primer 373 contains a *Kpn*I site on the 5' side and primer 374 contains a *Not*I site on the 5' side. Template plasmid scAAV-IGF1 was used (externally obtained) for the amplification. The PCR product was visualized on a 1% Agarose-gel and then directly used to ligate the PCR product into the pCR^{®} II-Blunt-TOPO^{®} vector from the Zero Blunt^{®} TOPO^{®} PCR Cloning Kit (Invitrogen). After the ligation reaction, the recombinant TOPO vector was then chemically transformed in Stabl III cells and grown o/n on Kanamycin LB-agar plates. The next day, 6 single colonies were picked and directly used for a colony PCR to screen for strains carrying the recombinant TOPO-IGF vector. For this PCR the Multiplex PCR kit (QIAGEN) and primers 373 and 374 were used to verify the presence of the PCR product within the TOPO vector. Then 1 positive colony was selected and grown o/n in 250 ml LB medium containing Kanamycin. After performing a maxiprep, 20 µg of the isolated TOPO-IGF plasmid was cut with 100 Units of *Not*I and 20 µg pVD158 was cut with 100 Units of *Nhe*I. After heat inactivation of 20 min at 80 °C, both linearized constructs were Blunt ended by adding 4µL Klenow Large Fragment (Westburg) was added to both reactions and were incubated 15 min. at 25 °C. After adding 20 µL of EDTA (100µM) and 20 min. heat inactivation at 75 °C, a DNA isolation of both reactions was performed using a Qiaquick PCR-purification kit, yielding 11,4 µg of linear TOPO-IGF and 7.2 µg of linear pVD157. Then the second digestion reaction was performed by cutting both 11.4 µg TOPO-IGF and 7.2 µg pVD157 with 100U *Kpn*I. Fragment separation was performed by agarose gel electrophoresis (1.0%) and the 600 bp insert fragment from the TOPO-IGF vector and the 10900 bp pVD157 vector fragment were isolated from the gel using a QiaQuick gel extraction kit. Ligation of vector and insert DNA was performed by using Quick-ligation (NEB) based on a 3:1 Vector/Insert Molar ratio. After a 15 minutes incubation time at room temperature, transformation into chemical competent SURE II cells (Stratagene) was performed as described in manufacturer's manual. 2 positive colonies were selected and were screened by colony PCR. The positive clone was then amplified and maxi-prepped and stored at -20 °C.

***Generation of baculovirus stocks.*** Baculovirus stocks are produced using a baculovirus expression sytem: Targeting plasmid pVD204 is co-transfected linearized baculovirus genomic DNA using Cellfectine into *S. frugiperda* Sf9 insect cells, where the sequence of interest will be transferred into the baculovirus genome via homologous recombination. The produced recombinant baculoviruses were identified and purified by using a standard plaque assay procedure. A well isolated virus plaque was picked and used to create viral stocks by infecting increasingly larger cultures of insect cells, reaching a P2 or higher, which were stored at -170 °C. These stocks were screened for stability of the inserted DNA, and presence of wildtype baculovirus impurities. For rAAV production, three different baculovirus stocks are used: 1) Baculoviruses harbouring an AAV2-Rep78/52 expression cassette; Bac.Rep 2) baculoviruses harbouring an AAV-Cap serotype 1 expression cassette; Bac.Cap and 3) baculoviruses presenting the vector genome flanked by AAV2 ITRs (Bac.VD204) to be packaged into AAV particle.

***Baculovirus amplifications for 1L rAAV production.*** Frozen baculovirus stock is thawed at 37°C and are then added to a 1.7 x 10⁶ Sf9 cell culture (3 µL per mL of cell culture). After 72 ± 5 hours the infected suspension is harvested by spinning down 15min at 1900 G. The supernatant is isolated and stored at 4 °C in the dark until use. For a 1.0 L rAAV production three baculovirus amplifications were prepared and equal amount of Bac.Rep P5; Bac.Cap P5 and Bac.VD204 P3 were administered to the production cells.

***rAAV production.*** Two shakers each containing 400 mL of Sf9 culture at 1.0 x 10⁶ cells are started 20 hours before infection. At the day of infection the cell density is monitored to be 2.0 x 10⁶ cells/mL and the viability is above 98%. The harvested baculoviruses (Bac.Rep, Bac.Cap (AAV1 viral proteins) and Bac.VD204 (AAV-IGF1 expression cassette)) were added to the shaker culture. After 72 ± 5 hours the rAAV is harvested by adding 45 mL of (10X) Tris-Lysis buffer in each shaker flask. After 1 hour of incubation at 28 °C, 4000 units of benzonase is added to each shaker and incubated 1 hour at 37°C in a shaker incubator at 128 rpm.

***rAAV purification:*** the crude lysed bulk was centrifuged at 3000g and the supernatant was 0.45 µm filtered using a millipak 60 filter (Millipore). Affinity chromatography was performed using an AKTA-Explorer chromatography system equipped with a XK-16 column packed with a 5mL bed volume of AVB Sepharose High performance affinity medium (All from GE healthcare, Piscataway, NJ. Following processing the elution fraction (~ 50 ml) was collected in a container containing 25 mL [0.1] Tris-HCL (pH 7.5). Subsequently, buffer exchange was performed using a crossflow diafiltration module. Eluate was diafiltrated and 0.22 µM filtered. The product was stored frozen at-80°C.

**Model of established liver cirrhosis.** Cirrhosis was induced in male Sprague-Dawley rats with weekly intragastric administrations of CCl₄ for 8 weeks as described for dsAAVIGF-I study above.

**Administration of viral vectors.** Healthy animals were untreated (Healthy), or used to induce liver cirrhosis. A group of healthy rats treated with saline (Healthy), and a group of cirrhotic untreated rats (Ci) were also included as controls.
One week after the end of cirrhosis induction, cirrhotic animals were treated with
- saline (Ci);
- a recombinant single stranded adenoassociated viral vector (ssAAV) encoding Luciferase (ssAAV-Luc), at a dose of 9.7 x 10⁹ vp/rat (very low dose), 4.8 x 10¹⁰ vp/rat (low dose), 2.4 x 10¹¹ vp/rat (medium dose), or alternatively 1.2 x 10¹² vp/rat (high dose);
- a recombinant ssAAV encoding IGF-I (ssAAVIGF-I), also at doses of 9.7 x 10⁹ (very low dose), 4.8 x 10¹⁰ (low dose), 2.4 x 10¹¹ (medium dose), or alternatively 1.2 x 10¹² vp/rat (high dose);
- a double stranded adenoassociated viral vector (dsAAV) encoding Luciferase (dsAAVLuc), at a dose of 3.4 x 10⁹ vp/rat; or
- a dsAAV encoding IGF-I (dsAAVIGF-I), at a dose of 3.4 x 10⁹ vp/rat.

The intra-arterial administration route was chosen for the treatment. Previous experiments with dsAAVLuc showed good expression levels when intra-arterial injection was performed. Probably administration by catheterism of the hepatic artery is also the most adequate procedure to be used in patients.

Blood samples for biochemical analysis of serum proteins and factors were collected at 4 days, 2 weeks, 3 weeks, 4 weeks, and 8 weeks, 12 weeks, and 1 year post-infection (treatment).
Some animals (n = 4) treated with dsAAVIGF-I or the medium dose of ssAAVLuc were evaluated for 2 months and then sacrificed to isolate different populations of liver cells where luciferase expression was measured.
At different times post-infection some animals were selected, sacrificed and hepatic samples were harvested for evaluation:
- 4 days (Healthy n = 4, saline n = 4, dsAAVLuc n = 4, dsAAVIGF-I n = 5, ssAAVLuc, high dose n = 4, medium dose n = 4, low dose n = 4 and very low dose n = 4 and ssAAVIGF-1, high dose n = 6, medium dose n = 5, low dose n = 4 and very low dose n = 5);
- 8 weeks (Healthy n = 5, saline n = 4, dsAAVLuc n = 3, dsAAVIGF-I n = 6, ssAAVLuc, high dose n = 5, medium dose = 4, low dose n = 6 and very low dose n = 6 and ssAAVIGF-I, high dose n = 6, medium dose n = 4, low dose n = 6 and very low dose n = 6);
- 16 weeks (Healthy n = 6, saline n = 6, ssAAVLuc low dose n = 6, ssAAVIGF-I low dose n = 6); and
- 1 year (Healthy n = 4, saline n = 4, ssAAVLuc high dose n = 4, ssAAVIGF-I high dose n = 6) after vector administration.

All procedural and analytical methods were performed as explained for the evaluation of the effects of dsAAVIGF-I.

### Example 9. IGF-I gene transfer to the cirrhotic liver.

IGF-I mRNA was evaluated in liver extracts by qRT-PCR 4 days, 8 weeks after vector inoculation (Fig. 15A). For the treatment with lower dose of ssAAVLuc and ssAAVIGF-I, IGF-I mRNA was further evaluated 16 weeks after vector administration (Fig.15 C). Total liver IGF-1 protein was evaluated by ELISA 4 days and 8 weeks after vector administration (Fig. 15 B).

The results showed that IGF-I mRNA was reduced in cirrhotic animals compared to healthy controls. IGF-I levels were increased in IGF-I treated animals (ssAAVIGF-I or dsAAVIGF-I) compared to healthy and cirrhotic controls. Increased levels of IGF-I were already visible at 4 days post administration of IGF-I expressing vectors. Then, IGF-I protein levels increased at 8 weeks post-administration of AAVIGF-I and remained constant until the end of the study. Also, higher levels of IGF-I were observed when higher doses of ssAAVIGF-I were used.

IGF-I was evaluated in serum 4 days, 2 weeks and 8 weeks after vector administration. The results show that 4 days after treatment total levels of IGF-I are lower in all cirrhotic groups than in healthy animals (Fig. 15D). However, free IGF-I protein is increased in the serum of animals injected with the high and medium dose of ssAAVIGF-I. At 2 and 8 weeks post vector administration both total and free (data not shown) IGF-I are similar in all groups. It would be possible that the increased serum free IGF-I in the animals treated with the highest doses of ssAAVIGF-I turns into total IGF-I at later time points by inducing increased expression of IGF-I binding proteins.

In addition, the IGF-I expressed from the vectors is active, as it can activate expression of IGF-IBP3 and IGF-IR, which are increased in IGF-I expressing animals injected with the vectors for 4 days, 8 weeks and 16 weeks (Fig. 15F-H).

### Example 10. IGF-I gene transfer to the cirrhotic liver improves liver function and causes a marked reduction of liver fibrosis.

Transaminases at 4 days after vector inoculation were similar in all cirrhotic groups and higher than in healthy animals (Fig. 16A). However, animals treated with ssAAVIGF-I or dsAAVIGF-I vectors showed lower transaminase levels than cirrhotic controls at 2 weeks after vector administration. At latter time points transaminase levels decreased further in IGF-I treated animals and 8 weeks after vector administration the levels of transaminases in animals treated with ssAAVIGF-I or dsAAVIGF-I vectors were similar to healthy animals (Fig. 16B). The same was observed in animals treated with the low dose of ssAAVIGF-I 12 weeks after vector administration (Fig. 16C). Interestingly, similar effects were observed in animals treated with dsAAVIGF-I and those treated with ssAAVIGF-I at the low dose. However, the effect was lower in animals treated with the highest dose of ss-AAV1-IGF-I (Fig. 16D).

Bilirubin was similar in all cirrhotic animals and higher than in healthy controls at day 4 after vector inoculation (Fig. 17A). However, animals treated with both dsAAVIGF-I or ssAAVIGF-I showed lower bilirubin levels than cirrhotic controls 2 weeks after vector administration. At latter time points bilirubin levels decrease further in AAVIGF-I (both single stranded and double stranded) treated animals, and 8 weeks after vector administration bilirubin levels in animals treated with AAVIGF-I were similar to healthy animals (Fig. 17B). The same was observed in animals treated with the low dose of ssAAVIGF-I 12 weeks after vector administration (Fig. 17C). Similar results were found 1 year after administration in animals treated with the high dose of ssAAVIGF-I and whose statistical analysis showed non-significant differences. Interestingly, similar effects were observed in animals treated with dsAAV1IGF-I or low doses of ssAAV1IGF-I. However, the effect was lower in animals treated with the high dose of ssAAVIGF-I (Fig. 17D).

Albumin was similar in all cirrhotic animals and lower than in healthy controls at day 4 and 2 weeks after vector inoculation (Fig. 18A,B). However, animals treated with AAVIGF-I (single stranded or double stranded) showed increased albumin levels compared to cirrhotic controls 8 weeks after vector administration (Fig. 18B). The same was observed in animals treated with the low dose of ssAAVIGF-I 12 weeks after vector administration (Fig. 18C). Similar results were found 1 year after administration in animals treated with ssAAVIGF-I at the high dose and whose statistical analysis showed non-significant differences (Fig 18D).

Liver samples were stained with Sirius Red and liver fibrosis was quantified (Fig. 19A). The results showed that cirrhotic livers are fibrotic and that fibrosis decreases in animals treated with AAVIGF-I (single stranded or double stranded). Surprisingly, the high dose of AAVIGF-I is less efficient than the rest. Similar results were observed when collagen I and IV mRNA expression levels were quantified by qRT-PCR (Fig. 19B, C). After 16 weeks of treatment with ssAAVIGF-I at the low dose, fibrosis and collagen mRNA expression levels were identical to those in healthy animals indicating a total reversion of liver cirrhosis (Fig. 19D-F). Collagen II mRNA was not detected and collagen III mRNA levels did not show differences among groups (data not shown). This was expected as they do not accumulate in liver fibrosis.

### Example 11. IGF-I gene expression within the cirrhotic liver induces fibrolysis.

Reduced fibrosis with AAVIGF-I vector could result from degradation of preexisting matrix deposition. MMPs can degrade extracellular collagen and therefore we evaluated MMP expression in the livers of all experimental groups.

MMP1, MMP2, MMP9 and MMP14 mRNA expression levels (Fig. 20A-D, and I-M) and MMP2 and MMP9 protein levels (Fig. 20F, G) were determined by qRT-PCR and ELISA, respectively, 8 weeks and 16 weeks after vector inoculation.
The results showed that the expression of all these MMPs is decreased in cirrhotic animals compared to healthy controls. However, the levels of these factors are dramatically increased compared to both cirrhotic and healthy animals in rats treated with an AAVIGF-I vector (single stranded or double stranded). This increase was not detected at 4 days post-treatment but at 8 or 16 weeks post AAVIGF-I vector administration. Even if MMP levels in AAVIGF-I treated animals decrease with time, they are still higher than those observed in healthy animals at 16 weeks post AAVIGF-I vector administration.

The reverse pattern was observed for TIMP-2 expression (Fig 20E and M). TIMP proteins are inhibitors of MMP activity. Therefore it is essential that TIMP levels are reduced for a high MMP activity. TIMP-2 mRNA expression was increased in cirrhotic animals compared to healthy controls. Levels of TIMP-2 were reduced compared to cirrhotic controls in rats treated with AAVIGF-I vectors. This decrease was detected at 8 weeks post- AAVIGF-I vector administration and TIMP-2 levels decreased further with time, but after 16 weeks of treatment, they were still over the levels observed in healthy animals. The balance of increased MMPs and decreased TIMPs correlates with increased MMP activity. Surprisingly, the highest dose of AAVIGF-I induces less MMP expression and activity than the rest.

### Example 12. IGF-I gene expression within the cirrhotic liver reduces the expression of profibrogenic factors.

Reduced fibrosis could also result from a decrease in profibrogenic cells. Activated hepatic stellate cells (HSCs), marked by smooth muscle actin (αSMA), are the major producers of extracellular collagen. Therefore, we evaluated the amount of activated HSCs by immunohistochemistry with antibody against αSMA (Fig. 21 A) or by quantification of αSMA mRNA (Fig. 21B, C).

The results showed high levels of αSMA expressing cells in cirrhotic animals. 8 weeks after administration of AAVIGF-I vector (single- or double-stranded), there was a marked decrease of αSMA mRNA expression level and αSMA expressing cells. 8 weeks later, αSMA mRNA expression decreased in cirrhotic animals, but was higher than healthy controls. However, the levels of αSMA mRNA in animals treated with ssAAVIGF-I were similar to the levels observed in healthy animals. Reduction of αSMA in animals treated with the high dose of ssAAVIGF-I was less dramatic.

Activation, proliferation, migration and collagen synthesis from HSCs is mediated by profibrogenic factors such as TGFβ, VEGF, PDGF, amphiregulin (AR) or CTGF. This response is favoured in an inflammatory environment labelled by IL-6 and TNFα expression. Therefore, liver expression of these factors was evaluated 8 weeks and 16 weeks after vector inoculation (Fig. 22). These factors were much increased in all cirrhotic controls compared to healthy animals and tended to decrease with time. However, all of these factors decreased drastically in AAVIGF-I treated cirrhotic animals, reaching levels which were non-significantly different than those observed in healthy animals. The only exception was IL6, which was similar in all animals. The reduction of profibrogenic and inflammatory factors was less apparent when the high dose of ssAAVIGF-I was used.

### Example 13. IGF-I gene expression within the cirrhotic liver increases the expression of antifibrogenic and hepatoprotective factors and does not stimulate proliferation.

Improved liver function with AAVIGF-I treatment could result from activation of hepatoprotective factors such as HGF or HNF4α. Therefore, we first measured mRNA and protein expression of HGF (Fig. 23).

The levels of HGF were reduced in cirrhotic controls compared to healthy rats. However, animals treated with AAVIGF-I (single- or double-stranded) showed levels of HGF similar to healthy rats 4 days after the administration of this vector. HGF levels in these animals were higher at later time points and much increased compared to the levels observed in healthy controls. The increase of HGF was less apparent when the high dose of ssAAVIGF-I was used.

The results observed for HGF were very similar to those observed for HNF4α mRNA (Fig. 24A-C). This is very interesting as HNF4a is a maturation factor. In fact, IGF-I is a growth factor and therefore, could activate proliferation of cells with IGF-I receptor. Besides IGF-I activates HGF, which is also a growth factor. Even if hepatocytes do not express IGF-I receptor in healthy livers, we wanted to assay proliferation and differentiation of liver cells upon AAVIGF-I treatment. We used as a liver differentiation marker WT-1, which is expressed in foetal livers and not in adult tissue. WT-1 mRNA levels were dramatically increased in cirrhotic animals compared to healthy rats (Fig. 24D, E). Interestingly, rats treated with AAVIGF-I showed mRNA levels of WT-1 similar to those of healthy animals. These effects were less apparent when the high dose of ssAAVIGF-I was used.

Proliferation was assayed by quantification of PCNA mRNA expression levels and by staining with the proliferation marker Ki67 (Fig.25). After staining, positive cells were counted in all experimental groups and the results were plotted in graphs that measure proliferation of liver cells. Proliferation levels were highest in cirrhotic controls compared to healthy controls or with animals treated with AAVIGF-I. No differences were observed in Ki67 levels between cirrhotic and AAVIGF-I treated animals 4 days after vector administration. However, animals expressing IGF-I showed lower levels of Ki67 at 8 weeks post-vector administration (Fig. 25A), and lower levels of PCNA mRNA at 4 days, 8 weeks or 16 weeks post-vector administration (Fig. 25B-D). The levels of these proliferation factors in IGF-I treated animals were similar to those observed in healthy controls. The only exception was observed in animals treated with the highest dose of AAVIGF-I, which showed proliferation levels higher than healthy animals but lower than cirrhotic controls.

### Example 14. Toxicological assessment in ssAAVIGF-I: treated animals (high dose) after one year of treatment.

Assessment was performed by comparing cirrhotic animals treated with the high dose of ssAAVIGF-I or ssAAVLuc, or saline, with healthy animals.

A necropsy was performed in animals sacrificed 1 year after virus injection. Blood was collected to evaluate several parameters. Kidney, lung, small intestine, liver, brain, cerebellum, skeletal muscle, testis, spleen, stomach, bone marrow, lymph nodes, thymus and heart were evaluated by histopathological analysis.

Relative weight of spleen, heart, testis, kidney, and liver did not show significant differences among the experimental groups studied.

Serum was used to quantify red blood cells, platelets, white blood cells, neutrophiles, lymphocytes, monocytes, eosynophiles and basophiles. All cells showed similar values among all experimental groups.

Several characteristics of hematopoietic cells were analyzed. These include haemoglobin (HGB), hematocrit (HCT), mean corpuscular volume, which is calculated by dividing the total number of packed red blood cells by the total number of red blood cells (MCV), mean corpuscular hemoglobin (MCH), which is a calculation of the average amount of oxygen-carrying hemoglobin inside a red blood cell and mean cell hemoglobin concentration (MCHC). These parameters resulted in values of low variability, with non-significant differences among all experimental groups.

Several parameters were analyzed in serum. These include transaminases (AST, ALT and ALP) (Fig. 16D), bilirubin (Fig. 17D), albumin (Fig. 18D), gamma glutamyl transpeptidase (GGT), creatinin, potassium, calcium, phosphorus, magnesium, sodium, and chlorum. These parameters showed similar values among all experimental groups and whose statistical analysis showed non-significant differences. Measurements in some cases such as bilirubin, albumin or GGT showed high variability.

Coagulation was evaluated by measurement of prothrombin time (PT), activated partial thromboplastin time (APTT) and fibrinogen. These measurements showed high variability, but similar means among all experimental groups. Statistical analysis showed non-significant differences.

Liver fibrosis was evaluated by quantification of sirius red staining of liver sections (Fig. 19D). The result together with the histopathological analysis of the liver did not revealed liver cirrhosis in any of the experimental groups. This indicates that cirrhosis is regressed even in cirrhotic controls one year after the last dose of hepatotoxant.

Kidney, lung, small intestine, liver, brain, cerebellum, skeletal muscle, testis, spleen, stomach, bone marrow, lymph nodes, thymus and heart were evaluated by histopathological analysis. Brain, cerebellum, skeletal muscle, testis, stomach, bone marrow, lymph nodes, and thymus did not show significant histopathological changes. Some cirrhotic controls show alterations in lung, small intestine, liver and spleen which are not observed in healthy or ssAAVIGF-I treated animals. Interestingly, these include two tumors in control cirrhotic animals, one in liver and one in lung. All alterations observed in ssAAVIGF-I treated animals are also observed in the control animals. The only exception is a mild enteritis observed in 2 out of the 6 animals treated with ssAAVIGF-I.

In conclusion, cirrhotic animals which expressed exogenous liver IGF-I by treatment with ssAAVIGF-I) are similar to healthy and cirrhotic controls after 1 year post-treatment. All serum parameters analyzed and histopathological studies show no reproducible significant differences between ssAAVIGF-I treated animals and controls.

## Claims

1. A parvoviral vector comprising a nucleotide sequence encoding insulin-like growth factor I IGF-I or a functionally equivalent variant thereof having the biological activity of IGF-I which is operably linked to a liver-specific promoter comprising the albumin gene enhancer region and the alphal-antitrypsin promoter.

2. A parvoviral vector as defined in claim 1 wherein the parvoviral vector is an AAV.

3. A parvoviral vector as defined in claim 2 wherein the AAV vector is an AAV1, AAV2, AAV5 or AAV8.

4. A parvoviral vector as defined in claims 2 or 3 wherein the AAV vector is a single-stranded AAV or a double-stranded AAV.

5. A parvoviral vector as defined in claim 4 wherein the AAV vector is a double-stranded AAV.

6. A parvoviral vector as defined in any of claims 1 to 5 wherein IGF-I is a human IGF-I.

7. A virion comprising a parvoviral vector as defined in any of claims 1 to 6.

8. A virion according to claim 7 for use as a medicament.

9. A virion as defined in claim 7 for use in a method for the treatment and/or prevention of hepatic cirrhosis or hepatic fibrosis.

10. An *in vitro* method for preparing a recombinant AAV virion comprising the steps of
(i) contacting a cell with
(a) a first nucleic acid sequence comprising
i. an expression cassette comprising a sequence encoding IGF-I or a functionally equivalent variant thereof having the biological activity of IGF-I which is operably linked to a liver-specific promoter comprising the albumin gene enhancer region and the alphal-antitrypsin promoter and
ii. an AAV 5'-ITR and a 3'-ITR flanking the expression cassette defined in (i),
(b) a second nucleic acid sequence encoding an AAV rep protein,
(c) a third nucleic acid sequence encoding an AAV cap protein and, optionally,
(d) a fourth nucleic acid sequence encoding viral and/or cellular functions upon which AAV is dependent for replication,
under conditions adequate for entry of the three components in the cell and
(ii) recovering the recombinant AAV virion from the cells.

11. A method as defined in claim 10 wherein the cell used in the step (i) is an insect cell and wherein the first, second and third nucleic acid sequences are comprised in a baculoviral vector.

12. A method as defined in claims 10 or 11 wherein the recombinant AAV virion is a single-stranded AAV virion or a double-stranded AAV virion.

13. A method as defined in claim 12 wherein the recombinant AAV virion is a double-stranded AAV virion.

14. A method as defined in claims 10 to 13 wherein the expression cassette (a) comprises a polyadenylation signal downstream of the sequence encoding IGF-I or the functional equivalent thereof having the biological activity of IGF-I.

15. A method as defined in claims 10 to 14 wherein component (b) comprises the gene encoding the AAV2 rep gene and/or component (c) comprises the AAV1, AAV2, AAV5 or the AAV8 cap gene.

## Patentansprüche

1. Parvoviraler Vektor, der eine Nucleotidsequenz umfasst, die den insulinartigen Wachstumsfaktor I IGF-I oder eine funktionell äquivalente Variante davon, die die biologische Aktivität des IGF-I hat, codiert, die mit einem Leber-spezifischen Promotor funktionell verknüpft ist, umfassend die Enhancer-Region des Albumin-Gens und den Alpha-1-Antitrypsin-Promotor.

2. Parvoviraler Vektor wie in Anspruch 1 definiert, wobei der parvovirale Vektor ein AAV ist.

3. Parvoviraler Vektor wie in Anspruch 2 definiert, wobei der AAV-Vektor ein AAV1, AAV2, AAV5 oder AAV8 ist.

4. Parvoviraler Vektor wie in Anspruch 2 oder 3 definiert, wobei der AAV-Vektor ein einzelsträngiger AAV oder ein doppelsträngiger AAV ist.

5. Parvoviraler Vektor wie in Anspruch 4 definiert, wobei der AAV-Vektor ein doppelsträngiger AAV ist.

6. Parvoviraler Vektor wie in einem der Ansprüche 1 bis 5 definiert, wobei der IGF-I ein humaner IGF-I ist.

7. Virion, das einen parvoviralen Vektor wie in einem der Ansprüche 1 bis 6 definiert umfasst.

8. Virion nach Anspruch 7 zur Verwendung als Medikament.

9. Virion wie in Anspruch 7 definiert zur Verwendung in einem Verfahren zur Behandlung und/oder Vorbeugung von Leberzirrhose oder Leberfibrose.

10. *In vitro*-Verfahren zur Herstellung eines rekombinanten AAV-Virions, umfassend die Schritte des
(i) Inkontaktbringens einer Zelle mit
(a) einer ersten Nucleinsäuresequenz, umfassend
i. eine Expressionskassette, umfassend eine Sequenz, die den IGF-I oder eine funktionell äquivalente Variante davon, die die biologische Aktivität des IGF-I hat, codiert, die mit dem Leber-spezifischen Promotor funktionell verknüpft ist, umfassend die Enhancer-Region des Albumin-Gens und den Alpha1-Antitrypsin-Promotor und
ii. eine AAV 5'-ITR und eine 3'-ITR, die die in (i) definierte Expressionskassette flankieren,
(b) einer zweiten Nucleinsäuresequenz, die ein AAV-rep-Protein codiert,
(c) einer dritten Nucleinsäuresequenz, die ein AAV-cap-Protein codiert, und gegebenenfalls
(d) einer vierten Nucleinsäuresequenz, die virale und/oder zelluläre Funktionen codiert, von der das AAV für die Replikation abhängig ist,
unter Bedingungen, die für den Eintritt der drei Komponenten in die Zelle geeignet sind und
(ii) Wiedergewinnens des rekombinanten AAV-Virions aus den Zellen.

11. Verfahren wie in Anspruch 10 definiert, wobei die in Schritt (i) verwendete Zelle eine Insektenzelle ist und wobei die erste, zweite und dritte Nucleinsäuresequenz in einem baculoviralen Vektor umfasst sind.

12. Verfahren wie in Anspruch 10 oder 11 definiert, wobei das rekombinante AAV-Virion ein einzelsträngiges AAV-Virion oder ein doppelsträngiges AAV-Virion ist.

13. Verfahren wie in Anspruch 12 definiert, wobei das rekombinante AAV-Virion ein doppelsträngiges AAV-Virion ist.

14. Verfahren wie in den Ansprüchen 10 bis 13 definiert, wobei die Expressionskassette (a) ein Polyadenylierungssignal umfasst, das stromabwärts der Sequenz ist, die den IGF-I oder das funktionelle Äquivalent davon, das die biologische Aktivität des IGF-I hat, codiert.

15. Verfahren wie in den Ansprüchen 10 bis 14 definiert, wobei Komponente (b) das Gen umfasst, das das AAV2-rep-Gen codiert und/oder Komponente (c) das AAV1-, AAV2-, AAV5- oder das AAV8-cap-Gen umfasst.

## Revendications

1. Vecteur parvoviral comprenant une séquence de nucléotides codant pour le facteur de croissance de type insuline I IGF-I ou un variant fonctionnellement équivalent de celui-ci ayant l'activité biologique de l'IGF-I qui est liée de manière opérationnelle à un promoteur spécifique du foie comprenant la région amplificatrice du gène de l'albumine et le promoteur de l'alpha 1-antitrypsine.

2. Vecteur parvoviral selon la revendication 1, dans lequel le vecteur parvoviral est un AAV.

3. Vecteur parvoviral selon la revendication 2, dans lequel le vecteur AAV est un AAV1, un AAV2, un AAV5 ou un AAV8.

4. Vecteur parvoviral selon la revendication 2 ou la revendication 3, dans lequel le vecteur AAV est un AAV monocaténaire ou un AAV bicaténaire.

5. Vecteur parvoviral selon la revendication 4, dans lequel le vecteur AAV est un AAV bicaténaire.

6. Vecteur parvoviral selon l'une quelconque des revendications 1 à 5, dans lequel l'IGF-I est un IGF-I humain.

7. Virion comprenant le vecteur parvoviral selon l'une quelconque des revendications 1 à 6.

8. Virion selon la revendication 7, pour son utilisation en tant que médicament.

9. Virion selon la revendication 7, pour son utilisation dans un procédé de traitement et/ou de prévention de la cirrhose hépatique ou de la fibrose hépatique.

10. Procédé *in vitro* de préparation d'un virion d'AAV recombinant, ledit procédé comprenant les étapes de
(i) mise en contact d'une cellule avec
(a) une première séquence d'acide nucléique comprenant
i. une cassette d'expression comprenant une séquence codant pour l'IGF-I ou un variant fonctionnellement équivalent de celui-ci ayant l'activité biologique de l'IGF-I qui est liée de manière opérationnelle à un promoteur spécifique du foie comprenant la région amplificatrice du gène de l'albumine et le promoteur de l'alpha 1-antitrypsine et
ii. une 5'-ITR et une 3'-ITR d'AAV flanquant la cassette d'expression définie en (i),
(b) une deuxième séquence d'acide nucléique codant pour une protéine rep d'AAV,
(c) une troisième séquence d'acide nucléique codant pour une protéine cap d'AAV et, facultativement,
(d) une quatrième séquence d'acide nucléique codant pour les fonctions virales et/ou cellulaires dont l'AAV est dépendant pour sa réplication,
dans des conditions adaptées à l'entrée des trois composants dans la cellule et
(ii) la récupération du virion d'AAV recombinant des cellules.

11. Procédé selon la revendication 10, dans lequel la cellule utilisée dans l'étape (i) est une cellule d'insecte et dans lequel les première, deuxième et troisième séquences d'acide nucléique sont comprises dans un vecteur baculoviral.

12. Procédé selon la revendication 10 ou la revendication 11, dans lequel le virion d'AAV recombinant est un virion d'AAV monocaténaire ou un virion d'AAV bicaténaire.

13. Procédé selon la revendication 12, dans lequel le virion d'AAV est un virion d'AAV bicaténaire.

14. Procédé selon les revendications 10 à 13, dans lequel la cassette d'expression (a) comprend un signal de polyadénylation en aval de la séquence codant pour l'IGF-I ou son équivalent fonctionnel ayant l'activité biologique de l'IGF-I.

15. Procédé selon l'une quelconque des revendications 10 à 14 dans lequel le composant (b) comprend le gène codant pour le gène rep de l'AAV2 et/ou le composant (c) comprend le gène cap de l'AAV1, de l'AAV2, de l'AAV5 ou de l'AAV8.


### SEQUENCE LISTING

<110> PROYECTO DE BIOMEDICINA CIMA, S. L.
<120> METHODS AND COMPOSITIONS FOR THE TREATMENT OF CIRRHOSIS AND LIVER
   FIBROSIS
<130> P4629PCOO
<150> ES P200900846
   <151> 2009-03-27
<160> 57
<170> PatentIn version 3.5
<210> 1
   <211> 158
   <212> PRT
   <213> Human
<400> 1
   Met Gly Lys Ile Ser Ser Leu Pro Thr Gln Leu Phe Lys Cys Cys Phe
   1               5                   10                  15
   Cys Asp Phe Leu Lys Val Lys Met His Thr Met Ser Ser Ser His Leu
               20                  25                  30
   Phe Tyr Leu Ala Leu Cys Leu Leu Thr Phe Thr Ser Ser Ala Thr Ala
           35                  40                  45
   Gly Pro Glu Thr Leu Cys Gly Ala Glu Leu Val Asp Ala Leu Gln Phe
       50                  55                  60
   Val Cys Gly Asp Arg Gly Phe Tyr Phe Asn Lys Pro Thr Gly Tyr Gly
   65                  70                  75                  80
   Ser Ser Ser Arg Arg Ala Pro Gln Thr Gly Ile Val Asp Glu Cys Cys
                   85                  90                  95
   Phe Arg Ser Cys Asp Leu Arg Arg Leu Glu Met Tyr Cys Ala Pro Leu
               100                 105                 110
   Lys Pro Ala Lys Ser Ala Arg Ser Val Arg Ala Gln Arg His Thr Asp
           115                 120                 125
   Met Pro Lys Thr Gln Lys Tyr Gln Pro Pro Ser Thr Asn Lys Asn Thr
       130                 135                 140
   Lys Ser Gln Arg Arg Lys Gly Ser Thr Phe Glu Glu Arg Lys
   145                 150                 155
<210> 2
   <211> 137
   <212> PRT
   <213> Human
<400> 2
   Met Ile Thr Pro Thr Val Lys Met His Thr Met Ser Ser Ser His Leu
   1               5                   10                  15
   Phe Tyr Leu Ala Leu Cys Leu Leu Thr Phe Thr Ser Ser Ala Thr Ala
               20                  25                  30
   Gly Pro Glu Thr Leu Cys Gly Ala Glu Leu Val Asp Ala Leu Gln Phe
           35                  40                  45
   Val Cys Gly Asp Arg Gly Phe Tyr Phe Asn Lys Pro Thr Gly Tyr Gly
       50                  55                  60
   Ser Ser Ser Arg Arg Ala Pro Gln Thr Gly Ile Val Asp Glu Cys Cys
   65                  70                  75                  80
   Phe Arg Ser Cys Asp Leu Arg Arg Leu Glu Met Tyr Cys Ala Pro Leu
                   85                  90                  95
   Lys Pro Ala Lys Ser Ala Arg Ser Val Arg Ala Gln Arg His Thr Asp
               100                 105                 110
   Met Pro Lys Thr Gln Lys Glu Val His Leu Lys Asn Ala Ser Arg Gly
           115                 120                 125
   Ser Ala Gly Asn Lys Asn Tyr Arg Met
       130                 135
<210> 3
   <211> 195
   <212> PRT
   <213> Human
<400> 3
   Met Gly Lys Ile Ser Ser Leu Pro Thr Gln Leu Phe Lys Cys Cys Phe
   1               5                   10                  15
   Cys Asp Phe Leu Lys Val Lys Met His Thr Met Ser Ser Ser His Leu
               20                  25                  30
   Phe Tyr Leu Ala Leu Cys Leu Leu Thr Phe Thr Ser Ser Ala Thr Ala
           35                  40                  45
   Gly Pro Glu Thr Leu Cys Gly Ala Glu Leu Val Asp Ala Leu Gln Phe
       50                  55                  60
   Val Cys Gly Asp Arg Gly Phe Tyr Phe Asn Lys Pro Thr Gly Tyr Gly
   65                  70                  75                  80
   Ser Ser Ser Arg Arg Ala Pro Gln Thr Gly Ile Val Asp Glu Cys Cys
                   85                  90                  95
   Phe Arg Ser Cys Asp Leu Arg Arg Leu Glu Met Tyr Cys Ala Pro Leu
               100                 105                 110
   Lys Pro Ala Lys Ser Ala Arg Ser Val Arg Ala Gln Arg His Thr Asp
           115                 120                 125
   Met Pro Lys Thr Gln Lys Tyr Gln Pro Pro Ser Thr Asn Lys Asn Thr
       130                 135                 140
   Lys Ser Gln Arg Arg Lys Gly Trp Pro Lys Thr His Pro Gly Gly Glu
   145                 150                 155                 160
   Gln Lys Glu Gly Thr Glu Ala Ser Leu Gln Ile Arg Gly Lys Lys Lys
                   165                 170                 175
   Glu Gln Arg Arg Glu Ile Gly Ser Arg Asn Ala Glu Cys Arg Gly Lys
               180                 185                 190
   Lys Gly Lys
           195
<210> 4
   <211> 673
   <212> DNA
   <213> Artificial sequence
<220>
   <223> Albumin enhancer and alphal-antitrypsin (AAT) promoter
<400> 4
   tcgaggttcc tagattacac tacacattct gcaagcatag cacagagcaa tgttctactt    60
   taattacttt cattttcttg tatcctcaca gcctagaaaa taacctgcgt tacagcatcc   120
   actcagtatc ccttgagcat gaggtgacac tacttaacat agggacgaga tggtactttg   180
   tgtctcctgc tctgtcagca gggcacagta cttgctgata ccagggaatg tttgttctta   240
   aataccatca ttccggacgt gtttgccttg gccagttttc catgtacatg cagaaagaag   300
   tttggactga tcaatacagt cctctgcctt taaagcaata ggaaaaggcc aacttgtcta   360
   cgtttagtat gtggctgtag atctgtaccc gccaccccct ccaccttgga cacaggacgc  420
   tgtggtttct gagccaggta caatgactcc tttcggtaag tgcagtggaa gctgtacact 480
   gcccaggcaa agcgtccggg cagcgtaggc gggcgactca gatcccagcc agtggactta 540
   gcccctgttt gctcctccga taactggggt gaccttggtt aatattcacc agcagcctcc 600
   cccgttgccc ctctggatcc actgcttaaa tacggacgag gacagggccc tgtctcctca 660
   gcttcaggca cca 673
<210> 5
   <211> 10
   <212> RNA
   <213> Artificial
<220>
   <223> Kozak consensus sequence
<400> 5
   gccrccauga 10
<210> 6
   <211> 145
   <212> DNA
   <213> AAV2
<400> 6
   ttggccactc cctctctgcg cgctcgctcg ctcactgagg ccgggcgacc aaaggtcgcc 60
   cgacgcccgg gctttgcccg ggcggcctca gtgagcgagc gagcgcgcag agagggagtg 120
   gccaactcca tcactagggg ttcct 145
<210> 7
   <211> 146
   <212> DNA
   <213> AAV2
<400> 7
   ttggccactc cctctctgcg cgctcgctcg ctcactgagg ccgcccgggc aaagcccggg 60
   cgtcgggcga cctttggtcg cccggcctca gtgagcgagc gagcgcgcag agagggagtg 120
   gccaactcca tcactagggg ttcccc 146
<210> 8
   <211> 21
   <212> DNA
   <213> Artificial
<220>
   <223> Primer pr300fw
<400> 8
   ccctgtttgc tcctccgata a 21
<210> 9
   <211> 24
   <212> DNA
   <213> Artificial
<220>
   <223> Primer pr301rv
<400> 9
   gtccgtattt aagcagtgga tcca 24
<210> 10
   <211> 20
   <212> DNA
   <213> Artificial
<220>
   <223> Forward Primer GADPH
<400> 10
   cttccacgat gccaaagttg 20
<210> 11
   <211> 19
   <212> DNA
   <213> Artificial
<220>
   <223> Reverse primer GADPH
<400> 11
   gatggtgaag gtcggtgtg 19
<210> 12
   <211> 20
   <212> DNA
   <213> Artificial
<220>
   <223> Forward primer IGF-I
<400> 12
   gtgtcgatag gggctgggac 20
<210> 13
   <211> 22
   <212> DNA
   <213> Artificial
<220>
   <223> Reverse primer IGF-I
<400> 13
   ttcagttcgt gtgtggacaa gg 22
<210> 14
   <211> 18
   <212> DNA
   <213> Artificial
<220>
   <223> Forward primer TGF-beta
<400> 14
   cggcagctgt acattgac 18
<210> 15
   <211> 20
   <212> DNA
   <213> Artificial
<220>
   <223> Reverse primer TGF-beta
<400> 15
   tcagctgcac ttgcaggagc 20
<210> 16
   <211> 21
   <212> DNA
   <213> Artificial
<220>
   <223> Forward primer collagen I
<400> 16
   caagaatggc gaccgtggtg a 21
<210> 17
   <211> 21
   <212> DNA
   <213> Artificial
<220>
   <223> Reverse primer Collagen I
<400> 17
   ggtgtgactc gtgcagccat c 21
<210> 18
   <211> 21
   <212> DNA
   <213> Artificial
<220>
   <223> Forward primer collagen IV
<400> 18
   gcaggtgtgc ggtttgtgaa g 21
<210> 19
   <211> 21
   <212> DNA
   <213> Artificial
<220>
   <223> Reverse primer Collagen IV
<400> 19
   agctcccctg ccttcaaggt g 21
<210> 20
   <211> 21
   <212> DNA
   <213> Artificial
<220>
   <223> Forward primer CTGF
<400> 20
   atcccctgcg agggacacaa g 21
<210> 21
   <211> 22
   <212> DNA
   <213> Artificial
<220>
   <223> Reverse primer CTGF
<400> 21
   caactgcttt ggaaggactc gc 22
<210> 22
   <211> 24
   <212> DNA
   <213> Artificial
<220>
   <223> Forward primer PDGF
<400> 22
   gaagccagtc ttcaagaagg ccac 24
<210> 23
   <211> 24
   <212> DNA
   <213> Artificial
<220>
   <223> Reverse primer PDGF
<400> 23
   aacggtcacc cgagtttgag gtgt 24
<210> 24
   <211> 24
   <212> DNA
   <213> Artificial
<220>
   <223> Forward primer VEGF
<400> 24
   aagccagcac ataggagaga tgag 24
<210> 25
   <211> 24
   <212> DNA
   <213> Artificial
<220>
   <223> Reverse primer VEGF
<400> 25
   tcaccgcctt ggcttgtcac atct 24
<210> 26
   <211> .20
   <212> DNA
   <213> Artificial
<220>
   <223> Forward primer alpha-SMA
<400> 26
   actgggacga catggaaaag 20
<210> 27
   <211> 20
   <212> DNA
   <213> Artificial
<220>
   <223> Reverse primer alpha-SMA
<400> 27
   catctccaga gtccagcaca 20
<210> 28
   <211> 22
   <212> DNA
   <213> Artificial
<220>
   <223> Forward primer HGF
<400> 28
   agggaatcct ctcgttcctt gg 22
<210> 29
   <211> 21
   <212> DNA
   <213> Artificial
<220>
   <223> Reverse primer HGF
<400> 29
   gaggcgaggc gaaacgcaaa c 21
<210> 30
   <211> 18
   <212> DNA
   <213> Artificial
<220>
   <223> Forward primer HNF4-a
<400> 30
   ccttggaccc agcctaca 18
<210> 31
   <211> 19
   <212> DNA
   <213> Artificial
<220>
   <223> Reverse primer HNF4-a
<400> 31
   gcttgaggct ccgtagtgt 19
<210> 32
   <211> 20
   <212> DNA
   <213> Artificial
<220>
   <223> Forward primer MMP-1
<400> 32
   ttgttgctgc ccatgagctt 20
<210> 33
   <211> 20
   <212> DNA
   <213> Artificial
<220>
   <223> Reverse primer MMP-1
<400> 33
   actttgtcgc caattccagg 20
<210> 34
   <211> 20
   <212> DNA
   <213> Artificial
<220> '
   <223> Forward primer MMP-2
<400> 34
   ctattctgtc agcactttgg 20
<210> 35
   <211> 21
   <212> DNA
   <213> Artificial
<220>
   <223> Reverse primer MMP-2
<400> 35
   cagactttgg ttctccaact t 21
<210> 36
   <211> 19
   <212> DNA
   <213> Artificial
<220>
   <223> Forward primer MMP-9
<400> 36
   aaggatggtc tactggcac 19
<210> 37
   <211> 18
   <212> DNA
   <213> Artificial
<220>
   <223> Reverse primer MMP-9
<400> 37
   agagattctc actggggc 18
<210> 38
   <211> 20
   <212> DNA
   <213> Artificial
<220>
   <223> Forward primer MMP-14
<400> 38
   aaagggaaca aatactggaa 20
<210> 39
   <211> 20
   <212> DNA
   <213> Artificial
<220>
   <223> Reverse primer MMP-14
<400> 39
   atgtagttag ggggatggaa 20
<210> 40
   <211> 20
   <212> DNA
   <213> Artificial
<220>
   <223> AR forward primer
<400> 40
   ctgctggtct taggctcagg 20
<210> 41
   <211> 21
   <212> DNA
   <213> Artificial
<220>
   <223> AR reverse primer
<400> 41
   ccaggttctc gatgtatctg c 21
<210> 42
   <211> 21
   <212> DNA
   <213> Artificial
<220>
   <223> WT-1 forward primer
<400> 42
   ggaaccagat gaacctcgga g 21
<210> 43
   <211> 25
   <212> DNA
   <213> Artificial
<220>
   <223> WT-1 reverse primer
<400> 43
   cgcttctcac tggtttcaga tgctg 25
<210> 44
   <211> 18
   <212> DNA
   <213> Artificial
<220>
   <223> TIMP-2 forward primer
<400> 44
   atttatctac agggcccc 18
<210> 45
   <211> 21
   <212> DNA
   <213> Artificial
<220>
   <223> TIMP-2 reverse primer
<400> 45
   caagaaccat cacttctgtt g 21
<210> 46
   <211> 20
   <212> DNA
   <213> Artificial
<220>
   <223> TNF-alpha forward primer
<400> 46
   gagcacagaa agcatgatcc 20
<210> 47
   <211> 20
   <212> DNA
   <213> Artificial
<220>
   <223> TNF-alpha reverse primer
<400> 47
   agcccatttg ggaacttctc 20
<210> 48
   <211> 20
   <212> DNA
   <213> Artificial
<220>
   <223> IL-6 forward primer
<400> 48
   cagttgcctt cttgggactg 20
<210> 49
   <211> 20
   <212> DNA
   <213> Artificial
<220>
   <223> IL-6 reverse primer
<400> 49
   gttcatacaa tcagaattgc 20
<210> 50
   <211> 22
   <212> DNA
   <213> Artificial
<220>
   <223> IGF Binding Protein-3 forward primer
<400> 50
   cctccgagtc taagcgggag ac 22
<210> 51
   <211> 21
   <212> DNA
   <213> Artificial
<220>
   <223> IGF Binding Protein-3 reverse primer
<400> 51
   gcattgcctc agcgtgcaga g 21
<210> 52
   <211> 19
   <212> DNA
   <213> Artificial
<220>
   <223> PCNA forward primer
<400> 52
   tttgaggcac gcctgatcc 19
<210> 53
   <211> 21
   <212> DNA
   <213> Artificial
<220>
   <223> PCNA reverse primer
<400> 53
   ggagacgtga gacgagtcca t 21
<210> 54
   <211> 20
   <212> DNA
   <213> Artificial
<220>
   <223> IGF-I receptor forward primer
<400> 54
   gacagtgaat gaggctgcaa 20
<210> 55
   <211> 20
   <212> DNA
   <213> Artificial
<220>
   <223> IGF-I receptor reverse primer
<400> 55
   tctccacctc tggccttaga 20
<210> 56
   <211> 24
   <212> DNA
   <213> Artificial
<220>
   <223> 373 forward primer
<400> 56
   ggtaccatgt cgtcttcaca tctc 24
<210> 57
   <211> 20
   <212> DNA
   <213> Artificial
<220>
   <223> 374 reverse primer
<400> 57
   gcggccgcga atgtttactt 20